# EUROPEAN PATENT APPLICATION

(11) **EP 3 950 933 A1**
(43) Date of publication of application: **09.02.2022**
(21) Application number: 20783155.3
(22) Date of filing: 25.03.2020
(51) Int. Cl.: C12N 5/0735, C12N 5/074, C12N 5/0775, C12N 5/078, C12N 5/0793, C12N 5/10, C12N 15/12, C12Q 1/06, C12Q 1/686, C12Q 1/6876

(54) **CELL POPULATION INCLUDING PLURIPOTENT STEM CELLS AND PRODUCTION METHOD THEREOF**

(30) Priority: 29.03.2019 JP 2019066845
(71) Applicant: Kaneka Corporation, Osaka-shi, Osaka 530-8288 (JP)
(72) Inventor: TAKEUCHI, Kazuhiro, Kobe-shi, Hyogo 650-0047 (JP); IBUKI, Masato, Kobe-shi, Hyogo 650-0047 (JP)
(74) Representative: Hoffmann Eitle
(86) International application number: PCT/JP2020/013279
(87) International publication number: WO 2020/203538

(57) **Abstract**

A production method for a cell population containing pluripotent stem cells includes: a step of culturing cell populations containing pluripotent stem cells; and a step of sorting out a cell population having the following characteristics (a) and (b) from the cell populations containing pluripotent stem cells.
(a) A relative expression level of a LEF1 gene with respect to an expression level of a β-actin gene is 5.5 × 10⁻⁴ or less in the cell population.
(b) A percentage of cells positive for LEF1 is 55% or less in the cell population.

## Description

### [Technical Field]

The present invention relates to a cell population containing pluripotent stem cells, and a production method therefor. The present invention further relates to a production method for somatic cells. The present invention further relates to a method for monitoring differentiation potential of pluripotent stem cells in a cell population containing pluripotent stem cells. The present invention further relates to a method for evaluating differentiation potential of pluripotent stem cells in a cell population containing pluripotent stem cells.

Priority is claimed on Japanese Patent Application No. 2019-066845, filed March 29, 2019, the content of which is incorporated herein by reference.

### [Background Art]

Pluripotent stem cells such as ES cells and iPS cells have an ability to grow indefinitely and an ability to differentiate into various types of somatic cells. The practical use of a therapeutic method for transplanting somatic cells obtained by inducing differentiation from pluripotent stem cells has a possibility of fundamentally revolutionizing a therapeutic method for intractable diseases and lifestyle-related diseases. For the practical use of regenerative medicine using pluripotent stem cells, it is required to establish a technique of efficiently inducing differentiation from pluripotent stem cells into target somatic cells. Various efforts have been reported for efficiently inducing differentiation from pluripotent stem cells into target cells. Examples thereof include efforts to increase homogeneity of pluripotent stem cell populations as starting materials, specifically, a method of culturing pluripotent stem cells while maintaining an undifferentiated state thereof, and a method of removing cells deviating from an undifferentiated state (undifferentiation-deviated cells).

Patent Document 1 discloses a method of predicting differentiation potential of pluripotent stem cells, the method including measuring an expression level of CHD7 in human pluripotent stem cells.

### [Citation List]

### [Patent Document]

[Patent Document 1] PCT International Publication No. WO2018/235583

### [Summary of Invention]

### [Technical Problem]

However, Patent Document 1 does not disclose or teach selective preparation of a stem cell population having specific excellent characteristics from cell populations containing pluripotent stem cells, specifically, selective preparation of a cell population, in which undifferentiation potential that enables efficient differentiation induction is maintained at a high level, using characteristics of pluripotent stem cells as indexes.

An object of the present invention is to provide a cell population containing pluripotent stem cells that can be induced to efficiently differentiate, and a method for producing pluripotent stem cells. Another object of the present invention is to provide a production method for somatic cells using the above-mentioned cell population containing pluripotent stem cells. Still another object of the present invention is to provide a method for monitoring differentiation potential of pluripotent stem cells in a cell population containing pluripotent stem cells.

### [Solution to Problem]

As a result of diligent studies to achieve the above-mentioned objects, the inventors of the present invention have found that, in undifferentiation maintenance culture of pluripotent stem cells, a frequency of appearance of undifferentiation-deviated cells is low, in a cell population which contains pluripotent stem cells and in which an expression level of a LEF1 gene and a percentage of pluripotent stem cells positive for LEF1 are equal to or less than predetermined values; and many undifferentiation-deviated cells appear in a cell population which contains pluripotent stem cells and in which an expression level of a LEF1 gene and a percentage of pluripotent stem cells positive for LEF1 are equal to or more than predetermined values. Accordingly, it was found that, by sorting out cells using LEF1 as an index, it is possible to prepare pluripotent stem cells among which a content of undifferentiation-deviated cells is small, and thereby differentiation into somatic cells can be induced with higher efficiency. The present invention has been completed based on these findings.

That is, according to the present invention, the following inventions are provided.
<1> A production method for a cell population containing pluripotent stem cells, the method including:
   a step of culturing cell populations containing pluripotent stem cells; and
   a step of sorting out a cell population having the following characteristics (a) and (b) from the cell populations containing pluripotent stem cells.
      (a) A relative expression level of a LEF1 gene with respect to an expression level of a β-actin gene is 5.5 × 10⁻⁴ or less in the cell population.
      (b) A percentage of cells positive for LEF1 is 55% or less in the cell population.
<2> A production method for a cell population containing pluripotent stem cells, the method including:
   a step of culturing a cell population containing pluripotent stem cells;
   a step of confirming whether the cell population is a cell population having the following characteristics (a) and (b); and
   a step of acquiring the cell population confirmed to be the cell population having the characteristics (a) and (b).
      (a) A relative expression level of a LEF1 gene with respect to an expression level of a β-actin gene is 5.5 × 10⁻⁴ or less in the cell population.
      (b) A percentage of cells positive for LEF1 is 55% or less in the cell population.
<3> A production method for somatic cells, the method including:
   a step of culturing cell populations containing pluripotent stem cells;
   a step of sorting out a cell population having the following characteristics (a) and (b) from the cell populations containing pluripotent stem cells; and
   a step of culturing the cell population having the characteristics (a) and (b) in the presence of a differentiation-inducing factor.
      (a) A relative expression level of a LEF1 gene with respect to an expression level of a β-actin gene is 5.5 × 10⁻⁴ or less in the cell population.
      (b) A percentage of cells positive for LEF1 is 55% or less in the cell population.
<4> A production method for somatic cells, the method including:
   a step of culturing a cell population containing pluripotent stem cells;
   a step of confirming whether the cell population is a cell population having the following characteristics (a) and (b); and
   a step of culturing the cell population confirmed to be the cell population having the characteristics (a) and (b) in the presence of a differentiation-inducing factor.
      (a) A relative expression level of a LEF1 gene with respect to an expression level of a β-actin gene is 5.5 × 10⁻⁴ or less in the cell population.
      (b) A percentage of cells positive for LEF1 is 55% or less in the cell population.
<5> The production method according to <3> or <4>, in which the somatic cells are at least one selected from the group consisting of endoderm cells, mesoderm cells, and ectoderm cells.
<6> The production method according to <3> or <4>, in which the somatic cells are at least one selected from the group consisting of cardiac muscle cells, skeletal muscle cells, nerve cells, megakaryocytes, hematopoietic stem cells, airway epithelial cells, germ cells, dendritic cells, eosinophils, mast cells, cartilage cells, T cells, erythropoietin-producing cells, intestinal epithelium, pancreatic cells, liver cells, alveolar epithelial cells, and kidney cells.
<6-1> The method according to any one of <1>, <3>, <5>, and <6>, in which the step of sorting out a cell population having characteristics (a) and (b) includes culturing the cell population containing pluripotent stem cells in the presence of a ROCK inhibitor.
<6-2> The method according to any one of <1> to <6>, in which the step of culturing cell populations containing pluripotent stem cells includes culturing the cell population containing pluripotent stem cells in the presence of a ROCK inhibitor.
<7> A cell population containing pluripotent stem cells, in which the cell population is a cell population having the following characteristics (a) and (b).
   (a) A relative expression level of a LEF1 gene with respect to an expression level of a β-actin gene is 5.5 × 10⁻⁴ or less in the cell population.
   (b) A percentage of pluripotent stem cells positive for LEF1 is 55% or less in the cell population.
<7-1> The cell population according to <7>, in which the cell population further has the following characteristics (c), (d), and (e).
   (c) A relative expression level of an Oct4 gene with respect to an expression level of a β-actin gene is 2.0 × 10⁻¹ or more in the cell population.
   (d) A relative expression level of a Sox2 gene with respect to an expression level of a β-actin gene is 1.0 × 10⁻² or more in the cell population.
   (e) A relative expression level of a Nanog gene with respect to an expression level of a β-actin gene is 1.2 × 10⁻² or more in the cell population.
<7-2> The cell population according to <7> or <7-1>, in which the cell population further has the following characteristics (f), (g), and (h).
   (f) A relative expression level of a Brachyury gene with respect to an expression level of a β-actin gene is 1.0 × 10⁻⁴ or less in the cell population.
   (g) A relative expression level of a Sox17 gene with respect to an expression level of a β-actin gene is 1.0 × 10⁻⁴ or less in the cell population.
   (h) A relative expression level of a Pax6 gene with respect to an expression level of a β-actin gene is 1.0 × 10⁻⁴ or less in the cell population.
<8> A method for monitoring differentiation potential of a cell population containing pluripotent stem cells using the following characteristics (a) and (b) as indexes.
   (a) A relative expression level of a LEF1 gene with respect to an expression level of a β-actin gene is 5.5 × 10⁻⁴ or less in the cell population.
   (b) A percentage of pluripotent stem cells positive for LEF1 is 55% or less in the cell population.
<8-1> The method according to <8>, in which the differentiation potential of pluripotent stem cells is monitored in the presence of a ROCK inhibitor.
<9> A method for evaluating differentiation potential of a cell population containing pluripotent stem cells using the following characteristics (a) and (b) as indexes.
   (a) A relative expression level of a LEF1 gene with respect to an expression level of a β-actin gene is 5.5 × 10⁻⁴ or less in the cell population.
   (b) A percentage of pluripotent stem cells positive for LEF1 is 55% or less in the cell population.

### [Advantageous Effects of Invention]

According to the methods of the present invention, it is possible to produce a cell population, containing pluripotent stem cells that can be induced to differentiate into somatic cells, with high efficiency. Furthermore, the cell population containing pluripotent stem cells according to the present invention can be induced to differentiate into somatic cells with high efficiency.

### [Brief Description of Drawings]

Fig. 1 shows phase-contrast images of day 5 and day 10 of culture of human iPS cells.
Figs. 2A to 2C show results of measuring expression levels of a LEF1 gene, expression levels of undifferentiation marker genes, and expression levels of differentiation marker genes of human iPS cells on day 5 of the culture. Fig. 2A shows the results of measuring the expression levels of the undifferentiation marker genes.
Fig. 2B shows the results of measuring the expression levels of the differentiation marker genes.
Fig. 2C shows the results of measuring the expression levels of the LEF1 gene.
Figs. 3A and 3B show results of measuring expression levels of undifferentiation marker genes and expression levels of differentiation marker genes of human iPS cells on day 10 of the culture. Fig. 3A shows the results of measuring the expression levels of the undifferentiation marker genes and the expression levels of the differentiation marker genes.
Fig. 3B shows the results of measuring the expression levels of the differentiation marker genes.
Fig. 4 shows results of measuring percentages of cells positive for LEF1 among human iPS cells.
Fig. 5 shows results of measuring expression of endoderm markers, mesoderm markers, and ectoderm markers after inducing differentiation from a human iPS cell line RPChiPS771-2 into three germ layer cells.

### [Description of Embodiments]

### [Cell population containing pluripotent stem cells]

A cell population containing pluripotent stem cells according to the present invention is a cell population having the following characteristics (a) and (b).
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

The β-actin gene is also denoted as ACTB, which is a housekeeping gene, and is one of genes used as an endogenous control of quantitative real-time PCR. Examples of cDNA sequences of a human β-Actin gene (GENE ID: 60) and amino acid sequence encoded by the cDNA sequences include those registered as NCBI RefSeq: NM_001101.5 and NP_001092.1.

The LEF1 gene is a Lymphoid enhancer-binding factor 1 gene. LEF1 is also denoted as LEF-1, TCF10, TCF1ALPHA, and TCF7L3 as aliases. LEF1 is a transcription factor in WNT/β-catenin signaling that is important for the development of organisms, and has been reported to be involved in the transcription of WNT target genes via β-catenin. Examples of cDNA sequences of a human LEF1 gene (GENE ID: 51176) and amino acid sequences encoded by the cDNA sequences include those registered as NCBI RefSeq: NM_016269.5 and NP_057353.1 (SEQ ID NOs: 31 and 32), NCBI RefSeq: 001130713.2 and NP_001124185.1 (SEQ ID NOs: 33 and 34), NCBI RefSeq: NM_001130714.2 and NP_001124186.1 (SEQ ID NOs: 35 and 36), and NCBI RefSeq: NM_001166119.1 and NP_001159591.1 (SEQ ID NOs: 37 and 38).

### (Pluripotent stem cells)

Pluripotent stem cells are cells which have multilineage potential (pluripotency) capable of differentiating into all kinds of cells constituting the biological body and are to cells which can continue to grow indefinitely while maintaining their pluripotency in in vitro culture under appropriate conditions. Specific examples thereof include, but are not limited to, embryonic stem cells (ES cells), pluripotent stem cells derived from fetal primordial germ cells (EG cells: Proc Natl Acad Sci USA. 1998, 95: 13726-31), pluripotent stem cells derived from testicles (GS cells: Nature. 2008, 456: 344-9), induced pluripotent stem cells (iPS cells), human somatic stem cells (tissue stem cells), and the like. Pluripotent stem cells used in the present invention are preferably iPS cells or ES cells, and more preferably iPS cells.

As ES cells, it is possible to use cells derived from any warm-blooded animal, preferably mammals. Examples of mammals include mice, rats, guinea pigs, hamsters, rabbits, cats, dogs, sheep, pigs, cattle, horses, goats, monkeys, or humans. Cells derived from humans can be preferably used.

Specific examples of ES cells include ES cells of mammals or the like which are established by culturing preimplantation early embryos, ES cells established by culturing early embryos produced by nuclear transplantation of nuclei of somatic cells, and ES cells in which genes on the chromosomes of these ES cells are modified using genetic engineering techniques. Each of the ES cells can be prepared according to a method generally performed in the field or a known document. ES cells of mice are established by Evans et al. in 1981 (Evans et al., 1981, Nature 292: 154-6) and Martin et al. (Martin GR. et al., 1981, Proc Natl Acad Sci 78: 7634-8). ES cells of humans are established by Thomson et al. in 1998 (Thomson et al., Science, 1998, 282: 1145-7). The cells are available from WiCell Research Institute (website: www.wicell.org/, Madison, Wisconsin, United States), National Institute of Health, Kyoto University, and the like, and can be purchased from, for example, Cellartis (website: www.cellartis.com/, Sweden).

Induced pluripotent stem cells (iPS cells) are cells which have pluripotency and are obtained by reprogramming somatic cells. The production of iPS cells has been successful by a plurality of groups including a group of a Professor Shinya Yamanaka et al. at Kyoto University, a group of Rudolf Jaenisch et al. at the Massachusetts Institute of Technology, a group of James Thomson et al. at the University of Wisconsin, a group of Konrad Hochedlinger et al. at Harvard University, and the like. For example, PCT International Publication No. WO2007/069666 discloses somatic cell nuclear reprogramming factors including gene products of Oct family genes, Klf family genes, and Myc family genes, and somatic cell nuclear reprogramming factors including gene products of Oct family genes, Klf family genes, Sox family genes, and Myc family genes; and further discloses a method for producing induced pluripotent stem cells by nuclear reprogramming of somatic cells, the method including a step of bringing the above-mentioned nuclear reprogramming factors into contact with somatic cells.

The type of somatic cell used for producing iPS cell is not particularly limited, and any somatic cell can be used. That is, somatic cells include all cells other than germ cells among the cells constituting the biological body, and may be differentiated somatic cells or undifferentiated stem cells. The origin of somatic cells is not particularly limited and may be any of mammals, birds, fish, reptiles, and amphibians, but the origin is preferably mammals (for example, rodents such as mice, or primates such as humans) and is particularly preferably mice or humans. Furthermore, when human somatic cells are used, somatic cells of any of fetuses, newborn infants, or adults may be used. Specific examples of somatic cells include fibroblasts (such as dermal fibroblasts), epithelial cells (such as gastric epithelial cells, hepatic epithelial cells, and alveolar epithelial cells), endothelial cells (such as blood vessels and lymph vessels), nerve cells (such as neurons and glial cells), pancreatic cells, white blood cells (B cells, T cells, and the like), bone marrow cells, muscle cells (for example, skeletal muscle cells, smooth muscle cells, and cardiac muscle cells), hepatic parenchymal cells, non-hepatic parenchymal cells, fat cells, osteoblasts, cells constituting periodontal tissues (such as periodontal ligament cells, cementoblasts, gingival fibroblasts, and osteoblasts), cells constituting the kidneys, eyes, and ears, and the like. Cells used in the present invention may be cells derived from any animal. For example, cells may be derived from rodents such as mice, rats, and hamsters; primates such as humans, gorillas, and chimpanzees; and mammals such as domestic animals or pet animals such as dogs, cats, rabbits, cattle, horses, sheep, and goats, but cells derived from humans are preferable.

iPS cells are stem cells having a long-term self-renewal ability under predetermined culture conditions (for example, conditions for culturing ES cells), and having multilineage potential into various somatic cells under predetermined differentiation induction conditions. Furthermore, iPS cells may be stem cells having an ability to form a teratoma when being transplanted into a test animal such as a mouse.

In order to produce iPS cells from somatic cells, first, at least one or more of reprogramming genes is introduced into somatic cells. A reprogramming gene is a gene encoding a reprogramming factor having an action of reprogramming somatic cells into iPS cells. Specific examples of combinations of reprogramming genes include, but are not limited to, the following combinations.
(i) Oct gene, Klf gene, Sox gene, Myc gene
(ii) Oct gene, Sox gene, NANOG gene, LIN28 gene
(iii) Oct gene, Klf gene, Sox gene, Myc gene, hTERT gene, SV40 large T gene
(iv) Oct gene, Klf gene, Sox gene

In addition to the above combinations, a method of further reducing transgenes (Nature. 2008 Jul 31; 454 (7204): 646-50), a method using low-molecular-weight compounds (Cell Stem Cell. 2009 Jan 9; 4 (1): 16-9, Cell Stem Cell. 2009 Nov 6; 5 (5): 491-503), a method using transcription factor proteins instead of genes (Cell Stem Cell. 2009 May 8; 4 (5): 381-4), and the like have been reported. iPS cells may be iPS cells produced by any method.

A form of introducing reprogramming factors into cells is not particularly limited, and examples thereof include gene introduction using a plasmid, introduction of synthetic RNA, direct introduction as a protein, and the like. Furthermore, iPS cells, which are produced by a method using microRNA or RNA, low-molecular-weight compounds, or the like, may be used. As pluripotent stem cells including ES cells and iPS cells, commercially available cells or cells for sale may be used, or newly produced cells may be used.

As iPS cells, it is possible to use, for example, cell lines 253G1, 253G4, 201B6, 201B7, 409B2, 454E2, 606A1, 610B1, 648A1, 1201C1, 1205D1, 1210B2, 1231A3, 1383D2, 1383D6, iPS-TIG120-3f7, iPS-TIG120-4f1, iPS-TIG114-4f1, RPChiPS771-2, 15M63, 15M66, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC38-2, MSC-iPSC1, BJ-iPSC1, and the like.

As ES cells, it is possible to use, for example, cell lines KhES-1, KhES-2, KhES-3, KhES-4, KhES-5, SEES1, SEES2, SEES3, HUES8, CyT49, HI, H9, HS-181, and the like. Newly produced clinical grade iPS cells or ES cells may be used.

In the cell population containing pluripotent stem cells in the present invention, a percentage of pluripotent stem cells is preferably 80% or more and is more preferably 90% or more, and it may be 100%. The cell population satisfying the above-described indexes (a) and (b) is generally a cell population containing pluripotent stem cells in the above-mentioned percentage.

### (Relative expression level of LEF1 gene with respect to expression level of β-actin gene)

In the cell population of the present invention, a relative expression level of a LEF1 gene with respect to an expression level of a β-actin gene is 5.5 × 10⁻⁴ or less. An upper limit of the relative expression level of the LEF1 gene with respect to the expression level of the β-actin gene is, for example, 5.0 × 10⁻⁴ or less, 4.5 × 10⁻⁴ or less, 4.0 × 10⁻⁴ or less, 3.5 × 10⁻⁴ or less, 3.0 × 10⁻⁴ or less, 2.5 × 10⁻⁴ or less, or 2.0 × 10⁻⁴ or less, and is preferably 1.9 × 10⁻⁴ or less, 1.8 × 10⁻⁴ or less, 1.7 × 10⁻⁴ or less, 1.6 × 10⁻⁴ or less, 1.5 × 10⁻⁴ or less, 1.4 × 10⁻⁴ or less, 1.3 × 10⁻⁴ or less, 1.2 × 10⁻⁴ or less, 1.1 × 10⁻⁴ or less, 1.0 × 10⁻⁴ or less, 9.0 × 10⁻⁵ or less, 8.0 × 10⁻⁵ or less, 7.0 × 10⁻⁵ or less, 6.0 × 10⁻⁵ or less, 5.0 × 10⁻⁵ or less, 4.0 × 10⁻⁵ or less, 3.0 × 10⁻⁵ or less, 2.0 × 10⁻⁵ or less, 1.0 × 10⁻⁵ or less, or 1.0 × 10⁻⁶ or less. Furthermore, as a lower limit, for example, 1.0 × 10⁻⁷ or more is preferable.

The relative expression level of the LEF1 gene with respect to the expression level of the β-actin gene can be measured by quantitative real-time PCR analysis. The quantitative real-time PCR analysis can be performed by methods known to those skilled in the art. For example, an expression level of a measurement target gene with respect to an expression level of a β-actin gene can be measured by recovering total RNA of cultured cells, synthesizing cDNA, and, using this cDNA as a template, performing quantitative real-time PCR on the measurement target gene and the β-actin gene.

A method of the quantitative real-time PCR analysis used in the present invention can be performed in the same manner as methods described in Examples to be described later. Specifically, for example, a part of a cell population is collected; RNA is isolated and purified; and cDNA is synthesized from the RNA by a reverse transcription reaction. Next, using the cDNA as a template, quantitative real-time PCR is performed by an intercalation method using SYBR Green. An expression level of a β-actin gene can be measured by performing real-time PCR using primers (for example, SEQ ID NOs: 1 and 2) designed from cDNA sequences of β-actin. An expression level of a LEF1 gene can be measured by performing real-time PCR using primers (for example, SEQ ID NOs: 15 and 16) designed from cDNA sequences of LEF1. An expression level of a LEF1 gene with respect to an expression level of a β-actin gene can be calculated by a comparative Ct method using the expression level of the β-actin gene and the expression level of the LEF1 gene which are measured as described above.

### (Percentage of pluripotent stem cells positive for LEF1)

In the cell population of the present invention, a percentage of pluripotent stem cells positive for LEF1 is 55% or less. An upper limit of the percentage of pluripotent stem cells positive for LEF1 is, for example, 54% or less, 53% or less, 52% or less, 51% or less, 50% or less, or 49% or less, and it may be 48% or less, 47% or less, 46% or less, 45% or less, 44% or less, 43% or less, 42% or less, 41% or less, 40% or less, 39% or less, or 38% or less. Furthermore, a lower limit may include, for example, 0%, and it is preferably 1% or more, 10% or more, 20% or more, or 30% or more.

The percentage of pluripotent stem cells positive for LEF1 in the cell population can be measured by flow cytometry analysis. Flow cytometry metry analysis can be performed by a method known to those skilled in the art. For example, it is possible to perform flow cytometry analysis by performing immobilization of cultured cells and membrane permeation treatment, fluorescently immunostaining with a fluorescently labeled anti-LEF1 antibody and a fluorescently labeled isotype control antibody, and detecting fluorescence of the stained cells using a flow cytometer. When a cell that emits stronger fluorescence as compared to a negative control (isotype control) is detected, the cell is determined to be "positive" for LEF1. A method of the flow cytometry analysis used in the present invention can be performed in the same manner as methods described in Examples to be described later.

The cell population, which contains pluripotent stem cells and satisfies the above-described conditions, is maintained in an undifferentiated state (pluripotency), has a high level of differentiation potential into somatic cells, and can be induced to efficiently differentiate into somatic cells.

### (Differentiation potential)

The differentiation potential in the present invention means an ability of pluripotent stem cells to differentiate into a specific cell type when the pluripotent stem cells are induced to differentiate into the cell type. The differentiation potential of the cell population containing pluripotent stem cells is evaluated as efficiency of inducing differentiation into a specific cell type when the cell population is induced to differentiate into the cell type. Specifically, after inducing differentiation from the cell population containing pluripotent stem cells into a specific cell type, efficiency of inducing differentiation into target cells can be evaluated using morphological characteristics of the target cells, gene expression, protein expression, secretions, and the like as indexes. The target cells are not particularly limited. Specific examples thereof include mesoderm cells, endoderm cells, ectoderm cells, somatic cells having a desired function, and the like, which will be described later. A differentiation-inducing method is not particularly limited, and it is sufficient to use an appropriate differentiation-inducing method depending on the cell type. Examples thereof include a differentiation-inducing method in which a differentiation-inducing medium to be described later and the like are used. A method for evaluating differentiation induction efficiency is not particularly limited. Examples thereof include a method for evaluating cell morphology characteristic to target cells by visual observation with a microscope or image analysis, a method for evaluating a gene expression level characteristic to target cells by quantitative real-time PCR analysis, a method for evaluating protein expression characteristic to target cells by flow cytometry analysis, immunoprecipitation, and immunostaining, an evaluation method using an amount of a substance secreted by target cells, and the like.

By using the indexes (a) and (b), differentiation potential of the cell population can be evaluated before inducing differentiation from the cell population containing pluripotent stem cells into the target cell type. That is, a cell population satisfying the indexes (a) and (b) can be evaluated to have a high level of differentiation potential.

### (Expression of undifferentiation marker)

An undifferentiated state of pluripotent stem cells in the cell population of the present invention can be confirmed by analyzing an expression state of an undifferentiation marker. Analysis of the expression state of the undifferentiation marker can be performed by, for example, quantitative real-time PCR analysis, flow cytometry analysis, or the like.

Examples of undifferentiation markers include, but are not limited to, Oct4, Sox2, Nanog, SSEA-3, SSEA-4, TRA-1-60, TRA-1-81, REX-1, LIN28, LEFTB, GDF3, ZFP42, FGF4, ESG1, DPPA2, TERT, KLF4, c-Myc, Alkaline Phosphatase, and the like. Among them, Oct4, Sox2, and Nanog are preferable as the undifferentiation marker. The undifferentiation marker is synonymous with a pluripotent stem cell marker, and both terms can be used interchangeably.

In the cell population of the present invention, a lower limit of a relative expression level of an Oct4 gene with respect to an expression level of a β-actin gene is preferably, for example, 2.0 × 10⁻¹ or more, 2.1 × 10⁻¹ or more, 2.2 × 10⁻¹ or more, 2.3 × 10⁻¹ or more, 2.4 × 10⁻¹ or more, 2.5 × 10⁻¹ or more, 2.6 × 10⁻¹ or more, 2.7 × 10⁻¹ or more, 2.8 × 10⁻¹ or more, 2.9 × 10⁻¹, 3.0 × 10⁻¹ or more, 4.0 × 10⁻¹ or more, 5.0 × 10⁻¹ or more, or 6.0 × 10⁻¹ or more. Furthermore, an upper limit is preferably, for example, 10 or less, or 1.0 or less.

In the cell population of the present invention, a lower limit of a relative expression level of a Sox2 gene with respect to an expression level of a β-actin gene is preferably, for example, 1.0 × 10⁻² or more, 1.1 × 10⁻² or more, 1.2 × 10⁻² or more, 1.3 × 10⁻² or more, 1.4 × 10⁻² or more, 1.5 × 10⁻² or more, 1.6 × 10⁻² or more, 1.7 × 10⁻² or more, 1.8 × 10⁻² or more, 1.9 × 10⁻² or more, 2.0 × 10⁻² or more, 2.1 × 10⁻² or more, 2.2 × 10⁻² or more, 2.3 × 10⁻² or more, 2.6 × 10⁻² or more, or 2.9 × 10⁻² or more. Furthermore, an upper limit is preferably, for example, 10 or less, 1.0 or less, or 0.1 or less.

In the cell population of the present invention, a lower limit of a relative expression level of a Nanog gene with respect to an expression level of a β-actin gene is preferably, for example, 1.2 × 10⁻² or more, 1.3 × 10⁻² or more, 1.4 × 10⁻² or more, 1.5 × 10⁻² or more, 1.6 × 10⁻² or more, 1.7 × 10⁻² or more, 1.8 × 10⁻² or more, 1.9 × 10⁻² or more, 2.0 × 10⁻² or more, 3.0 × 10⁻² or more, 4.0 × 10⁻² or more, 5.0 × 10⁻² or more, 6.0 × 10⁻² or more, 7.0 × 10⁻² or more, or 8.0 × 10⁻² or more.

Furthermore, an upper limit is preferably, for example, 10 or less, 1.0 or less, or 0.1 or less.

A relative expression level of the above- mentioned gene with respect to an expression level of a β-actin gene can be measured by quantitative real-time PCR analysis.

### (Expression of differentiation marker)

An undifferentiated state of pluripotent stem cells in the cell population of the present invention may be confirmed by analyzing an expression state of a differentiation marker. Analysis of the expression state of the differentiation marker can be performed by, for example, real-time PCR, flow cytometry, or the like.

Examples of differentiation markers include, but are not limited to, Brachyury, Sox17, Pax6, FOXA2, CXCR4, AFP, GATA4, EOMES, MESP1, MESP2, FOXF1, HAND1, EVX1, IRX3, CDX2, TBX6, MIXL1, ISL1, SNAI2, FOXC1, VEGFR2, PDGFRα, FGF5, OTX2, SOX1, NEST1N, and the like. Among them, Brachyury, Sox17, and Pax6 are preferable as differentiation markers.

The above-mentioned Brachyury is a gene specifically expressed in the mesoderm and can be used as a marker gene for mesoderm cells.

The above-mentioned Sox17 is a gene specifically expressed in the endoderm and can be used as a marker gene for endoderm cells.

The above-mentioned Pax6 is a gene specifically expressed in the ectoderm and can be used as a marker gene for ectoderm cells.

In the cell population of the present invention, an upper limit of a relative expression level of a Brachyury gene with respect to an expression level of a β-actin gene is preferably, for example, 1.0 × 10⁻⁴ or less, 1.0 × 10⁻⁵ or less, 1.0 × 10⁻⁶ or less, or 1.0 × 10⁻⁷ or less. Furthermore, as a lower limit, for example, 1.0 × 10⁻⁹ or more is preferable.

In the cell population of the present invention, an upper limit of a relative expression level of a Sox17 gene with respect to an expression level of a β-actin gene is preferably, for example, 1.0 × 10⁻⁴ or less, 1.0 × 10⁻⁵ or less, 1.0 × 10⁻⁶ or less, or 1.0 × 10⁻⁷ or less. Furthermore, as a lower limit, for example, 1.0 × 10⁻⁹ or more is preferable.

In the cell population of the present invention, an upper limit of a relative expression level of a Pax6 gene with respect to an expression level of a β-actin gene is, for example, 1.0 × 10⁻⁴ or less, and is more preferably 1.0 × 10⁻⁵ or less, or 1.5 × 10⁻⁶ or less. Furthermore, as a lower limit, for example, 1.0 × 10⁻⁹ or more is preferable.

A relative expression level of the above-mentioned gene with respect to an expression level of a β-actin gene can be measured by quantitative real-time PCR analysis.

A timing for analyzing the above-described undifferentiation markers and differentiation markers is not particularly limited, but examples thereof include during a culture step, after purification in a culture step, immediately after N times of passage (where N indicates an integer of 1 or more), during maintenance culture, before cryopreservation, after thawing, before formulation, after formulation, and the like.

### [Production method for cell population containing pluripotent stem cells]

The present invention relates to a production method for a cell population containing pluripotent stem cells, the method including a step of sorting out a cell population having the following characteristics (a) and (b) from the cell populations containing pluripotent stem cells.
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

In the present invention, the cell population containing pluripotent stem cells is prepared such that it satisfies the above-described conditions (a) and (b). The above-described conditions (a) and (b) are useful as indexes when acquiring a cell population containing pluripotent stem cells having a high level of differentiation potential while maintaining an undifferentiated state. A method for preparing the cell population is not particularly limited as long as a cell population satisfying the above-described indexes can be acquired thereby. As a such method, a method is an exemplary example, in which a cell population satisfying (b) is selected using a cell sorter, and then the obtained cell population is cultured under conditions satisfying (a). Furthermore, as another method for preparing the cell population satisfying the above-described indexes, a method is an exemplary example, in which a cell population is cultured under conditions satisfying (a) and (b) described above. Details of culture conditions and a culture method will be described below.

A preferable range of a relative expression level of a LEF1 gene with respect to an expression level of a β-actin gene and a preferable range of a percentage of pluripotent stem cells positive for LEF1 are as described above in the present specification.

The cell population containing the pluripotent stem cells can be produced by performing adhesion culture or suspension culture of pluripotent stem cells, after maintenance culture of pluripotent stem cells if desired.

### (Maintenance culture)

In the present invention, pluripotent stem cells before performing adhesion culture or suspension culture are preferably pluripotent stem cells in which an undifferentiated state is maintained by using an undifferentiation maintenance medium. A culture in which an undifferentiated state of pluripotent stem cells is maintained by using an undifferentiation maintenance medium is also referred to as maintenance culture of pluripotent stem cells.

The undifferentiation maintenance medium is not particularly limited as long as it can maintain an undifferentiated state of pluripotent stem cells. Examples of undifferentiation maintenance media include a medium containing, in a basal medium, one or more selected from the group consisting of a Basic fibroblast growth factor-2 (FGF2), a Transforming growth factor-β1 (TGF-β1), Activin A, 1GF-1, MCP-1, IL-6, PAI, PEDF, IGFBP-2, a Leukemia inhibitory factor (LIF), and IGFBP-7; and the like. The factors exemplified above are known to have a property of maintaining an undifferentiated state of pluripotent stem cells. As the undifferentiation maintenance medium, it is possible to use, for example, StemFit (registered trademark) (for example, StemFit (registered trademark) AK02N and the like) (Ajinomoto Co., Inc.), an Essential 8 medium (Life Technologies Japan Ltd.) (Thermo Fisher Scientific K.K.), STEMPRO (registered trademark) hESC FM (Life Technologies Japan Ltd.), mTeSR1 (VERITAS Corporation), TeSR2 (VERITAS Corporation), and the like, but examples are not limited thereto. Furthermore, into the undifferentiation maintenance medium, an antibiotic such as penicillin, streptomycin, and amphotericin B may be added, or a ROCK inhibitor such as CultureSure Y-27632 (Wako Pure Chemical Industries, Ltd.) may be added.

The maintenance culture of pluripotent stem cells can be performed on a cell culture dish coated with a cell adhesion protein such as vitronectin, fibronectin, laminin, or matrigel, using the above-mentioned undifferentiation maintenance medium.

A culture temperature in the maintenance culture of pluripotent stem cells is not particularly limited, but it is preferably from 36.0°C to 38.0°C, and more preferably from 36.5°C to 37.5°C. A culture period is not particularly limited, but it is preferably 1 day to 14 days. The maintenance culture of pluripotent stem cells may be performed, for example, every 1 day to 7 days, every 2 days to 5 days, every 3 days to 5 days, and every 3 days to 4 days, while passaging cells. The number of passages in the maintenance culture is not particularly limited. It is preferable to perform culture at an atmospheric CO₂ concentration of about 1% to 10%, preferably 5%, using a CO₂ incubator or the like.

When performing the maintenance culture of pluripotent stem cells, it is preferable to perform medium exchange at an appropriate frequency. A frequency of medium exchange is not particularly limited, and a frequency of medium exchange can be appropriately adjusted depending on the cell type and culture conditions. A medium exchange operation can be performed at a frequency of, for example, preferably one time or more every 5 days, one time or more every 4 days, one time or more every 3 days, one time or more every 2 days, or one time or more every 1 day. As a liquid medium used for the medium exchange, the same liquid medium as described above can be used. A method for medium exchange is not particularly limited. For example, preferably, maintenance culture can be continued by removing a supernatant by suction from a culture container with an aspirator, a pipette, or the like, thereafter, gently adding a fresh liquid medium, and thereafter, returning the culture container again to a culture environment such as a CO₂ incubator.

A passage method is not particularly limited. For example, preferably, it is possible to perform removing of a supernatant by suction from a culture container with an aspirator, a pipette, or the like, and thereafter washing if necessary. As a washing liquid, it is sufficient to use a buffer (including a PBS buffer), physiological saline, or a liquid medium (preferably a basal medium). For washed cells, the cells may be released by, for example, a mechanical, chemical, or biological method. For continuing the maintenance culture, the cells may be seeded in a fresh medium and a fresh culture container, which are for maintenance culture, and the maintenance culture may be continued. As a liquid medium and culture conditions used for the maintenance culture after passage, it is possible to use the same undifferentiation maintenance medium and conditions as described above. Furthermore, when releasing, cells may be released from a culture substrate or cells may be released from each other by using EDTA, TryPLE^{™} Select, Accutase^{™}, collagenase, DISPASE, trypsin, trypsin/EDTA, trypsin/collagenase, ReLeSR^{™}, or the like as a cell releasing solution; or cells may be released from a culture substrate using a cell scraper or the like. Released cells may be sufficiently dispersed and isolated by pipetting or using a strainer, or may be seeded in a colony form without being dispersed.

### (Adhesion culture)

In the present invention, pluripotent stem cells may be adhesion-cultured in a medium.

The adhesion culture is to culture cells in a state where the cells are adhered to a culture surface such as a culture dish.

A medium in the adhesion culture is not particularly limited as long as it can maintain an undifferentiated state of pluripotent stem cells. Examples of media in the adhesion culture include a medium containing, in a basal medium, one or more selected from the group consisting of a Basic fibroblast growth factor-2 (FGF2), a Transforming growth factor-β1 (TGF-β1), Activin A, IGF-1, MCP-1, IL-6, PAI, PEDF, IGFBP-2, a Leukemia inhibitory factor (LIF), and IGFBP-7; and the like. The factors exemplified above are known to have a property of maintaining an undifferentiated state of pluripotent stem cells. As the medium in the adhesion culture, it is possible to use, for example, StemFit (registered trademark) (for example, StemFit (registered trademark) AK02N and the like) (Ajinomoto Co., Inc.), an Essential 8 medium (Life Technologies Japan Ltd.) (Thermo Fisher Scientific K.K.), STEMPRO (registered trademark) hESC SFM (Life Technologies Japan Ltd.), mTeSR1 (VERITAS Corporation), TeSR2 (VERITAS Corporation), and the like, but examples are not limited thereto. Furthermore, into the medium in the adhesion culture, an antibiotic such as penicillin, streptomycin, and amphotericin B may be added. Furthermore, the medium in the adhesion culture may contain at least one selected from the group consisting of L-ascorbic acid, insulin, transferrin, selenium, and sodium hydrogen carbonate, or may contain all of L-ascorbic acid, insulin, transferrin, selenium, and sodium hydrogen carbonate.

The medium in the adhesion culture may contain FGF2. When the medium in the adhesion culture contains FGF2, an upper limit of a concentration of FGF2 is preferably, for example, 100 ng/mL or less, 90 ng/mL or less, 80 ng/mL or less, 70 ng/mL or less, 60 ng/mL or less, 50 ng/mL or less, 40 ng/mL or less, 30 ng/mL or less, 20 ng/mL or less, 10 ng/mL or less, 9 ng/mL or less, 8 ng/mL or less, 7 ng/mL or less, 6 ng/mL or less, 5 ng/mL or less, 4 ng/mL or less, 3 ng/mL or less, 2 ng/mL or less, or 1 ng/mL or less. Furthermore, a lower limit is preferably 0.1 ng/mL or more. By culturing in the presence of FGF2, it is possible to maintain undifferentiated state of pluripotent stem cells, and to promote cell growth.

The medium in the adhesion culture may contain a Rho-associated kinase (ROCK; Rho-binding kinase) inhibitor. By culturing in the presence of a ROCK inhibitor, it is possible to promote formation and growth of cell aggregates (spheroids) of pluripotent stem cells.

A ROCK inhibitor is defined as a substance inhibiting the kinase activity of Rho-kinase (Rho-associated protein kinase: ROCK). Examples thereof include Y-27632 (4-[(1R)-1-aminoethyl]-N-pyridin-4-ylcyclohexane-1-carboxamide) or dihydrochloride thereof (refer to, for example, Ishizaki et al., Mol. Pharmacol. 57, 976-983 (2000); Narumiya et al., Methods Enzymol. 325, 273-284 (2000)), Fasudil/HA1077 (1-(5-isoquinolinesulfonyl)homopiperazine) or dihydrochloride thereof (refer to, for example, Uenata et al., Nature 389: 990-994 (1997)), H-1152 ((S)-(+)-2-methyl-1-[(4-methyl-5-isoquinolinyl)sulfonyl]-hexahydro-1H-1,4-diazepine) or dihydrochloride thereof (refer to, for example, Sasaki et al., Pharmacol. Ther. 93: 225-232 (2002)), Wf-536 ((+)-(R)-4-(1-aminoethyl)-N-(4-pyridyl)benzamide monochloride) (refer to, for example, Nakajima et al., CancerChemother. Pharmacol. 52 (4): 319-324 (2003)), and derivatives of them; and antisense nucleic acids against ROCK, RNA interference-inducible nucleic acids (for example, siRNA), dominant negative mutants, and expression vectors of them. Furthermore, other low-molecular-weight compounds are also known as ROCK inhibitors, and therefore such compounds or derivatives thereof can also be used in the present invention (for example, refer to United States Patent Application, Publication No. 20050209261, United States Patent Application, Publication No. 20050192304, United States Patent Application, Publication No. 20040014755, United States Patent Application, Publication No. 20040002508, United States Patent Application, Publication No. 20040002507, United States Patent Application, Publication No. 20030125344, United States Patent Application, Publication No. 20030087919, PCT International Publication No. WO2003/062227, PCT International Publication No. WO2003/059913, PCT International Publication No. WO2003/062225, PCT International Publication No. WO2002/076976, and PCT International Publication No. WO2004/039796). At least one ROCK inhibitor can be used in the present invention.

When the medium contains a ROCK inhibitor such as Y-27632, a lower limit of a concentration of the ROCK inhibitor is preferably 0.1 µM or more, 0.2 µM or more, 0.5 µM or more, 1 µM or more, 2 µM or more, 3 µM or more, 4 µM or more, 5 µM or more, 6 µM or more, 7 µM or more, 8 µM or more, 9 µM or more, or 10 µM or more. An upper limit of a concentration of the ROCK inhibitor is preferably 200 µM or less, 150 µM or less, 100 µM or less, 90 µM or less, 80 µM or less, 70 µM or less, 60 µM or less, 50 µM or less, 40 µM or less, 30 µM or less, 20 µM or less, or 15 µM or less.

The adhesion culture in the present invention may include a step of culturing pluripotent stem cells in the presence of a WNT inhibitor. Examples of WNT inhibitors include XAV939, IWR-1-endo, IWR-1-exo, ICG-001, Ant 1.4Br, Ant 1.4Cl, Niclosamide, apicularen, bafilomycin, G007-LK, G244-LM, pyrvinium, NSC668036, 2,4-diamino-quinazoline, Quercetin, PKF115-584, BC2059, Shizokaol D, Dkk-1, and the like. When a WNT inhibitor such as XAV939 is used, a concentration added in the medium is not particularly limited, but a lower limit is preferably 1 nM or more, 10 nM or more, 0.1 µM or more, 0.2 µM or more, 0.5 µM or more, 1 µM or more, 2 µM or more, 3 µM or more, 4 µM or more, 5 µM or more, 6 µM or more, 7 µM or more, 8 µM or more, 9 µM or more, 10 µM or more, or 15 µM or more. An upper limit of a concentration of the WNT inhibitor is preferably 200 µM or less, 150 µM or less, 100 µM or less, 90 µM or less, 80 µM or less, 70 µM or less, 60 µM or less, 50 µM or less, 40 µM or less, 30 µM or less, or 25 µM or less.

As pluripotent stem cells for the adhesion culture, pluripotent stem cells prepared by a conventional method can be used. For example, pluripotent stem cells can be released by, for example, a mechanical, chemical, or biological method after the above-described maintenance culture, and seeded in a medium for the adhesion culture. It is possible to release cells using, as a cell releasing solution, for example, EDTA, TryPLE^{™} Select, Accutase^{™}, collagenase, DISPASE, trypsin, trypsin/EDTA, trypsin/collagenase, ReLeSR^{™} (STEMCELL Technologies Inc.), or the like.

A culture container for the adhesion culture is not particularly limited, and it is possible to use a plate for adhesion culture, or the like. The culture container is not particularly limited, and a culture container that has been subjected to artificial treatment to improve adhesiveness to cells (for example, coating treatment with an extracellular matrix or the like) may be used. The adhesion culture of pluripotent stem cells can be performed on, for example, a plate coated with a cell adhesion protein such as vitronectin, fibronectin, laminin, or matrigel.

When performing the adhesion culture, it is possible to perform the adhesion culture by seeding pluripotent stem cells in a medium at a cell density of, for example, 1 × 10³ cells to 1 × 10⁶ cells/mL, preferably 1 × 10⁴ cells to 1 × 10⁵ cells/mL, and more preferably 2 × 10⁴ cells to 1 × 10⁵ cells/mL.

A culture temperature in the adhesion culture is not particularly limited, but it is preferably from 36.0°C to 38.0°C, and more preferably from 36.5°C to 37.5°C. It is preferable to perform culture at an atmospheric CO₂ concentration of about 1% to 10%, preferably 5%, using a CO₂ incubator or the like.

A culture period of the adhesion culture is not particularly limited, but a lower limit of the culture period may be 1 day or longer, 2 days or longer, 3 days or longer, 4 days or longer, 5 days or longer, 6 days or longer, 7 days or longer, 8 days or longer, 9 days or longer, or 10 days or longer, and an upper limit of the culture period may be 30 days or shorter, 29 days or shorter, 28 days or shorter, 27 days or shorter, 26 days or shorter, or 25 days or shorter. The adhesion culture may be performed, for example, every 1 to 7 days, every 2 to 5 days, every 3 to 5 days, or every 3 to 4 days while passaging cells. The number of passages in the adhesion culture is not particularly limited.

When performing the adhesion culture, it is preferable to perform medium exchange at an appropriate frequency. A frequency of medium exchange is not particularly limited, and a frequency of medium exchange can be appropriately adjusted depending on the cell type and culture conditions. A medium exchange operation can be performed at a frequency of, for example, preferably one time or more every 5 days, one time or more every 4 days, one time or more every 3 days, one time or more every 2 days, or one time or more every 1 day. As a liquid medium used for the medium exchange, the same liquid medium as described above can be used. A method for medium exchange is not particularly limited. For example, preferably, maintenance culture can be continued by removing a supernatant by suction from a culture container with an aspirator, a pipette, or the like, thereafter, gently adding a fresh liquid medium, and thereafter, returning the culture container again to a culture environment such as a CO₂ incubator.

A passage method is not particularly limited. For example, preferably, it is possible to perform removing of a supernatant by suction from a culture container with an aspirator, a pipette, or the like, and thereafter washing if necessary. As a washing liquid, it is sufficient to use a buffer (including a PBS buffer), physiological saline, or a liquid medium (preferably a basal medium). For washed cells, the cells may be released by, for example, a mechanical, chemical, or biological method. For continuing the maintenance culture, the cells may be seeded in a fresh medium and a fresh culture container, which are for maintenance culture, and the maintenance culture may be continued. As a liquid medium and culture conditions used for the maintenance culture after passage, it is possible to use the same medium and conditions in the adhesion culture as described above. Furthermore, when releasing, cells may be released from a culture substrate or cells may be released from each other by using EDTA, TryPLE^{™} Select, Accutase^{™}, collagenase, DISPASE, trypsin, trypsin/EDTA, trypsin/collagenase, ReLeSR^{™}, or the like as a cell releasing solution; or cells may be released from a culture substrate using a cell scraper or the like. Released cells may be sufficiently dispersed and isolated by pipetting or using a strainer, or may be seeded in a colony form without being dispersed.

### (Suspension culture)

In the present invention, pluripotent stem cells may be suspension-cultured in a medium. Suspension culture is to culture cells in a state of being non-adherent to a culture container such as a culture dish. The form of suspension culture is not particularly limited as long as cells are cultured in a state of being non-adherent to a culture container. For example, cells adhered to a microcarrier or the like may be cultured, or suspension culture may be performed in the form of a cell aggregate in which a plurality of cells are adhered to each other to form a single mass as will be described later. Furthermore, a polymer such as collagen can be mixed into the cell aggregate. Suspension culture, in which cells are cultured while being suspended in a liquid medium as described above, is easily scaled up, and thus is expected to be suitable for mass production of cells.

The medium in the suspension culture is not particularly limited as long as it is a medium that can maintain an undifferentiated state of pluripotent stem cells. Examples thereof include a medium containing, in a basal medium, one or more selected from the group consisting of a Basic fibroblast growth factor-2 (FGF2), a Transforming growth factor-β1 (TGF-β1), Activin A, IGF-1, MCP-1, IL-6, PAI, PEDF, IGFBP-2, a Leukemia inhibitory factor (LIF), and IGFBP-7; and the like. The factors exemplified above are known to have a property of maintaining an undifferentiated state of pluripotent stem cells. As the medium in the suspension culture, it is possible to use, for example, StemFit (registered trademark) (for example, StemFit (registered trademark) AK02N and the like) (Ajinomoto Co., Inc.), an Essential 8 medium (Life Technologies Japan Ltd.) (Thermo Fisher Scientific K.K.), STEMPRO (registered trademark) hESC SFM (Life Technologies Japan Ltd.), mTeSR1 (VERITAS Corporation), TeSR2 (VERITAS Corporation), and the like, but examples are not limited thereto.

Furthermore, into the medium in the suspension cell, an antibiotic such as penicillin, streptomycin, and amphotericin B may be added. Furthermore, the medium in the suspension culture may contain at least one selected from the group consisting of L-ascorbic acid, insulin, transferrin, selenium, and sodium hydrogen carbonate, or may contain all of L-ascorbic acid, insulin, transferrin, selenium, and sodium hydrogen carbonate. The medium in the suspension culture may contain components such as fatty acids or lipids, amino acids (for example, non-essential amino acids), vitamins, cytokines, antioxidant agents, 2-mercaptoethanol, pyruvic acid, buffering agents, inorganic salts, and phosphorylation enzyme inhibitors.

The medium in the suspension culture may contain FGF2. When the medium in the suspension culture contains FGF2, an upper limit of a concentration of FGF2 is preferably, for example, 100 ng/mL or less, 90 ng/mL or less, 80 ng/mL or less, 70 ng/mL or less, 60 ng/mL or less, 50 ng/mL or less, 40 ng/mL or less, 30 ng/mL or less, 20 ng/mL or less, 10 ng/mL or less, 9 ng/mL or less, 8 ng/mL or less, 7 ng/mL or less, 6 ng/mL or less, 5 ng/mL or less, 4 ng/mL or less, 3 ng/mL or less, 2 ng/mL or less, or 1 ng/mL or less. Furthermore, a lower limit is preferably, for example, 0.1 ng/mL or more. By culturing in the presence of FGF2, it is possible to maintain undifferentiated state of pluripotent stem cells, and to promote cell growth.

The medium in the suspension culture may contain a Rho-associated kinase (ROCK; Rho-binding kinase) inhibitor. By culturing in the presence of a ROCK inhibitor, it is possible to promote formation and growth of cell aggregates (spheroids) of pluripotent stem cells. Detailed descriptions of the ROCK inhibitor are as described above.

When the medium contains a ROCK inhibitor such as Y-27632, a lower limit of a concentration of the ROCK inhibitor is preferably 0.1 µM or more, 0.2 µM or more, 0.5 µM or more, 1 µM or more, 2 µM or more, 3 µM or more, 4 µM or more, 5 µM or more, 6 µM or more, 7 µM or more, 8 µM or more, 9 µM or more, or 10 µM or more. An upper limit of a concentration of the ROCK inhibitor is preferably 200 µM or less, 150 µM or less, 100 µM or less, 90 µM or less, 80 µM or less, 70 µM or less, 60 µM or less, 50 µM or less, 40 µM or less, 30 µM or less, 20 µM or less, or 15 µM or less.

The suspension culture in the present invention may include a step of culturing pluripotent stem cells in the presence of a WNT inhibitor. Examples of WNT inhibitors include XAV939, IWR-1-endo, IWR-1-exo, ICG-001, Ant 1.4Br, Ant 1.4Cl, Niclosamide, apicularen, bafilomycin, G007-LK, G244-LM, pyrvinium, NSC668036, 2,4-diamino-quinazoline, Quercetin, PKF115-584, BC2059, Shizokaol D, Dkk-1, and the like. When a WNT inhibitor such as XAV939 is used, a concentration added in the medium is not particularly limited, but a lower limit is preferably 0.1 µM or more, 0.2 µM or more, 0.5 µM or more, 1 µM or more, 2 µM or more, 3 µM or more, 4 µM or more, 5 µM or more, 6 µM or more, 7 µM or more, 8 µM or more, 9 µM or more, 10 µM or more, or 15 µM or more. An upper limit of a concentration of the WNT inhibitor is preferably 200 µM or less, 150 µM or less, 100 µM or less, 90 µM or less, 80 µM or less, 70 µM or less, 60 µM or less, 50 µM or less, 40 µM or less, 30 µM or less, or 25 µM or less.

As pluripotent stem cells for the suspension culture, pluripotent stem cells prepared by a conventional method can be used. For example, pluripotent stem cells can be released by, for example, a mechanical, chemical, or biological method after the above-described maintenance culture, and seeded in a medium for the suspension culture. It is possible to release cells using, as a cell releasing solution, for example, EDTA, TryPLE^{™} Select, Accutase^{™}, collagenase, DISPASE, trypsin, trypsin/EDTA, trypsin/collagenase, ReLeSR^{™} (STEMCELL Technologies Inc.), or the like. The cells are used in the suspension culture after sufficiently dispersing the cells. For dispersing the cells, the cells can be caused to pass through a strainer and dispersed into single cells.

A culture container for the suspension culture is not particularly limited, and it is possible to use a plate for suspension culture, a bioreactor, and the like. The culture container is not particularly limited. A culture container that has not been subjected to artificial treatment to improve adhesiveness to cells may be used (for example, coating treatment with an extracellular matrix or the like), or a culture container that has been subjected to treatment to artificially inhibit adhesion may be used (for example, coating treatment with polyhydroxyethyl methacrylic acid). Furthermore, the shape of the culture container is not particularly limited. For example, culture containers, which have a shape such as a dish shape, a flask shape, a well shape, a bag shape, and a spinner flask shape, are exemplary examples.

The suspension culture may be static culture or may be culture under a condition in which a liquid medium flows. When performing the static culture, for example, a viscosity of a medium, or the like may be utilized, or microwells having unevenness, or the like may be used. The culture under a condition in which a liquid medium flows may be culture under a condition in which a liquid medium is suspended using a spinner or the like, but is preferably culture under a condition in which a liquid medium flows to promote cell aggregation. Examples of the culture under a condition in which a liquid medium flows to promote cell aggregation include culture under a condition in which a liquid medium flows so that cells gather at one point due to stress (centrifugal force, centripetal force) caused by flow such as gyrating flow and rocking flow, and culture under a condition in which a liquid medium flows by linear reciprocating motion, where the culture utilizing gyrating flow and/or rocking flow is particularly preferable.

Gyratory culture (shaking culture) is performed by gyrating a culture container accommodating a liquid medium and cells along approximately a horizontal plane in a closed orbit such as a circle, an ellipse, a flat circle, or a flat ellipse. A gyrating speed is not particularly limited, but it is preferably 200 rpm or less, and it may be 150 rpm or less, 120 rpm or less, 115 rpm or less, 110 rpm or less, 105 rpm or less, 100 rpm or less, 95 rpm or less, or 90 rpm or less. A lower limit of a gyrating speed is not particularly limited, but it is preferably 1 rpm or more, and it may be 10 rpm or more, 50 rpm or more, 60 rpm or more, 70 rpm or more, 80 rpm or more, or 90 rpm or more. When a gyrating speed is within this range, cell aggregates having appropriate sizes are easily formed, and cells can suitably grow.

A gyrating width during the gyratory culture is not particularly limited, but it is preferably 1 mm or more, and it may be 10 mm or more, 20 mm or more, or 25 mm or more. An upper limit of the gyrating width is not particularly limited, but it is preferably 200 mm or less, and it may be 100 mm or less, 50 mm or less, 30 mm or less, or 25 mm or less. A radius of rotation during the gyratory culture is also not particularly limited, but it is preferably set such that a gyrating width is within the above-mentioned range. The radius of rotation is preferably 5 mm or more, and it may be 10 mm or more. An upper limit of the radius of rotation is not particularly premised, but it is preferably 100 mm or less, and it may be 50 mm or less. When a gyrating width is within this range, cell aggregates having appropriate sizes are easily formed, and cells can suitably grow.

Rocking culture is culture performed while flowing a liquid medium by rocking stirring. The rocking culture is performed by rocking a culture container accommodating a liquid medium and cells in a plane substantially vertical to a horizontal plane. A rocking speed is not particularly limited, but rocking can be performed, for example, 2 to 50 times per minute, preferably 4 to 25 times per minute (where one round trip is one time). A rocking angle is not particularly limited, but it can be, for example, 0.1° to 20°, and can be more preferably 2° to 10°.

Furthermore, culture can also be performed while stirring by a motion in which the above-described gyrating and rocking are combined.

Culture using a spinner flask-shaped culture container is culture performed while stirring a liquid medium using a stirring blade in the culture container. A rotation speed and an amount of a medium are not particularly limited. In a case of a commercially available spinner flask-shaped culture container, an amount of a culture solution recommended by the manufacturer can be suitably used. A rotation speed is not particularly limited, but it can be, for example, 10 rpm or more and 300 rpm or less.

When performing the suspension culture, it is possible to appropriately adjust a seeding density of pluripotent stem cells in the medium (cell density at the start of the suspension culture). A lower limit of the seeding density is preferably, for example, 1 × 10⁴ cells/mL or more, 2 × 10⁴ cells/mL or more, or 1 × 10⁵ cells/mL or more, and an upper limit thereof is preferably, for example, 1 × 10⁷ cells/mL or less, or 1 × 10⁶ cells/mL or less. When a seeding density is within this range, cell aggregates having appropriate sizes are easily formed, and cells can suitably grow.

An amount of the medium at the time of the suspension culture can be appropriately adjusted depending on culture containers used. For example, when using a 12-well plate (where the area of a bottom surface of the well in a plan view per well is 3.5 cm²), an amount of the medium can be 0.5 mL/well or more and 1.5 mL/well or less, and can be more preferably 1 mL/well. For example, when using a 6-well plate (where the area of a bottom surface of the well in a plan view per well is 9.6 cm²), a lower limit of an amount of the medium is preferably 1.5 mL/well or more, and it may be 2 mL/well or more, or 3 mL/well or more; and an upper limit of an amount of the medium is preferably 6.0 mL/well or less, and it may be 5 mL/well or less, or 4 mL/well or less. For example, when using a 125 mL Erlenmeyer flask (Erlenmeyer flask having a capacity of 125 mL), a lower limit of an amount of the medium is preferably 10 mL/container or more, and it may be 30 mL/container or more; and an upper limit of an amount of the medium may be preferably 50 mL/container or less. For example, when using a 500 mL Erlenmeyer flask (Erlenmeyer flask having a capacity of 500 mL), a lower limit of an amount of the medium is preferably 100 mL/container or more, and it may be 120 mL/container or more; and an upper limit of an amount of the medium is preferably 150 mL/container or less, and it may be 125 mL/container or less. For example, when using a 1000 mL Erlenmeyer flask (Erlenmeyer flask having a capacity of 1000 mL), a lower limit of an amount of the medium is preferably 250 mL/container or more, and it may be 290 mL/container or more; and an upper limit of an amount of the medium is preferably 350 mL/container or less, and it may be 310 mL/container or less. For example, in a case of a 2000 mL Erlenmeyer flask (Erlenmeyer flask having a capacity of 2000 mL), a lower limit of an amount of the medium is preferably 500 mL/container or more, and it may be 600 mL/container or more; and an upper limit of an amount of the medium is preferably 1000 mL/container or less, and it may be 700 mL/container or less. For example, in a case of a 3000 mL Erlenmeyer flask (Erlenmeyer flask having a capacity of 3000 mL), a lower limit of an amount of the medium is preferably 1000 mL/container or more, and it can be 1500 mL/container or more; and an upper limit of an amount of the medium is preferably 2000 mL/container or less, and it may be 1600 mL/container or less. For example, in a case of a 2 L culture bag (disposable culture bag having a capacity of 2 L), a lower limit of an amount of the medium is preferably 100 mL/bag or more, and it may be 1000 mL/bag or more; and an upper limit of an amount of the medium is preferably 2000 mL/bag or less, and it may be 1100 mL/bag or less. For example, in a case of a 10 L culture bag (disposable culture bag having a capacity of 10 L), a lower limit of an amount of the medium is preferably 500 mL/bag or more, and it may be 5 L/bag or more; and an upper limit of an amount of the medium is preferably 10 L/bag or less, and it may be 6 L/bag or less. For example, in a case of a 20 L culture bag (disposable culture bag having a capacity of 20 L), a lower limit of an amount of the medium is preferably 1 L/bag or more, and it may be 10 L/bag or more; and an upper limit of an amount of the medium is preferably 20 L/bag or less, and it may be 11 L/bag or less. For example, in a case of a 50 L culture bag (disposable culture bag having a capacity of 50 L), a lower limit of an amount of the medium is preferably 1 L/bag or more, and it may be 25 L/bag or more; and an upper limit of an amount of the medium is preferably 50 L/bag or less, and it may be 30 L/bag or less. When an amount of a culture solution is within this range, cell aggregates having appropriate sizes are easily formed, and cells can suitably grow.

A capacity of a culture container used can be appropriately selected and is not particularly limited, but it is preferably 0.32 cm² or more, and it may be 0.65 cm² or more, 1.9 cm² or more, 3.0 cm² or more, 3.5 cm² or more, 9.0 cm² or more, or 9.6 cm² or more as a lower limit of an area when a bottom surface of a portion accommodating a liquid medium is viewed in a plan view. An upper limit of the area is preferably 1000 cm² or less, and it may be 500 cm² or less, 300 cm² or less, 150 cm² or less, 75 cm² or less, 55 cm² or less, 25 cm² or less, or 21 cm² or less.

A culture temperature in the suspension culture is not particularly limited, but it is preferably from 36.0°C to 38.0°C, and more preferably from 36.5°C to 37.5°C. It is preferable to perform culture at an atmospheric CO₂ concentration of about 1% to 10%, preferably 5%, using a CO₂ incubator or the like.

A culture period of the suspension culture is not particularly limited, but a lower limit of the culture period may be 1 day or longer, 2 days or longer, 3 days or longer, 4 days or longer, 5 days or longer, 6 days or longer, 7 days or longer, 8 days or longer, 9 days or longer, or 10 days or longer, and an upper limit of the culture period may be 30 days or shorter, 29 days or shorter, 28 days or shorter, 27 days or shorter, 26 days or shorter, or 25 days or shorter.

In the present invention, the step of performing suspension culture preferably includes a step of forming cell aggregates. A cell aggregate is a mass-like cell population formed by three-dimensionally aggregating a plurality of cells, and is also called a spheroid. A cell aggregate of pluripotent stem cells is formed from a cell population of pluripotent stem cells. Furthermore, a cell aggregate usually has a substantially spherical shape and generally has a diameter of about 30 µm to 2000 µm.

In the present invention, cell aggregates of pluripotent stem cells can be formed in a culture solution by the above-described suspension culture.

A dimension of a cell aggregate produced by the method of the present invention is not particularly limited, but in the case of observation with a microscope, an upper limit of the dimension of the widest portion in an observation image is preferably, for example, 1000 µm or less, 900 µm or less, 800 µm or less, 700 µm or less, 600 µm or less, 500 µm or less, 400 µm or less, or 300 µm or less. A lower limit of the dimension is preferably, for example, 30 µm or more, 40 µm or more, 50 µm or more, 60 µm or more, 70 µm or more, 80 µm or more, 90 µm or more, or 100 µm or more. A cell aggregate having such a dimensional range is preferable as a cell growth environment because oxygen and nutrient components are easily supplied to cells inside.

In a group of cell aggregates formed by the present invention, for example, 10% or more, 20% or more, 30% or more, 40% or more, 50% or more, 60% or more, 70% or more, 80% or more, or 90% or more of the cell aggregates constituting the group based on a weight can have a dimension within the above-mentioned range. In a group of cell aggregates which contains 20% or more of cell aggregates having a dimension within the above-mentioned range, each of individual cell aggregates is preferable as a cell growth environment because oxygen and nutrient components are easily supplied to cells inside.

Furthermore, in a cell aggregate formed by the present invention, a proportion (survival rate) of living cells among cells constituting the cell aggregate is preferably, for example, 50% or more, 60% or more, 70% or more, 80% or more, or 90% or more. A cell population in which a survival rate is within the above-mentioned range is in a preferable state for cell growth.

When performing the suspension culture, it is preferable to perform medium exchange at an appropriate frequency. A frequency of medium exchange is not particularly limited and varies depending on the cell type and culture conditions, but a medium exchange operation can be performed at a frequency of preferably one time or more every 5 days, one time or more every 4 days, one time or more every 3 days, one time or more every 2 days, or one time or more every 1 day. Medium exchange at these frequencies is particularly suitable when culturing cell aggregates of pluripotent stem cells used in the present invention. The same liquid medium as described above can be used as a liquid medium used for the medium exchange, and the same conditions as described above can be used as culture conditions. A method for medium exchange is not particularly limited. For example, preferably, a total amount of a culture solution containing cell aggregates is recovered in a centrifuge tube, and is subjected to centrifugal separation or left to stand in a static state for 5 minutes; the supernatant is removed while leaving the precipitated cell aggregates; a fresh liquid medium is added thereafter to gently disperse the cell aggregates; thereafter, the dispersed cells are again returned to a culture container such as a plate; and thereby culturing of the cell aggregates can be continued.

When performing the suspension culture, it is preferable to perform passage at an appropriate frequency. A frequency of passage is not particularly limited, but a passage operation can be performed at a frequency of preferably one time or more every 8 days, one time or more every 7 days, one time or more every 6 days, one time or more every 5 days, one time or more every 4 days, or one time or more every 3 days. Passage at these frequencies is particularly suitable when culturing cell aggregates of pluripotent stem cells used in the present invention. A passage method is not particularly limited. For example, preferably, a total amount of a culture solution containing cell aggregates is recovered in a centrifuge tube, and is subjected to centrifugal separation or left to stand in a static state for 5 minutes; the supernatant is removed while leaving the precipitated cell aggregates; and thereby cell aggregates can be recovered. Furthermore, the recovered cell aggregates can be washed if necessary. A washing method is not particularly limited, but for example, cell aggregates can be washed by adding a washing liquid to cell aggregates recovered in a centrifuge tube, precipitating the cell aggregates again by the above-described method, and removing the supernatant. The number of washing is not limited. As a washing liquid, it is possible to use a buffer (including a PBS buffer), physiological saline, or a liquid medium (preferably a basal medium). The recovered cell aggregates are isolated by for example, a mechanical, chemical, or biological method, and seeded in a fresh medium for the suspension culture. Thereby, the suspension culture can be restarted. As a liquid medium and culture conditions used for the suspension culture after the passage, it is possible to use the same liquid medium and conditions as described above. For example, by using a cell releasing solution including EDTA, TryPLE^{™} Select, Accutase^{™}, collagenase, DISPASE, trypsin, trypsin/EDTA, trypsin/collagenase, ReLeSR^{™}, and the like as a release agent used for the isolation, cells are released from a culture substrate or released from each other. The cells are sufficiently dispersed by pipetting or using a strainer, and can be seeded in an isolated state.

After the suspension culture, cells are present in a suspended state in a culture solution. Accordingly, cell recovery can be achieved by removing a liquid component of the supernatant, from a static state or by centrifugal separation. Furthermore, as a cell recovery method, it is possible to select a filter, a hollow fiber separation membrane, or the like. When removing a liquid component from a static state, it is sufficient to leave a container containing a culture solution in a static state for about 5 minutes to remove the supernatant while leaving the precipitated cells and cell aggregates. Furthermore, it is sufficient for centrifugal separation to be performed at a rotation speed and a treatment time at which cells are not damaged due to the centrifugal force. For example, a lower limit of the rotation speed is not particularly limited as long as cells can be precipitated, but the lower limit can be, for example, 100 rpm or more, 500 rpm or more, 800 rpm or more, or 1000 rpm or more. Meanwhile, it is sufficient for an upper limit to be a speed at which cells are not damaged or are unlikely to be damaged due to centrifugal force. The upper limit can be, for example, 1400 rpm or less, 1500 rpm or less, or 1600 rpm or less. Furthermore, a lower limit of the treatment time is not particularly limited as long as it is a time in which cells can be precipitated at the above-mentioned rotation speed. The lower limit can be, for example, 10 seconds or longer, 30 seconds or longer, 1 minute or longer, 3 minutes or longer, or 5 minutes or longer. Furthermore, it is sufficient for an upper limit of the treatment time to be a time during which cells are not damaged or are unlikely to be damaged by the rotation. The upper limit can be, for example, 30 seconds or shorter, 6 minutes or shorter, 8 minutes or shorter, or 10 minutes or shorter. The recovered cells can be washed if necessary. A washing method is not particularly limited. For example, washing may be performed in the same manner as the washing method described in the "passage method" in the above-described suspension culture step. As a washing liquid, it is possible to use a buffer (including a PBS buffer), physiological saline, or a liquid medium (preferably a basal medium).

In the present invention, cell aggregates of pluripotent stem cells can be formed in a culture solution by the above-described suspension culture.

Pluripotent stem cells constituting a cell aggregate preferably retain an undifferentiated state. The undifferentiated state of the cells can be confirmed by analyzing an expression state of an undifferentiation marker. Analysis of the expression state of the undifferentiation marker can be performed by, for example, quantitative real-time PCR analysis, flow cytometry analysis, or the like. Examples of undifferentiation markers include, but are not limited to, Oct4, Sox2, Nanog, SSEA-3, SSEA-4, TRA-1-60, TRA-1-81, REX-1, LIN28, LEFTB, GDF3, ZFP42, FGF4, ESG1, DPPA2, TERT, KLF4, c-Myc, and the like. Among them, Oct4, Sox2, and Nanog are preferable as the undifferentiation marker. The undifferentiation marker is synonymous with a pluripotent stem cell marker, and both terms can be used interchangeably.

A percentage of cells positive for Oct4 in a cell aggregate is preferably 90% or more, and it may be 91% or more, 92% or more, 93% or more, 94% or more, or 95% or more.

A percentage of cells positive for Sox2 in a cell aggregate is preferably 90% or more, and it may be 91% or more, 92% or more, 93% or more, 94% or more, 95% or more, 96% or more, 97% or more, 98% or more, or 99% or more.

A percentage of cells positive for Nanog in a cell aggregate is preferably 90% or more, and it may be 91% or more, 92% or more, 93% or more, 94% or more, 95% or more, or 96% or more.

The method of the present invention may include a step of sorting out a cell population having the following characteristics (a) and (b).
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

Sorting out a cell population having the characteristics (a) and (b) may be identifying and acquiring a cell population having the characteristics (a) and (b) from a plurality of cell populations, or may be preparing a cell population having the characteristics (a) and (b) from a certain cell population. When identifying and acquiring a cell population having the characteristics (a) and (b) from a plurality of cell populations, it is sufficient to confirm whether each of the plurality of cell populations has the characteristics (a) and (b) to acquire a cell population having the characteristics
(a) and (b). Examples of methods for preparing a cell population having the characteristics (a) and
(b) from a certain cell population include physical methods such as FACS, cell sorters, and separation with magnetic beads; and chemical methods in which cells not satisfying the above-described indexes are selected under appropriate culture conditions, and a cell population satisfying the above-described indexes is purified. The method is not particularly limited thereto, and it is sufficient to appropriately select a method depending on the culture method and the like. For example, in the physical methods such as FACS, cell sorters, and magnetic beads, it is possible to separate cells expressing LEF1 using an anti-LEF1 antibody. Furthermore, examples of culture conditions for selecting cells not satisfying the indexes (a) and (b) include the culture conditions described above. More specifically, a cell population may be cultured in the presence of a factor by which an undifferentiated state of pluripotent stem cells is maintained. Examples of such factors include FGF2, a ROCK inhibitor, a WNT inhibitor, and the like. These factors can be added into a medium such that a concentration thereof in the medium is similar to the concentration described above.

Before the sorting out, a step of identifying a cell population containing pluripotent stem cells using the above-described characteristics as indexes may be included.

A timing for sorting out a cell population having the above-described characteristics (a) and (b) is not particularly limited. Examples thereof include before maintenance culture, during maintenance culture, after maintenance culture, before adhesion culture, during adhesion culture, after adhesion culture, before suspension culture, during suspension culture, after suspension culture, before recovery of cell populations, after recovery of cell populations, before differentiation induction, before production of cryopreservation stock of cells, and the like.

The present invention further relates to a production method for a cell population containing pluripotent stem cells, the method including:
a step of culturing a cell population containing pluripotent stem cells;
a step of confirming whether the cell population is a cell population having the above-described characteristics (a) and (b); and
a step of acquiring the cell population confirmed to be the cell population having the characteristics (a) and (b).

The step of culturing a cell population containing pluripotent stem cells can be performed in the same manner as described above. For example, in the step of culturing a population containing pluripotent stem cells, the cell population can be cultured in the presence of a factor by which an undifferentiated state of pluripotent stem cells is maintained. Examples of such factors include FGF2, a ROCK inhibitor, a WNT inhibitor, and the like. These factors can be added into a medium such that a concentration thereof in the medium is similar to the concentration described above.

The step of confirming whether a cell population containing pluripotent stem cells is a cell population having the above-described characteristics (a) and (b) can be performed by quantitative real-time PCR and flow cytometry analysis in the same manner as described above.

It can be said that the cell population having the characteristics (a) and (b) is a cell population having a high level of differentiation potential. Therefore, it is possible to obtain a cell population containing pluripotent stem cells which has a high level of differentiation potential by acquiring a cell population confirmed to have the characteristics (a) and (b).

### [Method for monitoring differentiation potential of pluripotent stem cells]

The present invention further relates to a method for monitoring differentiation potential of a cell population containing pluripotent stem cells using the following characteristics (a) and (b) as indexes.
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

Examples of steps requiring monitoring include a culturing step, and a step of cryopreserving a cell population acquired by the culturing.

In the culturing step, differentiation potential of a cell population containing pluripotent stem cells can be quickly grasped and predicted by measuring the indexes over time. For example, during culturing, the indexes (a) and (b) can be investigated by collecting a part of cultured cells. It can be understood that, in a cell population containing pluripotent stem cells satisfying the above-described indexes, a level of differentiation potential of the cell population is high. Meanwhile, when a culture state in which values deviate from the above-described indexes continues, it can be predicted that a level of differentiation potential of a cell population containing pluripotent stem cells is reduced. When it is read from the indexes that the differentiation potential is declining, a level of differentiation potential of a cell population containing pluripotent stem cells can be increased by appropriately changing culture conditions (addition or change of media or additives, and the like) as necessary. For example, in order to improve differentiation potential of pluripotent stem cells, pluripotent stem cells may be cultured in the presence of a factor for maintaining an undifferentiated state. Examples of such factors include FGF2, a ROCK inhibitor, a WNT inhibitor, and the like. These factors can be added into a medium such that a concentration thereof in the medium is similar to the concentration described above. In an initial stage of culturing, it is sufficient for culture conditions (seeding density, media, additives, and the like) to be designed to satisfy the above-described indexes at a final stage of the step. In at least a final stage, it is sufficient for the above-described indexes to be satisfied.

When the above-described indexes are not satisfied in the final stage of culturing, a cell population containing pluripotent stem cells and satisfying the above-described indexes can be sorted out by utilizing, for example, a cell sorting technique using an anti-LEF1 antibody.

The present invention still further relates to a method for evaluating differentiation potential of a cell population containing pluripotent stem cells using the following characteristics (a) and (b) as indexes.
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

For example, when the above-described indexes (a) and (b) are confirmed for a cell population that is an evaluation target, and the indexes (a) and (b) are satisfied, the cell population is evaluated to have a high level of differentiation potential. On the other hand, when the above-described indexes (a) and (b) are not satisfied, the cell population can be evaluated to have a low level of differentiation potential.

Examples of cell populations that are evaluation targets include a cell population used in a production method for somatic cells to be described later.

The present invention still further relates to a method for monitoring an undifferentiated state of a cell population containing pluripotent stem cells using the following characteristics (a) and (b) as indexes.
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

The present invention still further relates to a method for evaluating an undifferentiated state of a cell population containing pluripotent stem cells using the following characteristics (a) and (b) as indexes.
(a) A relative expression level of a LEF1 gene with respect to an expression level of a β-actin gene is 5.5 × 10⁻⁴ or less in the cell population.
(b) A percentage of pluripotent stem cells positive for LEF1 is 55% or less in the cell population.

### [Method for producing somatic cells]

The present invention relates to a production method for somatic cells, the method including culturing a cell population of the present invention in the presence of a differentiation-inducing factor to cause the cell population to differentiate into somatic cells.

Examples of the differentiation-inducing factor used in the present invention include a substance that acts on TGFβ signaling, a substance that acts on WNT signaling, a substance that acts on Hedgehog signaling, a substance that acts on BMP signaling, and a substance that acts on Nodal/Activin signaling. Specifically, it is possible to use differentiation-inducing factors disclosed in PCT International Publication No. WO2016/063986, PCT International Publication No. WO2012/020845, and PCT International Publication No. WO2016/060260.

Furthermore, since the pluripotent stem cell of the present invention maintains an undifferentiated state, it is possible to efficiently induce differentiation into a desired somatic cell. Any differentiation-inducing medium can be used for inducing differentiation of pluripotent stem cells into desired cells. As the differentiation-inducing medium, it is possible to use, for example, a neuronal differentiation medium, an osteoblast differentiation medium, a cardiomyocyte differentiation medium, an adipocyte differentiation medium, an intestinal epithelial cell differentiation medium, and the like, but the differentiation-inducing medium is not particularly limited. As the differentiation-inducing medium, it is possible to use an ectoderm differentiation medium, a mesoderm differentiation medium, or an endoderm differentiation medium. A desired somatic cell can be prepared by culturing the pluripotent stem cells of the present invention using a differentiation medium suitable for inducing differentiation into a desired somatic cell.

When it is intended to induce differentiation from pluripotent stem cells into endoderm cells, it is possible to use, for example, a method disclosed in Production of pancreatic hormone-expressing endocrine cells from human embryonic stem cells, by D'Amour et al., Nat Biotechnol. 2006 Nov; 24 (11): 1392-401, which is the document showing that human ES cells can be induced to differentiate into endoderm cells by culturing them in a medium containing WNT3a and activin A.

When it is intended to induce differentiation from pluripotent stem cells into mesoderm cells, it is possible to use, for example, a method disclosed in Published Japanese Translation No. 2013-530680 of the PCT International Publication. Published Japanese Translation No. 2013-530680 of the PCT International Publication discloses a method for producing intermediate mesoderm cells from human pluripotent stem cells, the method including a step (i) of culturing human pluripotent stem cells in a medium containing activin A and WNT, and a step (ii) of culturing the cells obtained in the step (i) in a medium containing BMP and WNT or a functional equivalent of WNT. Furthermore, J Am Soc Nephrol 25: 1211-12225, 2015 by Albert Q Lam et al. discloses that it is possible to efficiently induce differentiation into mesoderm cells by treating human pluripotent stem cells with CHIR 99021 which is a GSK3β inhibitor and thereafter treating them with FGF2 and retinoic acid. Also in the present invention, pluripotent stem cells can be induced to differentiate into mesoderm cells by using the differentiation-inducing factors described in the above documents.

When it is intended to induce differentiation from pluripotent stem cells into ectoderm cells, it is possible to adopt, for example, a method for culturing pluripotent stem cells in a medium containing a BMP inhibitor (Noggin or the like) and a TGFβ/activin inhibitor (Chambers SM. et al., Nat Biotechnol. 27, 275-280 (2009)), and a method for culturing pluripotent stem cells in a medium containing a BMP inhibitor (Noggin or the like) and a Nodal/activin inhibitor (Beata Surnacz et al., Stem Cells, 2012; 30: 1875-1884).

Culture conditions for differentiation induction are not particularly limited as long as they are conditions under which animal cells can be cultured, but for example, it is possible to use the same conditions as for the above-described maintenance culture of pluripotent stem cells. A culture temperature for differentiation induction is not particularly limited, but it is preferably from 36.0°C to 38.0°C, and more preferably from 36.5°C to 37.5°C. It is preferable to perform culture at an atmospheric CO2 concentration of about 1% to 10%, preferably 5%, using a CO₂ incubator or the like.

Endoderm cells have an ability to differentiate into tissues of organs such as the digestive tract, lungs, thyroid, pancreas, and liver; secretory gland cells that open into the digestive tract; peritoneum; pleura; larynx; eustachian tube; trachea; bronchus; urinary tract (bladder, most parts of the urethra, a part of the ureter); and the like, and may be generally referred to as definitive endoderm (DE). Differentiation from pluripotent stem cells into endoderm cells can be confirmed by measuring an expression level of a gene specific to endoderm cells. Examples of genes specific to endoderm cells include SOX17, FOXA2, CXCR4, AFP, GATA4, EOMES, and the like.

An expression level of SOX17 in endoderm cells obtained by differentiation induction is preferably 1.0 × 10⁻⁴ or more, 1.0 × 10⁻³ or more, or 1.0 × 10⁻² or more as a relative gene expression level with respect to ACTB (β-Actin) that is a housekeeping gene.

An expression level of FOXA2 in endoderm cells obtained by differentiation induction is preferably 1.0 × 10⁻⁵ or more, 1.0 × 10⁻⁴ or more, or 1.0 × 10⁻³ or more as a relative gene expression level with respect to ACTB (β-Actin) that is a housekeeping gene.

An expression level of CXCR4 in endoderm cells obtained by differentiation induction is preferably 1.0 × 10⁻³ or more, 1.0 × 10⁻³ or more, or 1.0 × 10⁻¹ or more as a relative gene expression level with respect to ACTB (β-Actin) that is a housekeeping gene.

Mesoderm cells are differentiated into body cavity and the mesothelium that lines the body cavity, muscles, skeleton, skin dermis, connective tissue, heart, blood vessels (including vascular endothelium), blood (including blood cells), lymph vessels, spleen, kidneys, ureter, gonads (testicles, uterus, gonadal epithelium), and the like. Examples of genes specific to mesoderm cells include MESP1, MESP2, FOXF1, BRACHYURY, HAND1, EVX1, IRX3, CDX2, CXCR4, TBX6, MIXL1, ISL1, SNAI2, FOXC1, VEGFR2, PDGFRα, and the like.

An expression level of CDX2 in mesoderm cells obtained by differentiation induction is preferably 1.0 × 10⁻⁵ or more, 1.0 × 10⁻⁴ or more, or 1.0 × 10⁻³ or more as a relative gene expression level with respect to ACTB (β-Actin) that is a housekeeping gene.

An expression level of CXCR4 in mesoderm cells obtained by differentiation induction is preferably 1.0 × 10⁻⁵ or more, 1.0 × 10⁻⁴ or more, or 1.0 × 10⁻³ or more as a relative gene expression level with respect to ACTB (β-Actin) that is a housekeeping gene.

An expression level of VEGFR2 in mesoderm cells obtained by differentiation induction is preferably 1.0 × 10⁻⁴ or more, 1.0 × 10⁻³ or more, or 1.0 × 10⁻² or more as a relative gene expression level with respect to ACTB (β-Actin) that is a housekeeping gene.

An expression level of PDGFRα in mesoderm cells obtained by differentiation induction is preferably 1.0 × 10⁻⁴ or more, 1.0 × 10⁻³ or more, or 1.0 × 10⁻² or more as a relative gene expression level with respect to ACTB (β-Actin) that is a housekeeping gene.

Ectoderm cells form epidermis of the skin and epithelium at the end portion of the urethra in men, hair, nails, skin glands (including mammary glands and sweat glands), sensory organs (salivary glands including oral cavity, pharynx, nose, epithelium at the end portion of rectum) crystalline lens, and the like. Some ectoderm cells invaginate into grooves during a development stage and form a neural tube to also become the origin of neurons and melanocytes of the central nervous system such as the brain and the spinal cord. Ectoderm cells also form the peripheral nervous system. Examples of genes specific to ectoderm cells include FGF5, OTX2, SOX1, NESTIN, PAX6, and the like.

An expression level of PAX6 in ectoderm cells obtained by differentiation induction is preferably 1.0 × 10⁻⁶ or more, 1.0 × 10⁻⁵ or more, or 3.8 × 10⁻⁵ or more as a relative gene expression level with respect to ACTB (β-Actin) that is a housekeeping gene.

An expression level of SOX1 in ectoderm cells obtained by differentiation induction is preferably 1.0 × 10⁻⁴ or more, 1.0 × 10⁻³ or more, or 1.0 × 10⁻² or more as a relative gene expression level with respect to ACTB (β-Actin) that is a housekeeping gene.

An expression level of NESTIN in ectoderm cells obtained by differentiation induction is preferably 1.0 × 10⁻³ or more, 1.0 × 10⁻² or more, or 5.0 × 10⁻² or more as a relative gene expression level with respect to ACTB (β-Actin) that is a housekeeping gene.

The somatic cells in the present invention can be differentiation-induced from a cell population containing pluripotent stem cells of the present invention. Types of somatic cells are not particularly limited as long as they are somatic cells that can be present in the biological body. Examples thereof include somatic stem cells (mesenchymal stem cells, neural stem cells, and the like, which are derived from bone marrow, adipose tissue, dental pulp, placenta, egg membrane, umbilical cord blood, amniotic membrane, chorion membrane, or the like), neuronal cells, glial cells, oligodendrocytes, Schwann cells, cardiac muscle cells, cardiac muscle progenitor cells, liver cells, hepatic progenitor cells, α cells, β cells, fibroblasts, cartilage cells, corneal cells, vascular endothelial cells, vascular endothelial progenitor cells, pericytes, skeletal muscle cells, megakaryocytes, hematopoietic stem cells, airway epithelial cells, germ cells, dendritic cells, eosinophils, mast cells, T cells, erythropoietin-producing cells, intestinal epithelium, alveolar epithelial cells, kidney cells, and the like. The somatic cells may be in a form in which a gene has been introduced into the above-mentioned cell, or a form in which a target gene or the like on the genome is knocked down or knocked out in the above-mentioned cell.

The present invention still further relates to a production method for somatic cells, the method including:
a step of culturing a cell population containing pluripotent stem cells;
a step of confirming whether the cell population is a cell population having the following characteristics (a) and (b); and
a step of culturing the cell population confirmed to be the cell population having the characteristics (a) and (b) in the presence of a differentiation-inducing factor.
   (a) A relative expression level of a LEF1 gene with respect to an expression level of a β-actin gene is 5.5 × 10⁻⁴ or less in the cell population.
   (b) A percentage of cells positive for LEF1 is 55% or less in the cell population.

The step of culturing a cell population containing pluripotent stem cells can be performed using the same medium and culture conditions as described above.

The step of confirming whether the cell population is a cell population having the above-described characteristics (a) and (b) can be performed by quantitative real-time PCR and flow cytometry analysis in the same manner as described above.

The step of culturing the cell population confirmed to be the cell population having the characteristics (a) and (b) in the presence of a differentiation-inducing factor can be performed using a differentiation-inducing factor described above depending on the type of somatic cell into which cells are induced to differentiate.

The present invention will be specifically described with reference to the following examples, but the present invention is not limited to the examples.

### [Examples]

### <Comparative Example 1>

A human iPS cell line 201B7 (Center for iPS Cell Research and Application, Kyoto University) was prepared by maintenance-culturing the cell line at 37°C under a 5% CO₂ atmosphere on a cell culture dish coated with Vitronectin (Thermo Fisher Scientific K.K.). StemFit (registered trademark) AK02N (Ajinomoto Co., Inc.) medium was used as a medium in the maintenance culture. The maintenance-cultured cells were treated with Accutase (Innovative Cell Technologies, Inc.) for 3 to 5 minutes to release the cells from the dish. The cells were dispersed into single cells by pipetting. These cells were suspended in StemFit (registered trademark) AK02N medium containing Y-27632 at a final concentration of 10 µM. A part of the cells was stained with trypan blue to measure the number of living cells. A cell suspension was prepared so that it contained 2 × 10⁵ cells per mL using the StemFit (registered trademark) AK02N medium containing Y-27632 a final concentration of 10 µM. 4 mL of the cell suspension per well was seeded in a 6-well plate for suspension culture (Sumitomo Bakelite Co., Ltd.). The plate in which the cells were seeded was gyrated on a rotary shaker (Optima Instruments) at a speed of 83 rpm so that a gyrating width (diameter) drew a circle of 25 mm along a horizontal plane, and suspension culture was performed at 37°C in a 5% CO₂ environment. The day when the cells were seeded was defined as day 0 of the culture. Passage was performed on day 5 of the culture, and suspension culture was performed until day 10 of the culture. As a medium exchange method, a total amount of the medium containing cell aggregates was recovered in a centrifuge tube and left to stand for about 5 minutes to precipitate the cell aggregates. Thereafter, the culture supernatant was removed. The cell aggregates were gently suspended again in StemFit (registered trademark) AK02N medium and returned to the original wells. At the time of medium exchange, Y-27632 was added into the medium on day 1 and day 6 of the culture so that a final concentration was 5 µM, and Y-27632 was added into the medium on day 2 and day 7 of the culture so that a final concentration was 2 µM. On day 5 of the culture, the cell aggregates and the culture supernatant were recovered in a centrifuge tube from the wells and left to stand for about 5 minutes to precipitate the cell aggregates and recover the culture supernatant. 1 mL of Accutase was added to the cell aggregates, and the cell aggregates were treated for 10 minutes. The cell aggregates were dispersed into single cells by pipetting. These cells were suspended in StemFit (registered trademark) AK02N medium containing Y-27632 at a final concentration of 10 µM. A part of the cells was stained with trypan blue to measure the number of living cells. A cell suspension was prepared so that it contained 2 × 10⁵ cells per mL using the medium containing Y-27632 at a final concentration of 10 µM. 4 mL of the cell suspension per well was seeded in a 6-well plate for suspension culture. The plate in which the cells were seeded was gyrated on a rotary shaker at a speed of 83 rpm so that a gyrating width (diameter) drew a circle of 25 mm along a horizontal plane, and suspension culture was continued at 37°C in a 5% CO₂ environment.

### <Comparative Example 2>

Maintenance culture and suspension culture were carried out in the same procedure as in Comparative Example 1 except that FGF2 (PeproTech, Inc.) was added into the StemFit (registered trademark) AK02N medium used at the time of seeding and medium exchange in the suspension culture, and that a final concentration of the added FGF2 was prepared to 50 ng/mL.

### <Comparative Example 3>

A human iPS cell line 201B7 was maintenance-cultured in the same procedure as in Comparative Example 1. Suspension culture was carried out in the same procedure as in Comparative Example 1 except that XAV939 (FUJIFILM Wako Pure Chemical Corporation) was added into the StemFit (registered trademark) AK02N medium, which was used at the time of seeding and medium exchange in the suspension culture, so that a final concentration was 20 µM.

### <Comparative Example 4>

Maintenance culture and suspension culture were carried out in the same procedure as in Comparative Example 1 except that a line RPChiPS771-2 was used for human iPS cells.

<Example 1>

Cells were maintenance-cultured in the same procedure as in Comparative Example 1. The maintenance-cultured cells were treated with Accutase for 3 to 5 minutes to release the cells from the dish. The cells were dispersed into single cells by pipetting. These cells were suspended in a StemFit (registered trademark) AK02N medium containing Y-27632 at a final concentration of 10 µM. A part of the cells was stained with trypan blue to measure the number of living cells. A cell suspension was prepared so that it contained 4.6 × 10⁴ cells per mL using the StemFit (registered trademark) AK02N medium containing Y-27632 at a final concentration of 10 µM. 2 mL of the cell suspension per well was seeded in a 6-well plate for cell culture (Sumitomo Bakelite Co., Ltd.) coated with Vitronectin at 0.5 µg/cm², and adhesion culture was performed at 37°C in a 5% CO₂ environment. The day when the cells were seeded was defined as day 0 of the culture. Passage was performed on day 5 of the culture, and adhesion culture was performed until day 10 of the culture. Medium exchange was performed every day with a medium free of Y-27632. On day 5 of the culture, the culture supernatant was recovered from the wells. 0.5 mL of Accutase was added per well to treat the cells for 3 to 5 minutes. The cells were released from the wells, and were dispersed into single cells by pipetting. These cells were suspended in a StemFit (registered trademark) AK02N medium containing Y-27632 at a final concentration of 10 µM. A part of the cells was stained with trypan blue to measure the number of living cells. A cell suspension was prepared so that it contained 4.6 × 10⁴ cells per mL using the StemFit (registered trademark) AK02N medium containing Y-27632 a final concentration of 10 µM. 2 mL of the cell suspension per well was seeded in a 6-well plate for cell culture coated with Vitronectin at 0.5 µg/cm², and adhesion culture was continued at 37°C under a 5% CO₂ atmosphere.

### <Example 2>

Maintenance culture and adhesion culture were carried out in the same procedure as in Example 1 except that an Essential 8^{™} medium (Thermo Fisher Scientific K.K.) was used for the maintenance culture and the adhesion culture.

### <Example 3>

Maintenance culture and adhesion culture were carried out in the same procedure as in Example 1 except that a line RPChiPS771-2 was used for human iPS cells.

### <Example 4>

Maintenance culture and adhesion culture were carried out in the same procedure as in Example 1 except that a line RPChiPS771-2 was used for human iPS cells, and that an Essential 8^{™} medium was used for the maintenance culture and the adhesion culture.

### <Example 5>

Maintenance culture and suspension culture were carried out in the same procedure as in Comparative Example 1 except that an Essential 8^{™} medium was used for the maintenance culture and the suspension culture.

### <Example 6>

Maintenance culture and suspension culture were carried out in the same procedure as in Comparative Example 1 except that a line RPChiPS771-2 was used for human iPS cells, and that an Essential 8^{™} medium was used for the maintenance culture and the suspension culture.

### <Example 7>

Maintenance culture and suspension culture were carried out in the same procedure as in Comparative Example 1 except that a line RPChiPS771-2 was used for human iPS cells, that FGF2 was added into the StemFit (registered trademark) AK02N medium used at the time of seeding and medium exchange in the suspension culture, and that a final concentration of the added FGF2 was prepared to 50 ng/mL.

### <Example 8>

Maintenance culture and suspension culture were carried out in the same procedure as in Comparative Example 1 except that a line RPChiPS771-2 was used for human iPS cells, and that XAV939 was added into the StemFit (registered trademark) AK02N medium, which was used at the time of seeding and medium exchange in the suspension culture, so that a final concentration was 20 µM.

### <Example 9: observation of cell morphology>

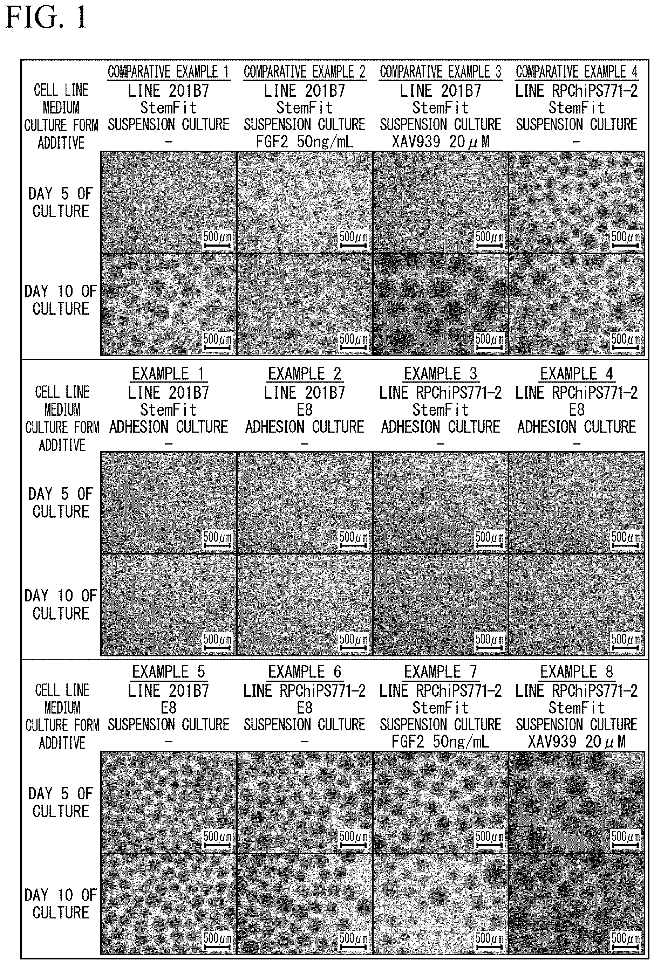

Fig. 1 shows phase-contrast images of cells acquired by using a phase-contrast microscope on day 5 of culture and day 10 of culture in Comparative Examples 1 to 4 and Examples 1 to 8.

### <Example 10: quantitative real-time PCR analysis>

Quantitative real-time PCR analysis was performed according to the procedure shown below. The cells on day 5 of the culture and day 10 of the culture which were obtained in Comparative Examples 1 to 4 and Examples 1 to 8 were treated with Accutase for 3 minutes to 10 minutes and dispersed into single cells by pipetting. These cells were lysed using TRIzol^{™} Reagent (Thermo Fisher Scientific K.K.). Total RNA was isolated and purified from a cell solution obtained by lysing with TRIzol^{™} Reagent using a PureLink (registered trademark) RNA Mini kit (Thermo Fisher Scientific K.K.). A concentration of the purified RNA was measured using BioSpec-nano (Shimadzu Corporation), and 40 ng was separated. 2 µL of ReverTra Ace (registered trademark) qPCR RT Master Mix (TOYOBO CO., LTD.) and RNase Free dH₂O were added to the separated RNA to prepare 10 µL. cDNA synthesis was performed using SimpliAmp Thermal Cycler (Thermo Fisher Scientific K.K.). Reaction conditions for the cDNA synthesis were as follows: a reaction was caused at 37°C for 15 minutes; thereafter, a reaction at 50°C for 5 minutes and a reaction at 98°C for 5 minutes were consecutively caused; and thereafter, cooling was performed to 4°C. A synthesized cDNA solution was diluted 100-fold with 10 mM Tris-HCl pH 8.0 (NACALAI TESQUE, INC.) and added to a 384-well PCR plate (Thermo Fisher Scientific K.K.) at 5 µL/well. KOD SYBR (registered trademark) qPCR Mix (TOYOBO CO., LTD.), a Forward primer prepared to 50 µM, a Reverse primer prepared to 50 µM, and DEPC-treated water (NACALAI TESQUE, INC.) were mixed at a ratio of 100:1:1:48. This mixed solution was added to the above-mentioned 384-well PCR plate at 15 µL/well and mixed. For the primers, ACTB, Oct4, Sox2, Nanog, Brachyury, Sox17, and Pax6 were used. The 384-well PCR plate was subjected to centrifugal separation to remove air bubbles in the wells, and quantitative real-time PCR analysis was performed using QuantStudio 7 Flex Real-Time PCR System (Thermo Fisher Scientific K.K.). Table 1 shows reaction conditions.

**[Table 1]**

| Step | Temperature | Time | Number of cycles |
|---|---|---|---|
| 1 Initial denaturation | 98°C | 1 min. | - |
| 2 Denaturation | 98°C | 15 sec. | 5 cycles |
| 3 Annealing and extension | 68°C | 30 sec. | |
| 4 Denaturation | 98°C | 15 sec. | 40 cycles |
| 5 Annealing | 60°C | 10 sec. | |
| 6 Extension | 68°C | 30 sec. | |
| 7 Melting curve | 95°C | 15 sec. | - |
| | 60°C | 1 min. | |
| | 98°C | 15 sec. | |

Detection was performed by an intercalation method using SYBR Green, and comparison of gene expression levels was performed by a comparative Ct method. An expression level of each gene was standardized by bACT, which is a housekeeping gene.

According to the procedure described above, quantitative real-time PCR analysis was performed on the cells on day 5 of the culture which were obtained in Comparative Examples 1 to 4 and Examples 1 to 8, and an expression level of a LEF1 gene was measured. Base sequences of the primers used in the quantitative real-time PCR analysis are as shown below (also shown in SEQ ID NOs: 1 to 16 in the sequence listing).

ACTB (F): 5'-CCTCATGAAGATCCTCACCGA-3' (SEQ ID NO: 1)
ACTB (R): 5'-TTGCCAATGGTGATGACCTGG-3' (SEQ ID NO: 2)
Oct4 (F): 5'-AGTGGGTGGAGGAAGCTGACAAC-3' (SEQ ID NO: 3)
Oct4 (R): 5'-TCGTTGTGCATAGTCGCTGCTTGA-3' (SEQ ID NO: 4)
Sox2 (F): 5'-CACCAATCCCATCCACACTCAC-3' (SEQ ID NO: 5)
Sox2 (R): 5'-GCAAAGCTCCTACCGTACCAC-3' (SEQ ID NO: 6)
Nanog (F): 5'-AGCCTCCAGCAGATGCAAGAACTC-3' (SEQ ID NO: 7)
Nanog (R): 5'-TTGCTCCACATTGGAAGGTTCCCA-3' (SEQ ID NO: 8)
Brachyury (F): 5'-TCACAAAGAGATGATGGAGGAAC-3' (SEQ ID NO: 9)
Brachyury (R): 5'-ACATGCAGGTGAGTTGTCAG-3' (SEQ ID NO: 10)
Sox17 (F): 5'-ATCTGCACTTCGTGTGCAAG-3' (SEQ ID NO: 11)
Sox17 (R): 5'-GAGTCTGAGGATTTCCTTAGCTC-3' (SEQ ID NO: 12)
Pax6 (F): 5'-AGGAATGGACTTGAAACAAGG-3' (SEQ ID NO: 13)
Pax6 (R): 5'-GCAAAGCTTGTTGATCATGG-3' (SEQ ID NO: 14)
LEF1 (F): 5'-AGAAAGTGCAGCTATCAACC-3' (SEQ ID NO: 15)
LEF1 (R): 5'- GAATGAGCTTCGTTTTCCAC-3' (SEQ ID NO: 16)

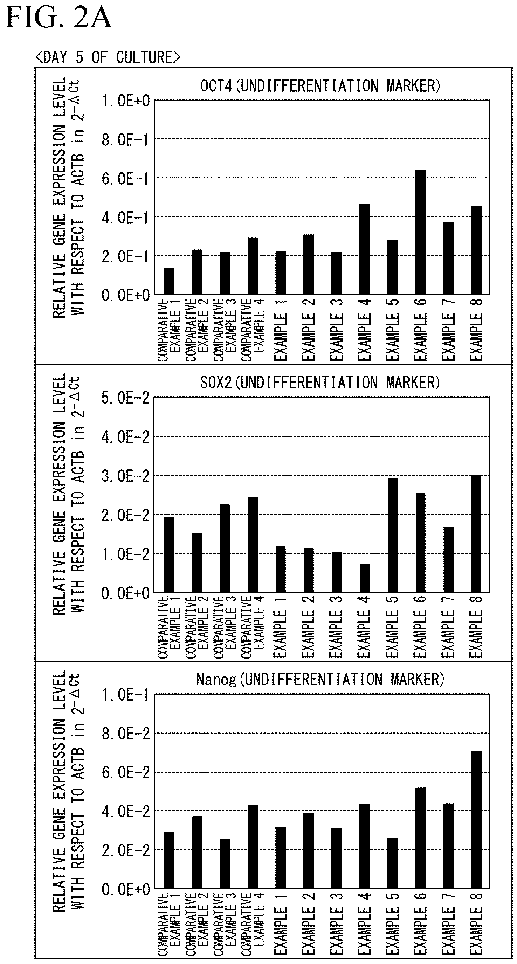

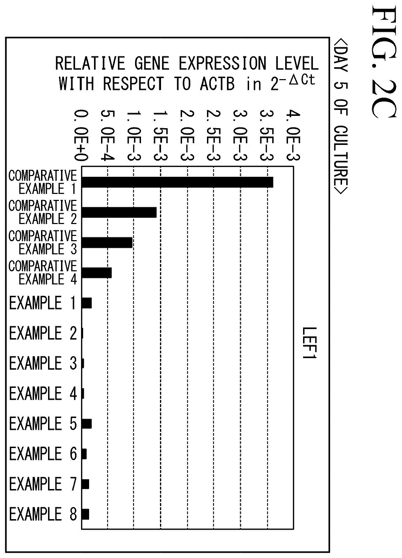

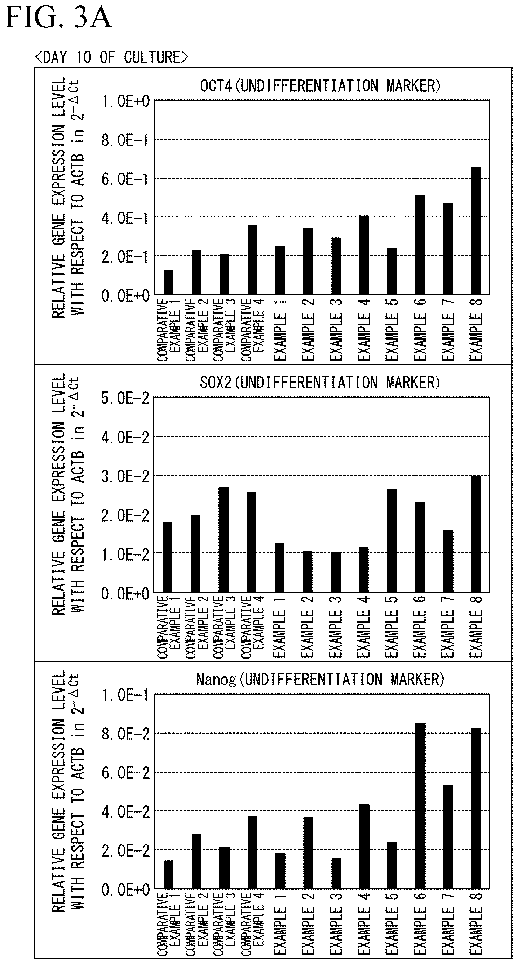

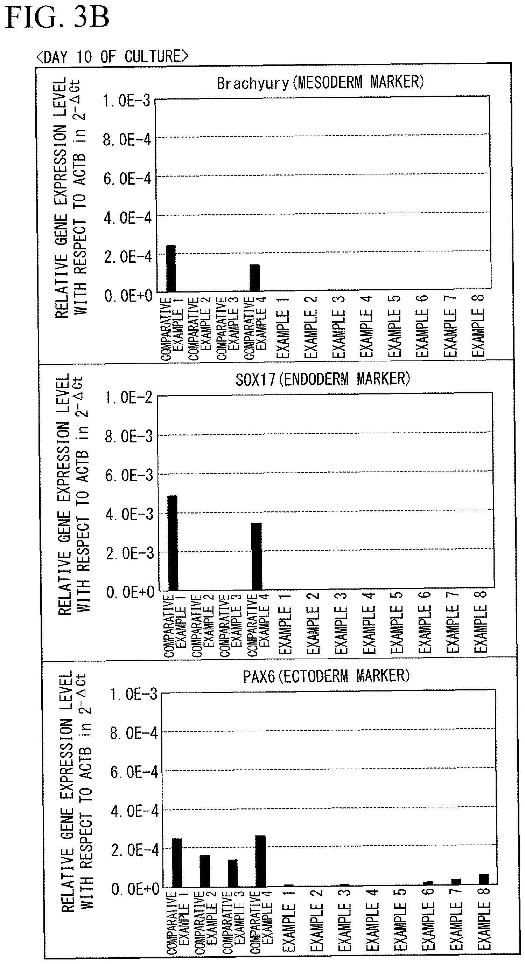

Tables 2 and 3, and Figs. 2A to 2C, 3A, and 3B show results of measuring gene expression levels.

**[Table 2]**

| | Relative gene expression level with respect to ACTB on day 5 of culture in 2^{-Δ Ct} | | | | | | |
|---|---|---|---|---|---|---|---|
| | Oct4 | Sox2 | Nanog | Brachyury | Sox17 | Pax6 | LEF1 |
| Comparative Example 1 | 1.36 × 10⁻¹ | 1.91 × 10⁻² | 2.90× 10⁻² | 2.20 × 10⁻⁵ | 1.53 × 10⁻⁵ | 9.48 × 10⁻⁶ | 3.61 × 10⁻³ |
| Comparative Example 2 | 2.28 × 10⁻¹ | 1.51 × 10⁻² | 3.68 × 10⁻² | - | - | 4.75 × 10⁻⁶ | 1.42 × 10⁻³ |
| Comparative Example 3 | 2.16 × 10⁻¹ | 2.25 × 10⁻² | 2.55 × 10⁻² | - | - | 2.78 × 10⁻⁶ | 9.67 × 10⁻⁴ |
| Comparative Example 4 | 2.91 × 10⁻¹ | 2.42 × 10⁻² | 4.26 × 10⁻² | - | - | 7.35 × 10⁻⁶ | 5.73 × 10⁻⁴ |
| Example 1 | 2.19 × 10⁻¹ | 1.17 × 10⁻² | 3.16 × 10⁻² | - | - | - | 1.91 × 10⁻⁴ |
| Example 2 | 3.05 × 10⁻¹ | 1.11 × 10⁻² | 3.85 × 10⁻² | - | - | - | 3.95 × 10⁻⁵ |
| Example 3 | 2.16 × 10⁻¹ | 1.03 × 10⁻² | 3.08 × 10⁻² | - | - | - | 4.25 × 10⁻⁵ |
| Example 4 | 4.62 × 10⁻¹ | 7.21 × 10⁻³ | 4.31 × 10⁻² | - | - | - | 5.12 × 10⁻⁵ |
| Example 5 | 2.78 × 10⁻¹ | 2.92 × 10⁻² | 2.61 × 10⁻² | - | - | 5.68 × 10⁻⁶ | 1.87 × 10⁻⁴ |
| Example 6 | 6.37 × 10⁻¹ | 2.52 × 10⁻² | 5.16 × 10⁻² | - | - | 4.99 × 10⁻⁶ | 9.83 × 10⁻⁵ |
| Example 7 | 3.72 × 10⁻¹ | 1.67 × 10⁻² | 4.34 × 10⁻² | - | - | 5.39 × 10⁻⁶ | 1.40 × 10⁻⁴ |
| Example 8 | 4.55 × 10⁻¹ | 3.01 × 10⁻² | 7.04 × 10⁻² | - | - | 8.96 × 10⁻⁶ | 1.47 × 10⁻⁴ |

**[Table 3]**

| | Relative gene expression level with respect to ACTB on day 10 of culture in 2^{-Δ Ct} | | | | | | |
|---|---|---|---|---|---|---|---|
| | Oct4 | Sox2 | Nanog | Brachyury | Sox17 | Pax6 | LEF1 |
| Comparative Example 1 | 1.23 × 10⁻¹ | 1.79 × 10⁻² | 1.44 × 10⁻² | 2.41 × 10⁻⁴ | 4.88 × 10⁻³ | 2.46 × 10⁻⁴ | 3.61 × 10⁻³ |
| Comparative Example 2 | 2.26 × 10⁻¹ | 1.96 × 10⁻² | 2.79 × 10⁻² | - | - | 1.61 × 10⁻⁴ | 1.42 × 10⁻³ |
| Comparative Example 3 | 2.04 × 10⁻¹ | 2.68 × 10⁻² | 2.15 × 10⁻² | - | - | 1.35 × 10⁻⁴ | 9.67 × 10⁻⁴ |
| Comparative Example 4 | 3.55 × 10⁻¹ | 2.56 × 10⁻² | 3.68 × 10⁻² | 1.36 × 10⁻⁴ | 3.44 × 10⁻³ | 2.60 × 10⁻⁴ | 5.73 × 10⁻⁴ |
| Example 1 | 2.49 × 10⁻¹ | 1.24 × 10⁻² | 1.80 × 10⁻² | - | - | 1.17 × 10⁻⁶ | 1.91 × 10⁻⁴ |
| Example 2 | 3.40 × 10⁻¹ | 1.04 × 10⁻² | 3.63 × 10⁻² | - | - | - | 3.95 × 10⁻⁵ |
| Example 3 | 2.90 × 10⁻¹ | 1.04 × 10⁻² | 1.57 × 10⁻² | - | - | 1.32 × 10⁻⁶ | 4.25 × 10⁻⁵ |
| Example 4 | 4.06 × 10⁻¹ | 1.15 × 10⁻² | 4.31 × 10⁻² | - | - | - | 5.12 × 10⁻⁵ |
| Example 5 | 2.38 × 10⁻¹ | 2.64 × 10⁻² | 2.36 × 10⁻² | - | - | - | 1.87 × 10⁻⁴ |
| Example 6 | 5.13 × 10⁻¹ | 2.30 × 10⁻² | 8.49 × 10⁻² | - | - | 1.38 × 10⁻⁵ | 9.83 × 10⁻⁵ |
| Example 7 | 4.72 × 10⁻¹ | 1.58 × 10⁻² | 5.30 × 10⁻² | - | - | 2.69 × 10⁻⁵ | 1.40 × 10⁻⁴ |
| Example 8 | 6.53 × 10⁻¹ | 2.95 × 10⁻² | 8.23 × 10⁻² | - | - | 4.97 × 10⁻⁵ | 1.47 × 10⁻⁴ |

In Comparative Examples 1 to 4 and Examples 1 to 8, it was recognized that expression levels of a differentiation marker gene and a LEF1 gene tended to be different.

Specifically, a relative expression level of a LEF1 gene with respect to an expression level of a β-actin gene on day 5 of the culture was 5.5 × 10⁻⁴ or more in the pluripotent stem cell populations obtained in Comparative Examples 1 to 4, but was 5.5 × 10⁻⁴ or less in the pluripotent stem cell populations obtained in Examples 1 to 8 (Tables 2 and 3).

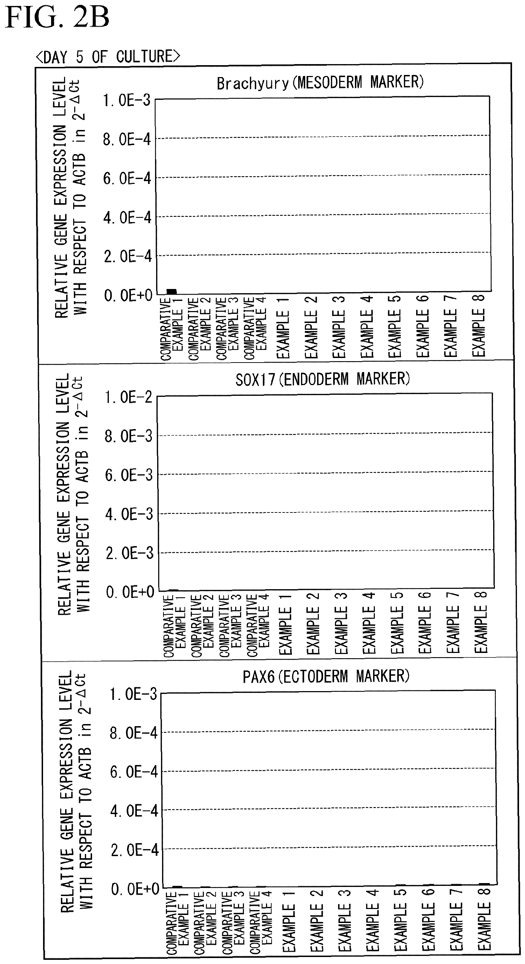

Furthermore, a relative expression level of the differentiation marker gene with respect to an expression level of the β-actin gene was low in all of Comparative Examples 1 to 4 and Examples 1 to 8 until day 5 of the culture, and no large difference between the conditions was recognized (Table 2 and Fig. 2B). However, the relative expression level of the differentiation marker gene with respect to the expression level of the β-actin gene increased significantly on day 10 of the culture as compared to day 5 of the culture in Comparative Example 1 to Comparative Example 4, whereas the relative expression level thereof on day 10 of the culture was as low as that on day 5 of the culture in Examples 1 to 8, meaning that there was almost no expression (Table 3 and Fig. 3B).

Based on these results, it became clear that, in the cell population in which the relative expression level of the LEF1 gene with respect to the expression level of the β-actin gene was 5.5 × 10⁻⁴ or more, a frequency of appearance of undifferentiation-deviated cells was high, and an undifferentiated state was not maintained; whereas the cell population, in which the relative expression level of the LEF1 gene with respect to the expression level of the β-actin gene was 5.5 × 10⁻⁴ or less, was a cell population in which a frequency of appearance of undifferentiation-deviated cells was low and an undifferentiated state was maintained.

That is, the condition in which the relative expression level of the LEF1 gene is 5.5 × 10⁻⁴ or less can be used as an index for determining an undifferentiated state of a pluripotent stem cell population.

### <Example 11: flow cytometry analysis>

The human iPS cells on day 5 of the culture which were obtained in Comparative Examples 1 to 4 and Examples 1 to 8 were treated with Accutase for 3 minutes to 10 minutes and dispersed into single cells by pipetting. These cells were washed with phosphate buffered saline (PBS). Thereafter, the cells were fixed with 4% paraformaldehyde (PFA) at room temperature for 20 minutes, and thereafter washed with PBS three times. Permeation treatment was performed with cold methanol at -20°C overnight. After washing three times with PBS, the cells were blocked with 3% fetal bovine serum (FBS)/PBS for 1 hour at room temperature. Thereafter, a sample of the cells was dispensed into two samples, and each thereof was suspended again in 50 µL. 5 µL of a fluorescently labeled anti-LEF1 antibody (Cat. No. 8490, Cell Signaling Technology) was added to and mixed with one of the samples, and 5 µL of a fluorescently labeled isotype control antibody (Cat. No. 2975, Cell Signaling Technology) was added to and mixed with the other one. Staining was performed at 4°C for 1 hour in a light-shielded state. After washing once with 3% fetal bovine serum (FBS)/PBS, the cells caused to pass through a cell strainer were analyzed with Guava easyCyte 8HT (Merck K.K.). On a gain control screen, a check mark in high sensitivity mode was removed for GRN-B, a gain value was set to 1.68 to start measurement.

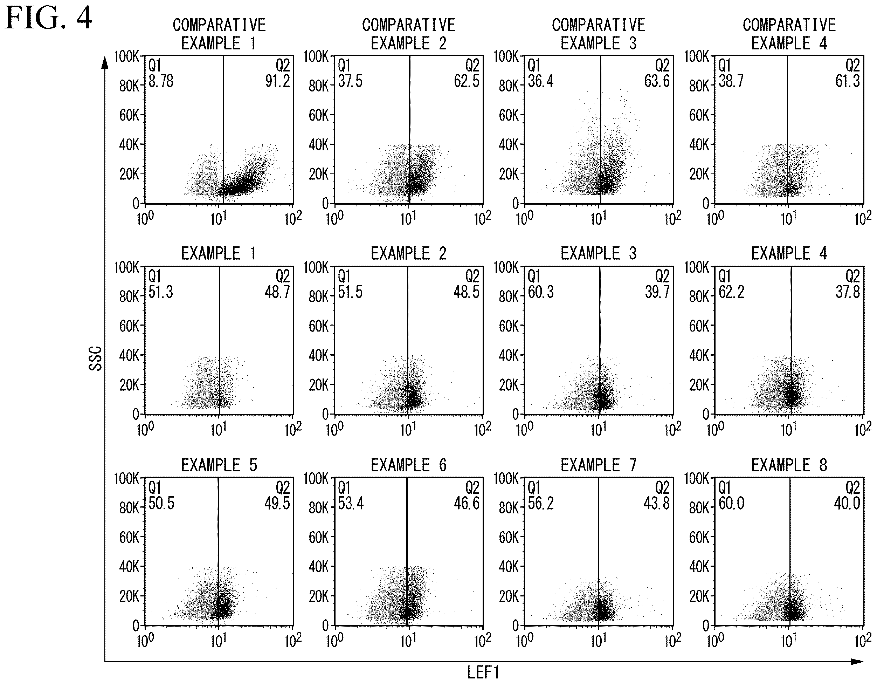

After the measurement, a dot plot of FSC/SSC was displayed for all the samples, a region in which an FSC value was 5000 or more and 40000 or less and an SSC value was 1000 or more and 40000 or less was selected, and cell populations within this region were extracted. Next, for the sample treated with the isotype control antibody, all regions in which a percentage of cell populations with stronger GRN-B fluorescence intensity was 1.5% or less were selected from the cell populations extracted by the above-described FSC/SSC dot plot. For the sample treated with the anti-LEF1 antibody, a proportion of cells contained in the GRN-B region in the cell populations extracted by the FSC/SSC dot plot was calculated, and this proportion was used as a percentage of cells positive for LEF1. Table 4 and Fig. 4 show results thereof.

**[Table 4]**

| | Percentage of cells positive for LEF1 (%) |
|---|---|
| Comparative Example 1 | 91.2 |
| Comparative Example 2 | 62.5 |
| Comparative Example 3 | 63.6 |
| Comparative Example 4 | 61.3 |
| Example 1 | 48.7 |
| Example 2 | 48.5 |
| Example 3 | 39.7 |
| Example 4 | 37.8 |
| Example 5 | 49.5 |
| Example 6 | 46.6 |
| Example 7 | 43.8 |
| Example 8 | 40.0 |

In Comparative Examples 1 to 4 and Examples 1 to 8, it was recognized that percentages of cells positive for LEF1 tended to be different. Specifically, in the pluripotent stem cell populations on day 5 of the culture which were obtained in Comparative Examples 1 to 4, a percentage of cells positive for LEF1 was 55% or more; whereas in all of the pluripotent stem cell populations on day 5 of the culture which were obtained in Examples 1 to 8, a percentage of cells positive for LEF1 was 55% or less.

In light of the results of the expression of the differentiation marker genes analyzed in Example 10 (Figs. 2B and 3B), it was found that the pluripotent stem cell population, in which a percentage of cells positive for LEF1 was 55% or less, was a cell population in which a frequency of appearance of undifferentiation-deviated cells was low and an undifferentiated state was maintained. That is, the condition in which a percentage of cells positive for LEF1 is 55% or less is an index for determining an undifferentiated state of the cell population containing pluripotent stem cells.

Based on the above results, it was shown that the cell population containing pluripotent stem cells and having the following characteristics (a) and (b) is a cell population in which an undifferentiated state was maintained, and that the following characteristics (a) and (b) are indexes for determining an undifferentiated state of the cell population containing pluripotent stem cells.
(a) A relative expression level of a LEF1 gene with respect to an expression level of a β-actin gene is 5.5 × 10⁻⁴ or less.
(b) A percentage of pluripotent stem cells positive for LEF1 is 55% or less.

Furthermore, based on the results of gene expression analyzed in Example 10 (Figs. 2A to 2C, 3A, and 3B) and the results of gene expression levels and flow cytometry in Examples 10 and 11, it was found that, using the above-described (a) and (b) as indexes, it is possible to predict appearance of undifferentiation-deviated cells in the cell population containing pluripotent stem cells, or monitor differentiation potential (undifferentiated state) of pluripotent stem cells in the cell population. Specifically, on day 5 of the culture, appearance of undifferentiation-deviated cells was hardly recognized in both of the cell population satisfying the above-described indexes or the cell population not satisfying the indexes. However, when the culture was continued thereafter, appearance of undifferentiation-deviated cells increased remarkably only in the cell population that did not satisfy the indexes. That is, it was shown that, for that cell population not satisfying the indexes, even when appearance of undifferentiation-deviated cells was not recognized at the time of measuring the indexes, a frequency of appearance of undifferentiation-deviated cells will increase if culture is continued.

Therefore, in the present invention, the following (a) and (b) can be used as indexes for predicting appearance of undifferentiation-deviated cells: (a) a relative expression level of a LEF1 gene with respect to an expression level of a β-actin gene is 5.5 × 10⁻⁴ or less; and (b) a percentage of pluripotent stem cells positive for LEF1 is 55% or less.

Furthermore, (a) and (b) above can also be used as indexes for determining and/or predicting optimum culture conditions for the cell population containing pluripotent stem cells to maintain its undifferentiated state.

Furthermore, using (a) and (b) above as indexes, differentiation potential of pluripotent stem cells in the cell population can be monitored by measuring a positive rate of gene expression and protein expression in the cell population containing pluripotent stem cells over time. Based on the results of measuring the indexes, when it is determined that differentiation potential is low, it is possible prepare a pluripotent stem cell population with high differentiation potential by a method of selecting cells with a cell sorter or the like using (a) and (b) as indexes, or a method of appropriately changing culture conditions to promote maintenance of an undifferentiated state of pluripotent stem cells.

### <Comparative Example 5: induction of differentiation into three germ layer cells>

Induction of differentiation of pluripotent stem cells was performed by the following procedure. For all culture days in the following steps, the start date of suspension culture in Step 1 is counted as day 0.

### (Step 1: preparation of pluripotent stem cell population)

Suspension culture was performed by the same method as in Comparative Example 4, and on day 10 of the culture, cell aggregates and a culture supernatant were recovered in a centrifuge tube from wells. The centrifuge tube was left to stand for about 5 minutes to precipitate the cell aggregates. Thereafter, the culture supernatant was removed. 1 mL of Accutase was added to the cell aggregates to treat them for 10 minutes. The cell aggregates were dispersed into single cells by pipetting. These cells were suspended in a StemFit (registered trademark) AK02N medium containing Y-27632 at a final concentration of 10 µM. A part of the cells was stained with trypan blue to measure the number of living cells. A cell suspension was prepared so that it contained 2 × 10⁵ cells per mL using the StemFit (registered trademark) AK02N medium containing Y-27632 a final concentration of 10 µM. 4 mL of the cell suspension per well was seeded in a 6-well plate for suspension culture. The plate in which the cells were seeded was gyrated on a rotary shaker at a speed of 83 rpm so that a gyrating width (diameter) drew a circle of 25 mm along a horizontal plane, and suspension culture was continued at 37°C in a 5% CO₂ environment until day 12 of the culture. On day 11 of the culture, medium exchange was performed with a StemFit (registered trademark) AK02N medium containing Y-27632 at a final concentration of 5 µM. The medium exchange was performed by recovering a total amount of the medium containing the cell aggregates in a centrifuge tube and leaving the medium to stand for about 5 minutes to precipitate the cell aggregates, thereafter, removing the culture supernatant, gently suspending the cell aggregates again in a fresh medium, and returning the cell aggregates to the original wells.

It is thought that the pluripotent stem cell population obtained in Step 1 exhibits the same level of LEF1 expression as in Comparative Example 4. That is, it is a pluripotent stem cell population not satisfying the indexes (a) and (b).

### (Step 2-A: induction of differentiation into endoderm)

After Step 1 above, induction of differentiation into endoderm was performed. On day 12 and day 13 of the culture, medium exchange was performed with a medium 1 for induction of differentiation into endoderm shown in Table 5. On day 14 of the culture, medium exchange was performed with a medium 2 for induction of differentiation into endoderm shown in Table 5. On day 15 of the culture, medium exchange was performed with a medium 3 for induction of differentiation into endoderm shown in Table 5. The medium exchange was performed by recovering a total amount of the medium containing the cell aggregates in a centrifuge tube and leaving the medium to stand for about 5 minutes to precipitate the cell aggregates, thereafter, removing the culture supernatant, gently suspending the cell aggregates again in a fresh medium, and returning the cell aggregates to the original wells. Thereafter, the suspension culture was continued until day 16 of the culture to prepare a cell sample obtained by induction of differentiation into endoderm.

**[Table 5]**

| | Medium composition (manufacturer/No.) |
|---|---|
| Medium 1 for induction of differentiation into endoderm | RPMI 1640 medium (FUJIFILM Wako Pure Chemical Corporation/189-02025) |
| | 0.25 vol% BSA (FUJIFILM Wako Pure Chemical Corporation/0 17-22231) |
| | 1 mM sodium pyruvate (FUJIFILM Wako Pure Chemical Corporation/190-14881) |
| | 1 vol% MEM non-essential amino acid solution (FUJIFILM Wako Pure Chemical Corporation/139-15651) |
| | 55 µM 2-Mercaptoethanol (Thermo Fisher Scientific K.K./21985023) |
| | 80 ng/mL Activin A (R&D Systems, Inc./338-AC) |
| | 50 ng/mL FGF-2 (PeproTech, Inc./AF-100-18) |
| | 20 ng/mL BMP-4 (Miltenvi Biotec LLC./130-111-168) |
| | 3 µM CHIR 99021 (FUJIFILM Wako Pure Chemical Corporation/038-23101) |
| Medium 2 for induction of differentiation into endoderm | RPMI 1640 medium (FUJIFILM Wako Pure Chemical Corporation/189-02025) |
| | 0.25 vol% BSA (FUJIFILM Wako Pure Chemical Corporation/017-22231) |
| | 1 mM sodium pyruvate (FUJIFILM Wako Pure Chemical Corporation/190-14881) |
| | 1 vol% MEM non-essential amino acid solution (FUJIFILM Wako Pure Chemical Corporation/139-15651) |
| | 55 µM 2-Mercaptoethanol (Thermo Fisher Scientific K.K./21985023) |
| | 80 ng/mL Activin A (R&D Systems, Inc./338-AC) |
| Medium 3 for induction of differentiation into endoderm | RPMI 1640 medium (FUJIFILM Wako Pure Chemical Corporation/189-02025) |
| | 0.25 vol% BSA (FUJIFILM Wako Pure Chemical Corporation/017-22231) |
| | 1 mM sodium pyruvate (FUJIFILM Wako Pure Chemical Corporation/190-14881) |
| | 1 vol% MEM non-essential amino acid solution (FUJIFILM Wako Pure Chemical Corporation/139-15651) |
| | 55 µM 2-Mercaptoethanol (Thermo Fisher Scientific K.K./21985023) |
| | 80 ng/mL Activin A (R&D Systems, Inc./338-AC) |
| | 0.5 vol% KSR (Thermo Fisher Scientific K.K./10828028) |

### (Step 2-B: induction of differentiation into mesoderm)

After Step 1 above, induction of differentiation into mesoderm was performed. On day 12 of the culture, medium exchange was performed with a medium 1 for induction of differentiation into mesoderm shown in Table 6. On day 13 of the culture, medium exchange was performed with a medium 2 for induction of differentiation into mesoderm shown in Table 6. The medium exchange was performed by recovering a total amount of the medium containing the cell aggregates in a centrifuge tube and leaving the medium to stand for about 5 minutes to precipitate the cell aggregates, thereafter, removing the culture supernatant, gently suspending the cell aggregates again in a fresh medium, and returning the cell aggregates to the original wells. Thereafter, the suspension culture was continued until day 15 of the culture to prepare a cell sample obtained by induction of differentiation into mesoderm.

**[Table 6]**

| | Medium composition (manufacturer/No.) |
|---|---|
| Medium 1 for induction of differentiation into mesoderm | RPMI 1640 medium (FUJIFILM Wako Pure Chemical Corporation/189-02025) |
| | 2 vol% B-27 Plus Supplement (Thermo Fisher Scientific K.K./A3582801) |
| | 3 µM CHIR 99021 (FUJIFILM Wako Pure Chemical Corporation/038-23101) |
| Medium 2 for induction of differentiation into mesoderm | RPMI 1640 medium (FUJIFILM Wako Pure Chemical Corporation/189-02025) |
| | 2 vol% B-27 Plus Supplement (Thermo Fisher Scientific K.K./A3582801) |

### (Step 2-C: induction of differentiation into ectoderm)

After Step 1 above, induction of differentiation into ectoderm was performed. From day 12 to day 16 of the culture, medium exchange was performed every day with a medium for induction of differentiation into ectoderm shown in Table 7. The medium exchange was performed by recovering a total amount of the medium containing the cell aggregates in a centrifuge tube and leaving the medium to stand for about 5 minutes to precipitate the cell aggregates, thereafter, removing the culture supernatant, gently suspending the cell aggregates again in a fresh medium, and returning the cell aggregates to the original wells. Thereafter, the suspension culture was continued until day 17 of the culture to prepare a cell sample obtained by induction of differentiation into ectoderm.

**[Table 7]**

| | Medium composition (manufacturer/No.) |
|---|---|
| Medium for induction of differentiation into ectoderm | KnockOut DMEM/F-12 medium (Thermo Fisher Scientific K.K./12660012) + Neurobasal medium (Thermo Fisher Scientific K.K./21103049) (1:1 blending) |
| | 1 vol% GlutaMAX Supplement (Thermo Fisher Scientific K.K./35050061) |
| | 0.1 mM L-ascorbic acid (FUJIFILM Wako Pure Chemical Corporation/100769) |
| | 1 vol% MEM non-essential amino acid solution (FUJIFILM Wako Pure Chemical Corporation/139-15651) |
| | 2 µM SB431542 (FUJIFILM Wako Pure Chemical Corporation/192-16541) |
| | 1 µM Dorsomorphin dihydrochloride (FUJIFILM Wako Pure Chemical Corporation/047-33763) |
| | 3 µM CHIR 99021 (FUJIFILM Wako Pure Chemical Corporation/038-23101) |

### <Example 12: induction of differentiation into three germ layer cells>

Induction of differentiation of pluripotent stem cells was performed by the following procedure.

### (Step 1: preparation of pluripotent stem cell population)

A pluripotent stem cell population was prepared by the same method as in Step 1 of Comparative Example 5 except the point that the suspension culture was performed by the same method as in Example 8 until day 10 of the culture, and the point that XAV939 was added, so that a final concentration was 20 µM, to a seeding medium on day 10 of the culture and a medium for medium exchange on day 11 of the culture.

It is thought that the pluripotent stem cell population obtained in Step 1 exhibits the same level of LEF1 expression as in Example 8. That is, it is a pluripotent stem cell population in which (a) a relative expression level of a LEF1 gene with respect to an expression level of a β-actin gene is 5.5 × 10⁻⁴ or less, and (b) a percentage of pluripotent stem cells positive for LEF1 is 55% or less.

### (Step 2-A: induction of differentiation into endoderm)

After Step 1 above, induction of differentiation into endoderm was performed in the same procedure as in Step 2-A of Comparative Example 4.

### (Step 2-B: induction of differentiation into mesoderm)

After Step 1 above, induction of differentiation into mesoderm was performed in the same procedure as in Step 2-B of Comparative Example 4.

### (Step 2-C: induction of differentiation into ectoderm)

After Step 1 above, induction of differentiation into ectoderm was performed in the same procedure as in Step 2-C of Comparative Example 4.

### <Example 13: comparison of differentiation induction efficiency by quantitative real-time PCR analysis>

The cell populations induced to differentiate into three germ layer cells in Comparative Example 5 and Example 12 were subjected to quantitative real-time PCR analysis by the same procedure as in Example 10.

Base sequences of primers used in the quantitative real-time PCR analysis of the samples obtained by induction of differentiation into endoderm are shown below.
ACTB (F): 5'-CCTCATGAAGATCCTCACCGA-3' (SEQ ID NO: 1)
ACTB (R): 5'-TTGCCAATGGTGATGACCTGG-3' (SEQ ID NO: 2)
SOX17 (F): 5'-ATCTGCACTTCGTGTGCAAG-3' (SEQ ID NO: 11)
SOX17 (R): 5'-GAGTCTGAGGATTTCCTTAGCTC-3' (SEQ ID NO: 12)
FOXA2 (F): 5'-GGTGATTGCTGGTCGTTTGTTGTG-3' (SEQ ID NO: 17)
FOXA2 (R): 5'-GCCGACATGCTCATGTACGTGTT-3' (SEQ ID NO: 18)
CXCR4 (F): 5'-ACTGAGAAGCATGACGGACAAG-3' (SEQ ID NO: 19)
CXCR4 (R): 5'-AGGTAGCGGTCCAGACTGATG-3' (SEQ ID NO: 20)

Base sequences of primers used in the quantitative real-time PCR analysis of the samples obtained by induction of differentiation into mesoderm are shown below.
ACTB (F): 5'-CCTCATGAAGATCCTCACCGA-3' (SEQ ID NO: 1)
ACTB (R): 5'-TTGCCAATGGTGATGACCTGG-3' (SEQ ID NO: 2)
CDX2 (F): 5'-CACCCACAGCCATAGACCTAC-3' (SEQ ID NO: 21)
CDX2 (R): 5'-GTCAGTCCAGGCAATGCTTC-3' (SEQ ID NO: 22)
CXCR4 (F): 5'-ACTGAGAAGCATGACGGACAAG-3' (SEQ ID NO: 19)
CXCR4 (R): 5'-AGGTAGCGGTCCAGACTGATG-3' (SEQ ID NO: 20)
VEGFR2 (F): 5'-AGCCAAGCTGTCTCAGTGAC-3' (SEQ ID NO: 23)
VEGFR2 (R): 5'-TCTCCCGACTTTGTTGACCG-3' (SEQ ID NO: 24)
PDGFRα (F): 5'-GCTGAGCCTAATCCTCTGCC-3' (SEQ ID NO: 25)
PDGFRα (R): 5'-ACTGCTCACTTCCAAGACCG-3' (SEQ ID NO: 26)

Base sequences of primers used in the quantitative real-time PCR analysis of the samples obtained by induction of differentiation into ectoderm are shown below.
ACTB (F): 5'-CCTCATGAAGATCCTCACCGA-3' (SEQ ID NO: 1)
ACTB (R): 5'-TTGCCAATGGTGATGACCTGG-3' (SEQ ID NO: 2)
PAX6 (F): 5'-AGGAATGGACTTGAAACAAGG-3' (SEQ ID NO: 13)
PAX6 (R): 5'-GCAAAGCTTGTTGATCATGG-3' (SEQ ID NO: 14)
SOX1 (F): 5'-AGGCAGGTCCAAGCACTTAC-3' (SEQ ID NO: 27)
SOX1 (R): 5'-ATAACTCCGCCGTCTGAAGG-3' (SEQ ID NO: 28)
NESTIN (F): 5'-TCAAGCACCACTGTGGACTC-3' (SEQ ID NO: 29)
NESTIN (R): 5'-AGGTTCCATGCTCCCAGAGA-3' (SEQ ID NO: 30)

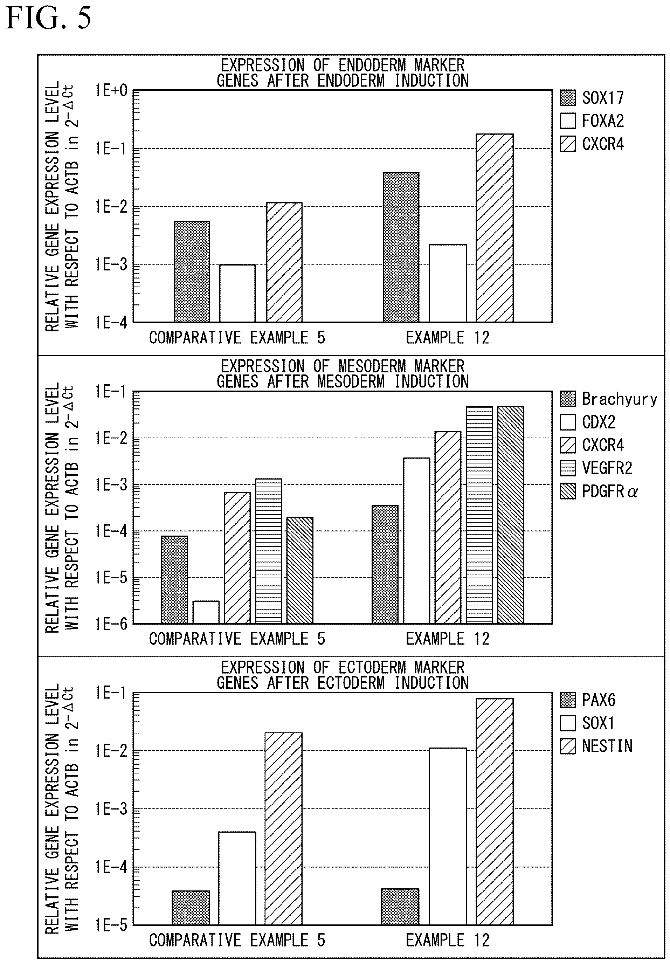

Tables 8 to 10 and Fig. 5 show results of measuring gene expression levels.

**[Table 8]**

| | Relative gene expression level with respect to ACTB in 2^{-Δ Ct} | | |
|---|---|---|---|
| | SOX17 | FOXA2 | CXCR4 |
| Comparative Example 5 (induction of differentiation into endoderm) | 5.25 × 10⁻³ | 9.20 × 10⁻⁴ | 1.15 × 10⁻² |
| Example 12 (induction of differentiation into endoderm) | 4.08 × 10⁻² | 2.17 × 10⁻³ | 1.76 × 10⁻¹ |

**[Table 9]**

| | Relative gene expression level with respect to ACTB in 2^{-Δ Ct} | | | |
|---|---|---|---|---|
| | CDX2 | CXCR4 | VEGFR2 | PDGFRα |
| Comparative Example 5 (induction of differentiation into mesoderm) | 3.10 × 10⁻⁶ | 6.91 × 10⁻⁴ | 1.37 × 10⁻³ | 1.93 × 10⁻⁴ |
| Example 12 (induction of differentiation into mesoderm) | 3.84 × 10⁻³ | 1.40 × 10⁻² | 4.98 × 10⁻² | 4.78 × 10⁻² |

**[Table 10]**

| | Relative gene expression level with respect to ACTB in 2^{-Δ Ct} | | |
|---|---|---|---|
| | PAX6 | SOX1 | NESTIN |
| Comparative Example 5 (induction of differentiation into ectoderm) | 3.69 × 10⁻⁵ | 3.90 × 10⁻⁴ | 2.03 × 10⁻² |
| Example 12 (induction of differentiation into ectoderm) | 3.96 × 10⁻⁵ | 1.17 × 10⁻² | 7.48 × 10⁻² |

Example 12 showed a tendency in which expression levels of the endoderm differentiation marker genes (SOX17, FOXA2, and CXCR4) after the induction of differentiation into endoderm were higher as compared to Comparative Example 5. Furthermore, similarly, Example 12 showed a tendency in which expression levels of the mesoderm differentiation marker genes (CDX2, CXCR4, VEGFR2, and PDGFRα) after the induction of differentiation into mesoderm, and expression levels of ectoderm differentiation marker genes (PAX6, SOX1, and NESTIN) after the induction of differentiation into ectoderm were higher as compared to Comparative Example 5.

Based on these results, it became clear that efficiency of inducing differentiation into three germ layer cells increased by preparing a pluripotent stem cell population satisfying the above-described indexes (a) and (b).

### <Example 13: differentiation induction culture>

The cells on day 10 of the culture which were obtained in Comparative Examples 1 to 4 and Examples 1 to 8 can be induced to differentiate into three germ layer cells by the following procedure disclosed in Non Patent Document (Sundari Chetty et al., Nature Methods 10 (6): 553-556, January 2013).

### <Step 1: preparation of cells to be induced to differentiate>

The cells on day 10 of the culture which were obtained in Comparative Examples 1 to 4 and Examples 1 to 8 were treated with Accutase for 3 to 10 minutes and dispersed into single cells by pipetting. These cells were suspended in an MEF-conditioned medium containing Y-27632 at a final concentration of 10 µM and bFGF (Thermo Fisher Scientific K.K.) at a final concentration of 20 ng/mL. A part of the cells was stained with trypan blue to measure the number of living cells. A cell suspension was prepared so that it contained 5 × 10⁵ cells per mL using the MEF-conditioned medium containing Y-27632 at a final concentration of 10 µM and bFGF at a final concentration of 20 ng/mL. 2 mL of the cell suspension per well was seeded on a 6-well plate for cell culture coated with Growth factor Reduced Matrigel (BD Biosciences), and adhesion culture was performed at 37°C in a 5% CO₂ environment. The day on which the cells were seeded was defined as day 0 of the culture. On day 1 of the culture, medium exchange was performed with a MEF-conditioned medium containing bFGF at a final concentration of 20 ng/mL.

### <Step 2-A: induction of differentiation into ectoderm cells>

For the cells prepared in Step 1 of Example 13, medium exchange was performed on day 2 of the culture with KnockOut-DMEM (Thermo Fisher Scientific K.K.) containing knockout serum replacement (Thermo Fisher Scientific K.K.) at a final concentration of 10%, Noggin (R&D Systems, Inc.) at a final concentration of 500 ng/mL, and SB431542 (Tocris Bioscience) at a final concentration of 10 µM. Medium exchange was performed using the same medium on day 3 of the culture, and thereby an ectoderm cell population could be obtained on day 4 of the culture.

### <Step 2-B: induction of differentiation into mesoderm cells>

For the cells prepared in Step 1 of Example 13, medium exchange was performed on day 2 of the culture with an RPMI-B27 (Thermo Fisher Scientific K.K.) medium to which recombinant human Activin A (R&D Systems, Inc.) was added at a final concentration of 100 ng/mL. Medium exchange was performed on days 3, 4, and 5 of the culture with an RPMI-B27 (Thermo Fisher Scientific K.K.) medium to which recombinant human BMP4 (R&D Systems, Inc.) was added at a final concentration of 10 ng/mL, and thereby a mesoderm cell population could be obtained on day 6 of the culture.

### <Step 2-C: induction of differentiation into endoderm cells>

For the cells prepared in Step 1 of Example 13, medium exchange was performed on day 2 of the culture with an MCDB-131 medium to which NaHCO₃ at a final concentration of 2.5 g/L, Glutamax (Thermo Fisher Scientific K.K.) at a final concentration of 1%, glucose at a final concentration of 5.5 mM, FAF-BSA (Proliant Inc. and Lampire Biological Laboratories, Inc.) at a final concentration of 0.1%, ITS: X (Thermo Fisher Scientific K.K.) diluted 50000-fold, WNT3A (R&D Systems, Inc.) at a final concentration of 20 ng/mL, and Activin A at a final concentration of 100 ng/mL were added. Medium exchange was performed on days 3, 4, and 5 of the culture with an MCDB-131 medium to which the above-mentioned additives other than WNT3A were added, and thereby an endoderm cell population could be obtained on day 6 of the culture.

### <Step 3: measurement of differentiation induction efficiency>

The cell populations obtained in Steps 2-A, 2-B, and 2-C were immunofluorescently stained by the following method to obtain efficiency of inducing differentiation into three germ layer cells.

The cells obtained in Steps 2-A, 2-B, and 2-C above were washed with PBS, and thereafter reacted with 4% paraformaldehyde for 30 minutes to immobilize the cells. The cells are washed with PBS. PBS containing donkey serum at a final concentration of 5% and Triton at a final concentration of 0.3% was added. The reaction was allowed at room temperature for 1 hour to perform blocking. Thereafter, a primary antibody was added to each sample so that the antibody was diluted 500-fold, and the reaction was allowed overnight. For primary antibodies, an anti-Sox1 antibody (R&D Systems, Inc.) was used for ectoderm cells, an anti-Brachyury antibody (R&D Systems, Inc.) was used for mesoderm cells, and an anti-Sox17 antibody (R&D Systems, Inc.) was used for endoderm cells. After the primary antibody reaction, the cells are washed with PBS.

A secondary antibody labeled with Alexa Fluor 488 or Alexa Fluor 594 corresponding to the primary antibody was added so that the secondary antibody was diluted 500-fold, and the reaction was allowed at room temperature for 1 hour. After the reaction, the cells are washed with PBS. Hoechst 33342 was added so that it was diluted 1000-fold to perform nuclear staining. Using Celllnsight CX5 High-Content Screening (HCS) Platform (Thermo Fisher Scientific K.K.), 30 images of immunofluorescently stained cells per well in a 10-fold field of view were acquired. The number of cells was calculated from the number of labeled nuclei in the acquired images, and the number of each of the three germ layer cells was calculated from the number of cells labeled with each of the antibodies. Differentiation induction efficiency could be obtained from the number of each of the three germ layer cells with respect to the number of cells in the visual field.

It was shown that, in Comparative Examples 1 to 4 and Examples 1 to 8, differentiation induction efficiencies tended to be different. Differentiation induction efficiencies into each of the three germ layer cells were high in Examples 1 to 8 as compared to Comparative Examples 1 to 4. Based on these results, differentiation induction efficiency is high in the cell population containing pluripotent stem cells, in which an expression level of the LEF1 gene and a proportion of the pluripotent stem cell population positive for LEF1 are within the range of the present invention.

### <Example 14: induction of differentiation into somatic cells (pancreatic β cells)>

For the first 2 days, in a state where cell populations of the iPS cells obtained in Examples 1 to 8 were adhered on a dish, the cell populations were cultured in RPMI 1640 containing 0.5% Bovine Serum Albumin, 0.4 × PS, 1 mmol/L sodium pyruvate, 1 × NEAA, 80 ng/mL recombinant human activin A, 50 ng/mL FGF2, 20 ng/mL recombinant bone morphogenetic protein 4, and 3 µmol/L CHIR 99021. On the 3rd day, CHIR 99021 was removed from this medium, and culture was performed in a state where the cells were adhered on the dish. On the 4th day, in a medium into which 1% (volume/volume) KSR was further added, culture was performed for 1 day in a state where the cells were adhered on the dish to induce differentiation from pluripotent stem cells into endoderm cells. Next, culture was performed for 2 days in RPMI 1640 containing 0.5% BSA, 1 mmol/L sodium pyruvate, 1 × NEAA, 0.4 × PS, 50 ng/mL FGF2, 50 ng/mL recombinant human FGF7 (PeproTech, Inc.), 2% B27 supplement (GIBCO), 0.67 µmol/L EC23 (Santa Cruz Biotechnology, Inc.), 1 µmol/L dorsomorphin (FUJIFILM Wako Pure Chemical Corporation), 10 µmol/L SB431542 (FUJIFILM Wako Pure Chemical Corporation), and 0.25 mol/L SANT1 (FUJIFILM Wako Pure Chemical Corporation) to induce differentiation from the endoderm cells into primitive gut tube (PGT) cells. Next, culture was performed for 4 days in DMEM-high glucose (FUJIFILM Wako Pure Chemical Corporation) containing 0.4 × PS, 1 × NEAA, 50 ng/mL FGF2, 2% B27, 0.67 µmol/L EC23, 1 µmol/L dorsomorphin, 10 µmol/L SB431542, and 0.25 µmol/L SANT1 to induce differentiation from the primitive gut tube (PGT) cells into posterior foregut (PFG) cells. Next, culture was performed for 3 days in DMEM-high glucose containing 0.4 × PS, 1 × NEAA, 50 ng/mL recombinant human FGF10 (PeproTech, Inc.), 2% B27, 0.5 µmol/L EC23, 1 µmol/L dorsomorphin, 0.25 µmol/L SANT1, 5 µmol/L Alk5 inhibitor II (BioVision Inc.), and 0.3 µmol/L indolactam V (ILV; Cayman Chemical Company) to induce differentiation from the posterior foregut (PFG) cells into pancreatic progenitor (PP) cells. Next, culture was performed for 3 days in Advanced-DMEM (GIBCO) containing 0.4 × PS, 2 mmol/L L-glutamine, 2% B27, 0.2 µmol/L EC23, 1 µmol/L dorsomorphin, 0.25 µmol/L SANT1, 5 µmol/L Alk5 inhibitor II, and 50 ng/mL Exendin 4 (Sigma-Aldrich) to induce differentiation from the pancreatic progenitor (PP) cells into endocrine progenitor (EP) cells. Next, culture was performed for 6 days in Advanced-DMEM containing 0.4 × PS, 2 mmol/L L-glutamine, 2% B27, 10 ng/mL BMP4, 10 ng/mL FGF2, 50 ng/mL recombinant human hepatocyte growth factor (HGF; PeproTech, Inc.), 50 ng/mL insulin-like growth factor 1 (IGF1; PeproTech, Inc.), 5 µmol/L Alk5 inhibitor II, 50 ng/mL Exendin 4, 5 mmol/L nicotinamide (Sigma-Aldrich), and 5 µmol/L forskolin (FUJIFILM Wako Pure Chemical Corporation) to induce differentiation from the endocrine progenitor (EP) cells into pancreatic β cells. Although the above-described method of inducing differentiation is one embodiment, it is possible to induce differentiation from pluripotent stem cells into pancreatic β cells, and thereby it is possible to produce somatic cells.

## Claims

1. A production method for a cell population containing pluripotent stem cells, the method comprising:
a step of culturing cell populations containing pluripotent stem cells; and
a step of sorting out a cell population having the following characteristics (a) and (b) from the cell populations containing pluripotent stem cells,
(a) a relative expression level of a LEF1 gene with respect to an expression level of a β-actin gene is 5.5 × 10⁻⁴ or less in the cell population, and
(b) a percentage of cells positive for LEF1 is 55% or less in the cell population.

2. A production method for a cell population containing pluripotent stem cells, the method comprising:
a step of culturing a cell population containing pluripotent stem cells;
a step of confirming whether the cell population is a cell population having the following characteristics (a) and (b); and
a step of acquiring the cell population confirmed to be the cell population having the characteristics (a) and (b),
(a) a relative expression level of a LEF1 gene with respect to an expression level of a β-actin gene is 5.5 × 10⁻⁴ or less in the cell population, and
(b) a percentage of cells positive for LEF1 is 55% or less in the cell population.

3. A production method for somatic cells, the method comprising:
a step of culturing cell populations containing pluripotent stem cells;
a step of sorting out a cell population having the following characteristics (a) and (b) from the cell populations containing pluripotent stem cells; and
a step of culturing the cell population having the characteristics (a) and (b) in the presence of a differentiation-inducing factor,
(a) a relative expression level of a LEF1 gene with respect to an expression level of a β-actin gene is 5.5 × 10⁻⁴ or less in the cell population, and
(b) a percentage of cells positive for LEF1 is 55% or less in the cell population.

4. A production method for somatic cells, the method comprising:
a step of culturing a cell population containing pluripotent stem cells;
a step of confirming whether the cell population is a cell population having the following characteristics (a) and (b); and
a step of culturing the cell population confirmed to be the cell population having the characteristics (a) and (b) in the presence of a differentiation-inducing factor,
(a) a relative expression level of a LEF1 gene with respect to an expression level of a β-actin gene is 5.5 × 10⁻⁴ or less in the cell population, and
(b) a percentage of cells positive for LEF1 is 55% or less in the cell population.

5. The production method according to Claim 3 or 4, wherein the somatic cells are selected from the group consisting of endoderm cells, mesoderm cells, and ectoderm cells.

6. The production method according to Claim 3 or 4, wherein the somatic cells are at least one selected from the group consisting of cardiac muscle cells, skeletal muscle cells, nerve cells, megakaryocytes, hematopoietic stem cells, airway epithelial cells, germ cells, dendritic cells, eosinophils, mast cells, cartilage cells, T cells, erythropoietin-producing cells, intestinal epithelium, pancreatic cells, liver cells, alveolar epithelial cells, and kidney cells.

7. A cell population containing pluripotent stem cells,
wherein the cell population is a cell population having the following characteristics (a) and (b),
(a) a relative expression level of a LEF1 gene with respect to an expression level of a β-actin gene is 5.5 × 10⁻⁴ or less in the cell population, and
(b) a percentage of pluripotent stem cells positive for LEF1 is 55% or less in the cell population.

8. A method for monitoring differentiation potential of a cell population containing pluripotent stem cells using the following characteristics (a) and (b) as indexes,
(a) a relative expression level of a LEF1 gene with respect to an expression level of a β-actin gene is 5.5 × 10⁻⁴ or less in the cell population, and
(b) a percentage of pluripotent stem cells positive for LEF1 is 55% or less in the cell population.

9. A method for evaluating differentiation potential of a cell population containing pluripotent stem cells using the following characteristics (a) and (b) as indexes,
(a) a relative expression level of a LEF1 gene with respect to an expression level of a β-actin gene is 5.5 × 10⁻⁴ or less in the cell population, and
(b) a percentage of pluripotent stem cells positive for LEF1 is 55% or less in the cell population.


SEQUENCE LISTING

<110>  Kaneka Corporation

<120>  A cell population comprising pluripotent stem cells and a method
       thereof

<130>  B190097WO01

<150>  JP2019-66845
<151>  2019-03-29

<160>  38

<170>  PatentIn version 3.5

<210>  1
<211>  21
<212>  DNA
<213>  Artificial Sequence

<220>
<223>  Description of Artificial Sequence: primer

<400>  1
cctcatgaag atcctcaccg a                                                21


<210>  2
<211>  21
<212>  DNA
<213>  Artificial Sequence

<220>
<223>  Description of Artificial Sequence: primer

<400>  2
ttgccaatgg tgatgacctg g                                                21


<210>  3
<211>  23
<212>  DNA
<213>  Artificial Sequence

<220>
<223>  Description of Artificial Sequence: primer

<400>  3
agtgggtgga ggaagctgac aac                                              23


<210>  4
<211>  24
<212>  DNA
<213>  Artificial Sequence

<220>
<223>  Description of Artificial Sequence: primer

<400>  4
tcgttgtgca tagtcgctgc ttga                                             24

<210>  5
<211>  22
<212>  DNA
<213>  Artificial Sequence

<220>
<223>  Description of Artificial Sequence: primer

<400>  5
caccaatccc atccacactc ac                                                22


<210>  6
<211>  21
<212>  DNA
<213>  Artificial Sequence

<220>
<223>  Description of Artificial Sequence: primer

<400>  6
gcaaagctcc taccgtacca c                                                 21


<210>  7
<211>  24
<212>  DNA
<213>  Artificial Sequence

<220>
<223>  Description of Artificial Sequence: primer

<400>  7
agcctccagc agatgcaaga actc                                              24


<210>  8
<211>  24
<212>  DNA
<213>  Artificial Sequence

<220>
<223>  Description of Artificial Sequence: primer

<400>  8
ttgctccaca ttggaaggtt ccca                                              24


<210>  9
<211>  23
<212>  DNA
<213>  Artificial Sequence

<220>
<223>  Description of Artificial Sequence: primer

<400>  9
tcacaaagag atgatggagg aac                                               23


<210>  10
<211>  20

<212>  DNA
<213>  Artificial Sequence

<220>
<223>  Description of Artificial Sequence: primer

<400>  10
acatgcaggt gagttgtcag                                                   20


<210>  11
<211>  20
<212>  DNA
<213>  Artificial Sequence

<220>
<223>  Description of Artificial Sequence: primer

<400>  11
atctgcactt cgtgtgcaag                                                   20


<210>  12
<211>  23
<212>  DNA
<213>  Artificial Sequence

<220>
<223>  Description of Artificial Sequence: primer

<400>  12
gagtctgagg atttccttag ctc                                               23


<210>  13
<211>  21
<212>  DNA
<213>  Artificial Sequence

<220>
<223>  Description of Artificial Sequence: primer

<400>  13
aggaatggac ttgaaacaag g                                                 21


<210>  14
<211>  20
<212>  DNA
<213>  Artificial Sequence

<220>
<223>  Description of Artificial Sequence: primer

<400>  14
gcaaagcttg ttgatcatgg                                                   20


<210>  15
<211>  20
<212>  DNA
<213>  Artificial Sequence

<220>
<223>  Description of Artificial Sequence: primer

<400>  15
agaaagtgca gctatcaacc                                                    20


<210>  16
<211>  20
<212>  DNA
<213>  Artificial Sequence

<220>
<223>  Description of Artificial Sequence: primer

<400>  16
gaatgagctt cgttttccac                                                    20


<210>  17
<211>  24
<212>  DNA
<213>  Artificial sequence

<220>
<223>  Description of Artificial Sequence: primer

<400>  17
ggtgattgct ggtcgtttgt tgtg                                               24


<210>  18
<211>  23
<212>  DNA
<213>  Artificial sequence

<220>
<223>  Description of Artificial Sequence: primer

<400>  18
gccgacatgc tcatgtacgt gtt                                                23


<210>  19
<211>  22
<212>  DNA
<213>  Artificial sequence

<220>
<223>  Description of Artificial Sequence: primer

<400>  19
actgagaagc atgacggaca ag                                                 22


<210>  20
<211>  21
<212>  DNA
<213>  Artificial sequence

<220>
<223>  Description of Artificial Sequence: primer

<400>  20
aggtagcggt ccagactgat g                                                21

<210>  21
<211>  21
<212>  DNA
<213>  Artificial sequence

<220>
<223>  Description of Artificial Sequence: primer

<400>  21
cacccacagc catagaccta c                                                21

<210>  22
<211>  20
<212>  DNA
<213>  Artificial sequence

<220>
<223>  Description of Artificial Sequence: primer

<400>  22
gtcagtccag gcaatgcttc                                                  20

<210>  23
<211>  20
<212>  DNA
<213>  Artificial sequence

<220>
<223>  Description of Artificial Sequence: primer

<400>  23
agccaagctg tctcagtgac                                                  20

<210>  24
<211>  20
<212>  DNA
<213>  Artificial sequence

<220>
<223>  Description of Artificial Sequence: primer

<400>  24
tctcccgact ttgttgaccg                                                  20

<210>  25
<211>  20
<212>  DNA
<213>  Artificial sequence

<220>
<223>  Description of Artificial Sequence: primer

<400>  25
gctgagccta atcctctgcc                                                  20

<210>  26
<211>  20
<212>  DNA
<213>  Artificial sequence

<220>
<223>  Description of Artificial Sequence: primer

<400>  26
actgctcact tccaagaccg                                                    20


<210>  27
<211>  20
<212>  DNA
<213>  Artificial sequence

<220>
<223>  Description of Artificial Sequence: primer

<400>  27
aggcaggtcc aagcacttac                                                    20


<210>  28
<211>  20
<212>  DNA
<213>  Artificial sequence

<220>
<223>  Description of Artificial Sequence: primer

<400>  28
ataactccgc cgtctgaagg                                                    20


<210>  29
<211>  20
<212>  DNA
<213>  Artificial sequence

<220>
<223>  Description of Artificial Sequence: primer

<400>  29
tcaagcacca ctgtggactc                                                    20


<210>  30
<211>  20
<212>  DNA
<213>  Artificial sequence

<220>
<223>  Description of Artificial Sequence: primer

<400>  30
aggttccatg ctcccagaga                                                    20


<210>  31
<211>  3575

<212>  DNA
<213>  Homo sapiens

<220>
<221>  CDS
<222>  (1166)..(2365)

<400>  31
gagaagcagt ggggaggcgc agccgctcac ctgcggggca gggcgcggag gagggacccg      60

ggctgcgcgc tctcgggccg aggaaccagg acgcgcccgg agcctcgcac gcggccaagc     120

tcggggcgtc ccctcccctc ggccgggcga actcaagggg cgcagctctt tgctttgaca     180

gagctggccg gcggaggcgt gcagagcggc gagccggcga gccaggctga gaaactcgag     240

ccgggaacaa agaggggtcg gactgagtgt gtgtgtcggc tcgagctccg ggcagaggca     300

tttgggcccg aggcccccgc tgtgactccc cgagactccg cagtgccctc cactgcggag     360

tccccgcgct tgccggcaaa aactttattc ttggcaaact tctctttctc ttcccctcct     420

cctcggcccc catcttctgc tcctcctcct tctctagcag attaaatgag cctcgagaag     480

aaaaaccgaa gcgaaaggga agaaaataag aagatctaaa acggacatct ccagcgtggg     540

tggctccttt ttctttttct ttttttccca cccttcagga agtggacgtt tcgttatctt     600

ctgatccttg caccttcttt tggggcaaac ggggcccttc tgcccagatc ccctctcttt     660

tctcggaaaa caaactacta agtcggcatc cggggtaact acagtggaga gggtttccgc     720

ggagacgcgc cgccggaccc tcctctgcac tttggggagg cgtgctccct ccagaaccgg     780

cgttctccgc gcgcaaatcc cggcgacgcg gggtcgcggg gtggccgccg gggcagcctc     840

gtctagcgcg cgccgcgcag acgcccccgg agtcgccagc taccgcagcc ctcgccgccc     900

agtgcccttc ggcctcgggg gcgggcgcct gcgtcggtct ccgcgaagcg ggaaagcgcg     960

gcggccgccg ggattcgggc gccgcggcag ctgctccggc tgccggccgg cggccccgcg    1020

ctcgcccgcc ccgcttccgc ccgctgtcct gctgcacgaa cccttccaac tctcctttcc    1080

tcccccaccc ttgagttacc cctctgtctt tcctgctgtt gcgcgggtgc tcccacagcg    1140

gagcggagat tacagagccg ccggg atg ccc caa ctc tcc gga gga ggt ggc      1192
                            Met Pro Gln Leu Ser Gly Gly Gly Gly
                            1               5

ggc ggc ggg ggg gac ccg gaa ctc tgc gcc acg gac gag atg atc ccc      1240
Gly Gly Gly Gly Asp Pro Glu Leu Cys Ala Thr Asp Glu Met Ile Pro
10                  15                  20                  25

ttc aag gac gag ggc gat cct cag aag gaa aag atc ttc gcc gag atc      1288
Phe Lys Asp Glu Gly Asp Pro Gln Lys Glu Lys Ile Phe Ala Glu Ile
                30                  35                  40

agt cat ccc gaa gag gaa ggc gat tta gct gac atc aag tct tcc ttg      1336
Ser His Pro Glu Glu Glu Gly Asp Leu Ala Asp Ile Lys Ser Ser Leu
            45                  50                  55

gtg aac gag tct gaa atc atc ccg gcc agc aac gga cac gag gtg gcc 1384
Val Asn Glu Ser Glu Ile Ile Pro Ala Ser Asn Gly His Glu Val Ala
        60                  65                  70

aga caa gca caa acc tct cag gag ccc tac cac gac aag gcc aga gaa 1432
Arg Gln Ala Gln Thr Ser Gln Glu Pro Tyr His Asp Lys Ala Arg Glu
    75                  80                  85

cac ccc gat gac gga aag cat cca gat gga ggc ctc tac aac aag gga 1480
His Pro Asp Asp Gly Lys His Pro Asp Gly Gly Leu Tyr Asn Lys Gly
90                  95                  100                 105

ccc tcc tac tcg agt tat tcc ggg tac ata atg atg cca aat atg aat 1528
Pro Ser Tyr Ser Ser Tyr Ser Gly Tyr Ile Met Met Pro Asn Met Asn
                110                 115                 120

aac gac cca tac atg tca aat gga tct ctt tct cca ccc atc ccg aga 1576
Asn Asp Pro Tyr Met Ser Asn Gly Ser Leu Ser Pro Pro Ile Pro Arg
            125                 130                 135

aca tca aat aaa gtg ccc gtg gtg cag cca tcc cat gcg gtc cat cct 1624
Thr Ser Asn Lys Val Pro Val Val Gln Pro Ser His Ala Val His Pro
        140                 145                 150

ctc acc ccc ctc atc act tac agt gac gag cac ttt tct cca gga tca 1672
Leu Thr Pro Leu Ile Thr Tyr Ser Asp Glu His Phe Ser Pro Gly Ser
    155                 160                 165

cac ccg tca cac atc cca tca gat gtc aac tcc aaa caa ggc atg tcc 1720
His Pro Ser His Ile Pro Ser Asp Val Asn Ser Lys Gln Gly Met Ser
170                 175                 180                 185

aga cat cct cca gct cct gat atc cct act ttt tat ccc ttg tct ccg 1768
Arg His Pro Pro Ala Pro Asp Ile Pro Thr Phe Tyr Pro Leu Ser Pro
                190                 195                 200

ggt ggt gtt gga cag atc acc cca cct ctt ggc tgg caa ggt cag cct 1816
Gly Gly Val Gly Gln Ile Thr Pro Pro Leu Gly Trp Gln Gly Gln Pro
            205                 210                 215

gta tat ccc atc acg ggt gga ttc agg caa ccc tac cca tcc tca ctg 1864
Val Tyr Pro Ile Thr Gly Gly Phe Arg Gln Pro Tyr Pro Ser Ser Leu
        220                 225                 230

tca gtc gac act tcc atg tcc agg ttt tcc cat cat atg att ccc ggt 1912
Ser Val Asp Thr Ser Met Ser Arg Phe Ser His His Met Ile Pro Gly
    235                 240                 245

cct cct ggt ccc cac aca act ggc atc cct cat cca gct att gta aca 1960
Pro Pro Gly Pro His Thr Thr Gly Ile Pro His Pro Ala Ile Val Thr
250                 255                 260                 265

cct cag gtc aaa cag gaa cat ccc cac act gac agt gac cta atg cac 2008
Pro Gln Val Lys Gln Glu His Pro His Thr Asp Ser Asp Leu Met His
                270                 275                 280

gtg aag cct cag cat gaa cag aga aag gag cag gag cca aaa aga cct 2056
Val Lys Pro Gln His Glu Gln Arg Lys Glu Gln Glu Pro Lys Arg Pro
            285                 290                 295

cac att aag aag cct ctg aat gct ttt atg tta tac atg aaa gaa atg 2104
His Ile Lys Lys Pro Leu Asn Ala Phe Met Leu Tyr Met Lys Glu Met
        300                 305                 310

aga gcg aat gtc gtt gct gag tgt act cta aaa gaa agt gca gct atc     2152
Arg Ala Asn Val Val Ala Glu Cys Thr Leu Lys Glu Ser Ala Ala Ile
    315                 320                 325

aac cag att ctt ggc aga agg tgg cat gcc ctc tcc cgt gaa gag cag     2200
Asn Gln Ile Leu Gly Arg Arg Trp His Ala Leu Ser Arg Glu Glu Gln
330                 335                 340                 345

gct aaa tat tat gaa tta gca cgg aaa gaa aga cag cta cat atg cag     2248
Ala Lys Tyr Tyr Glu Leu Ala Arg Lys Glu Arg Gln Leu His Met Gln
                350                 355                 360

ctt tat cca ggc tgg tct gca aga gac aat tat ggt aag aaa aag aag     2296
Leu Tyr Pro Gly Trp Ser Ala Arg Asp Asn Tyr Gly Lys Lys Lys Lys
            365                 370                 375

agg aag aga gag aaa cta cag gaa tct gca tca ggt aca ggt cca aga     2344
Arg Lys Arg Glu Lys Leu Gln Glu Ser Ala Ser Gly Thr Gly Pro Arg
        380                 385                 390

atg aca gct gcc tac atc tga aacatggtgg aaaacgaagc tcattcccaa     2395
Met Thr Ala Ala Tyr Ile
    395

cgtgcaaagc caaggcagcg accccaggac ctcttctgga gatggaagct tgttgaaaac     2455

ccagactgtc tccacggcct gcccagtcga ccccaaagga acactgacat caattttacc     2515

ctgaggtcac tgctagagac gctgatccat aaagacaatc actgccaacc cctctttcgt     2575

ctactgcaag agccaagttc caaaataaag cataaaaagg ttttttaaaa ggaaatgtaa     2635

aagcacatga gaatgctagc aggctgtggg gcagctgagc agcttttctc ccctcatatc     2695

tgcgtgcact tcccagagca tcttgcatcc aaacctgtaa cctttcggca aggacggtaa     2755

cttggctgca tttgcctgtc atgcgcaact ggagccagca accagcacat ccatcagcac     2815

cccagtggag gagttcatgg aagagttccc tctttgtttc tgcttcattt ttctttcttt     2875

tcttttctcc taaagctttt atttaacagt gcaaaaggat cgtttttttt tgctttttta     2935

aacttgaatt tttttaattt acacttttta gttttaattt tcttgtatat tttgctagct     2995

atgagctttt aaataaaatt gaaagttctg gaaaagtttg aaataatgac ataaaaagaa     3055

gccttctttt tctgagacag cttgtctggt aagtggcttc tctgtgaatt gcctgtaaca     3115

catagtggct tctccgccct tgtaaggtgt tcagtagagc taaataaatg taatagccaa     3175

acccactctg ttggtagcaa ttggcagccc tatttcagtt tattttttct tctgttttct     3235

tcttttcttt ttttaaacag taaaccttaa cagatgcgtt cagcagactg gtttgcagtg     3295

aattttcatt tctttcctta tcaccccctt gttgtaaaaa gcccagcact tgaattgtta     3355

ttactttaaa tgttctgtat ttgtatctgt ttttattagc caattagtgg gattttatgc     3415

cagttgttaa aatgagcatt gatgtaccca ttttttaaaa aagcaaggca cagcctttgc     3475

ccaaaactgt catcctaacg tttgtcattc cagtttgagt taatgtgctg agcatttttt     3535

taaaagaagc tttgtaataa aacattttta aaaattgtca                    3575

<210>  32
<211>  399
<212>  PRT
<213>  Homo sapiens

<400>  32

Met Pro Gln Leu Ser Gly Gly Gly Gly Gly Gly Gly Gly Asp Pro Glu
1               5                   10                  15

Leu Cys Ala Thr Asp Glu Met Ile Pro Phe Lys Asp Glu Gly Asp Pro
            20                  25                  30

Gln Lys Glu Lys Ile Phe Ala Glu Ile Ser His Pro Glu Glu Glu Gly
        35                  40                  45

Asp Leu Ala Asp Ile Lys Ser Ser Leu Val Asn Glu Ser Glu Ile Ile
    50                  55                  60

Pro Ala Ser Asn Gly His Glu Val Ala Arg Gln Ala Gln Thr Ser Gln
65                  70                  75                  80

Glu Pro Tyr His Asp Lys Ala Arg Glu His Pro Asp Asp Gly Lys His
                85                  90                  95

Pro Asp Gly Gly Leu Tyr Asn Lys Gly Pro Ser Tyr Ser Ser Tyr Ser
            100                 105                 110

Gly Tyr Ile Met Met Pro Asn Met Asn Asn Asp Pro Tyr Met Ser Asn
        115                 120                 125

Gly Ser Leu Ser Pro Pro Ile Pro Arg Thr Ser Asn Lys Val Pro Val
    130                 135                 140

Val Gln Pro Ser His Ala Val His Pro Leu Thr Pro Leu Ile Thr Tyr
145                 150                 155                 160

Ser Asp Glu His Phe Ser Pro Gly Ser His Pro Ser His Ile Pro Ser
                165                 170                 175

Asp Val Asn Ser Lys Gln Gly Met Ser Arg His Pro Pro Ala Pro Asp
            180                 185                 190

Ile Pro Thr Phe Tyr Pro Leu Ser Pro Gly Gly Val Gly Gln Ile Thr
        195                 200                 205

Pro Pro Leu Gly Trp Gln Gly Gln Pro Val Tyr Pro Ile Thr Gly Gly

210                 215                 220

Phe Arg Gln Pro Tyr Pro Ser Ser Leu Ser Val Asp Thr Ser Met Ser
225                 230                 235                 240

Arg Phe Ser His His Met Ile Pro Gly Pro Pro Gly Pro His Thr Thr
                245                 250                 255

Gly Ile Pro His Pro Ala Ile Val Thr Pro Gln Val Lys Gln Glu His
            260                 265                 270

Pro His Thr Asp Ser Asp Leu Met His Val Lys Pro Gln His Glu Gln
        275                 280                 285

Arg Lys Glu Gln Glu Pro Lys Arg Pro His Ile Lys Lys Pro Leu Asn
    290                 295                 300

Ala Phe Met Leu Tyr Met Lys Glu Met Arg Ala Asn Val Val Ala Glu
305                 310                 315                 320

Cys Thr Leu Lys Glu Ser Ala Ala Ile Asn Gln Ile Leu Gly Arg Arg
                325                 330                 335

Trp His Ala Leu Ser Arg Glu Glu Gln Ala Lys Tyr Tyr Glu Leu Ala
            340                 345                 350

Arg Lys Glu Arg Gln Leu His Met Gln Leu Tyr Pro Gly Trp Ser Ala
        355                 360                 365

Arg Asp Asn Tyr Gly Lys Lys Lys Lys Arg Lys Arg Glu Lys Leu Gln
    370                 375                 380

Glu Ser Ala Ser Gly Thr Gly Pro Arg Met Thr Ala Ala Tyr Ile
385                 390                 395

<210>  33
<211>  3536
<212>  DNA
<213>  Homo sapiens

<220>
<221>  CDS
<222>  (1190)..(2305)

<400>  33
   agcgccggcg aggcgcggga ggaggagaag cagtggggag gcgcagccgc tcacctgcgg      60
   ggcagggcgc ggaggaggga cccgggctgc gcgctctcgg gccgaggaac caggacgcgc     120
   ccggagcctc gcacgcggcc aagctcgggg cgtcccctcc cctcggccgg gcgaactcaa     180
   ggggcgcagc tctttgcttt gacagagctg gccggcggag gcgtgcagag cggcgagccg 240
   gcgagccagg ctgagaaact cgagccggga acaaagaggg gtcggactga gtgtgtgtgt 300
   cggctcgagc tccgggcaga ggcatttggg cccgaggccc ccgctgtgac tccccgagac 360
   tccgcagtgc cctccactgc ggagtccccg cgcttgccgg caaaaacttt attcttggca 420
   aacttctctt tctcttcccc tcctcctcgg cccccatctt ctgctcctcc tccttctcta 480
   gcagattaaa tgagcctcga gaagaaaaac cgaagcgaaa gggaagaaaa taagaagatc 540
   taaaacggac atctccagcg tgggtggctc ctttttcttt ttcttttttt cccacccttc 600
   aggaagtgga cgtttcgtta tcttctgatc cttgcacctt cttttggggc aaacggggcc 660
   cttctgccca gatcccctct cttttctcgg aaaacaaact actaagtcgg catccggggt 720
   aactacagtg gagagggttt ccgcggagac gcgccgccgg accctcctct gcactttggg 780
   gaggcgtgct ccctccagaa ccggcgttct ccgcgcgcaa atcccggcga cgcggggtcg 840
   cggggtggcc gccggggcag cctcgtctag cgcgcgccgc gcagacgccc ccggagtcgc 900
   cagctaccgc agccctcgcc gcccagtgcc cttcggcctc gggggcgggc gcctgcgtcg 960
   gtctccgcga agcgggaaag cgcggcggcc gccgggattc gggcgccgcg gcagctgctc 1020
   cggctgccgg ccggcggccc cgcgctcgcc cgccccgctt ccgcccgctg tcctgctgca 1080
   cgaacccttc caactctcct ttcctccccc acccttgagt tacccctctg tctttcctgc 1140
   tgttgcgcgg gtgctcccac agcggagcgg agattacaga gccgccggg atg ccc caa      1198
                                                        Met Pro Gln
                                                        1
   ctc tcc gga gga ggt ggc ggc ggc ggg ggg gac ccg gaa ctc tgc gcc 1246
   Leu Ser Gly Gly Gly Gly Gly Gly Gly Gly Asp Pro Glu Leu Cys Ala
   5 10 15
   acg gac gag atg atc ccc ttc aag gac gag ggc gat cct cag aag gaa 1294
   Thr Asp Glu Met Ile Pro Phe Lys Asp Glu Gly Asp Pro Gln Lys Glu
   20 25 30 35
   aag atc ttc gcc gag atc agt cat ccc gaa gag gaa ggc gat tta gct 1342
   Lys Ile Phe Ala Glu Ile Ser His Pro Glu Glu Glu Gly Asp Leu Ala
   40 45 50
   gac atc aag tct tcc ttg gtg aac gag tct gaa atc atc ccg gcc agc 1390
   Asp Ile Lys Ser Ser Leu Val Asn Glu Ser Glu Ile Ile Pro Ala Ser
   55 60 65
   aac gga cac gag gtg gcc aga caa gca caa acc tct cag gag ccc tac 1438
   Asn Gly His Glu Val Ala Arg Gln Ala Gln Thr Ser Gln Glu Pro Tyr
   70 75 80
   cac gac aag gcc aga gaa cac ccc gat gac gga aag cat cca gat gga 1486
   His Asp Lys Ala Arg Glu His Pro Asp Asp Gly Lys His Pro Asp Gly
   85 90 95
   ggc ctc tac aac aag gga ccc tcc tac tcg agt tat tcc ggg tac ata 1534
   Gly Leu Tyr Asn Lys Gly Pro Ser Tyr Ser Ser Tyr Ser Gly Tyr Ile
   100                 105                 110                 115
   atg atg cca aat atg aat aac gac cca tac atg tca aat gga tct ctt       1582
   Met Met Pro Asn Met Asn Asn Asp Pro Tyr Met Ser Asn Gly Ser Leu
                   120                 125                 130
   tct cca ccc atc ccg aga aca tca aat aaa gtg ccc gtg gtg cag cca       1630
   Ser Pro Pro Ile Pro Arg Thr Ser Asn Lys Val Pro Val Val Gln Pro
               135                 140                 145
   tcc cat gcg gtc cat cct ctc acc ccc ctc atc act tac agt gac gag       1678
   Ser His Ala Val His Pro Leu Thr Pro Leu Ile Thr Tyr Ser Asp Glu
           150                 155                 160
   cac ttt tct cca gga tca cac ccg tca cac atc cca tca gat gtc aac       1726
   His Phe Ser Pro Gly Ser His Pro Ser His Ile Pro Ser Asp Val Asn
       165                 170                 175
   tcc aaa caa ggc atg tcc aga cat cct cca gct cct gat atc cct act       1774
   Ser Lys Gln Gly Met Ser Arg His Pro Pro Ala Pro Asp Ile Pro Thr
   180                 185                 190                 195
   ttt tat ccc ttg tct ccg ggt ggt gtt gga cag atc acc cca cct ctt       1822
   Phe Tyr Pro Leu Ser Pro Gly Gly Val Gly Gln Ile Thr Pro Pro Leu
                   200                 205                 210
   ggc tgg ttt tcc cat cat atg att ccc ggt cct cct ggt ccc cac aca       1870
   Gly Trp Phe Ser His His Met Ile Pro Gly Pro Pro Gly Pro His Thr
               215                 220                 225
   act ggc atc cct cat cca gct att gta aca cct cag gtc aaa cag gaa       1918
   Thr Gly Ile Pro His Pro Ala Ile Val Thr Pro Gln Val Lys Gln Glu
           230                 235                 240
   cat ccc cac act gac agt gac cta atg cac gtg aag cct cag cat gaa       1966
   His Pro His Thr Asp Ser Asp Leu Met His Val Lys Pro Gln His Glu
       245                 250                 255
   cag aga aag gag cag gag cca aaa aga cct cac att aag aag cct ctg       2014
   Gln Arg Lys Glu Gln Glu Pro Lys Arg Pro His Ile Lys Lys Pro Leu
   260                 265                 270                 275
   aat gct ttt atg tta tac atg aaa gaa atg aga gcg aat gtc gtt gct       2062
   Asn Ala Phe Met Leu Tyr Met Lys Glu Met Arg Ala Asn Val Val Ala
                   280                 285                 290
   gag tgt act cta aaa gaa agt gca gct atc aac cag att ctt ggc aga       2110
   Glu Cys Thr Leu Lys Glu Ser Ala Ala Ile Asn Gln Ile Leu Gly Arg
               295                 300                 305
   agg tgg cat gcc ctc tcc cgt gaa gag cag gct aaa tat tat gaa tta       2158
   Arg Trp His Ala Leu Ser Arg Glu Glu Gln Ala Lys Tyr Tyr Glu Leu
           310                 315                 320
   gca cgg aaa gaa aga cag cta cat atg cag ctt tat cca ggc tgg tct       2206
   Ala Arg Lys Glu Arg Gln Leu His Met Gln Leu Tyr Pro Gly Trp Ser
       325                 330                 335
   gca aga gac aat tat ggt aag aaa aag aag agg aag aga gag aaa cta       2254
   Ala Arg Asp Asn Tyr Gly Lys Lys Lys Lys Arg Lys Arg Glu Lys Leu
   340                 345                 350                 355
   cag gaa tct gca tca ggt aca ggt cca aga atg aca gct gcc tac atc       2302
   Gln Glu Ser Ala Ser Gly Thr Gly Pro Arg Met Thr Ala Ala Tyr Ile
                   360                 365                 370
   tga aacatggtgg aaaacgaagc tcattcccaa cgtgcaaagc caaggcagcg      2355
   accccaggac ctcttctgga gatggaagct tgttgaaaac ccagactgtc tccacggcct      2415
   gcccagtcga ccccaaagga acactgacat caattttacc ctgaggtcac tgctagagac      2475
   gctgatccat aaagacaatc actgccaacc cctctttcgt ctactgcaag agccaagttc      2535
   caaaataaag cataaaaagg ttttttaaaa ggaaatgtaa aagcacatga gaatgctagc      2595
   aggctgtggg gcagctgagc agcttttctc ccctcatatc tgcgtgcact tcccagagca      2655
   tcttgcatcc aaacctgtaa cctttcggca aggacggtaa cttggctgca tttgcctgtc      2715
   atgcgcaact ggagccagca accagcacat ccatcagcac cccagtggag gagttcatgg      2775
   aagagttccc tctttgtttc tgcttcattt ttctttcttt tcttttctcc taaagctttt      2835
   atttaacagt gcaaaaggat cgtttttttt tgctttttta aacttgaatt tttttaattt      2895
   acacttttta gttttaattt tcttgtatat tttgctagct atgagctttt aaataaaatt      2955
   gaaagttctg gaaaagtttg aaataatgac ataaaaagaa gccttctttt tctgagacag      3015
   cttgtctggt aagtggcttc tctgtgaatt gcctgtaaca catagtggct tctccgccct      3075
   tgtaaggtgt tcagtagagc taaataaatg taatagccaa acccactctg ttggtagcaa      3135
   ttggcagccc tatttcagtt tattttttct tctgttttct tcttttcttt ttttaaacag      3195
   taaaccttaa cagatgcgtt cagcagactg gtttgcagtg aattttcatt tctttcctta      3255
   tcaccccctt gttgtaaaaa gcccagcact tgaattgtta ttactttaaa tgttctgtat      3315
   ttgtatctgt ttttattagc caattagtgg gattttatgc cagttgttaa aatgagcatt      3375
   gatgtaccca ttttttaaaa aagcaaggca cagcctttgc ccaaaactgt catcctaacg      3435
   tttgtcattc cagtttgagt taatgtgctg agcatttttt taaaagaagc tttgtaataa      3495
   aacattttta aaaattgtca tttaaaaaaa aaaaaaaaaa a                    3536

<210>  34
<211>  371
<212>  PRT
<213>  Homo sapiens

<400>  34

Met Pro Gln Leu Ser Gly Gly Gly Gly Gly Gly Gly Gly Asp Pro Glu
1               5                   10                  15

Leu Cys Ala Thr Asp Glu Met Ile Pro Phe Lys Asp Glu Gly Asp Pro
            20                  25                  30

Gln Lys Glu Lys Ile Phe Ala Glu Ile Ser His Pro Glu Glu Glu Gly
        35                  40                  45

Asp Leu Ala Asp Ile Lys Ser Ser Leu Val Asn Glu Ser Glu Ile Ile
    50                  55                  60

Pro Ala Ser Asn Gly His Glu Val Ala Arg Gln Ala Gln Thr Ser Gln
65                  70                  75                  80

Glu Pro Tyr His Asp Lys Ala Arg Glu His Pro Asp Asp Gly Lys His
                85                  90                  95

Pro Asp Gly Gly Leu Tyr Asn Lys Gly Pro Ser Tyr Ser Ser Tyr Ser
            100                 105                 110

Gly Tyr Ile Met Met Pro Asn Met Asn Asn Asp Pro Tyr Met Ser Asn
        115                 120                 125

Gly Ser Leu Ser Pro Pro Ile Pro Arg Thr Ser Asn Lys Val Pro Val
    130                 135                 140

Val Gln Pro Ser His Ala Val His Pro Leu Thr Pro Leu Ile Thr Tyr
145                 150                 155                 160

Ser Asp Glu His Phe Ser Pro Gly Ser His Pro Ser His Ile Pro Ser
                165                 170                 175

Asp Val Asn Ser Lys Gln Gly Met Ser Arg His Pro Pro Ala Pro Asp
            180                 185                 190

Ile Pro Thr Phe Tyr Pro Leu Ser Pro Gly Gly Val Gly Gln Ile Thr
        195                 200                 205

Pro Pro Leu Gly Trp Phe Ser His His Met Ile Pro Gly Pro Pro Gly
    210                 215                 220

Pro His Thr Thr Gly Ile Pro His Pro Ala Ile Val Thr Pro Gln Val
225                 230                 235                 240

Lys Gln Glu His Pro His Thr Asp Ser Asp Leu Met His Val Lys Pro
                245                 250                 255

Gln His Glu Gln Arg Lys Glu Gln Glu Pro Lys Arg Pro His Ile Lys
            260                 265                 270

Lys Pro Leu Asn Ala Phe Met Leu Tyr Met Lys Glu Met Arg Ala Asn
        275                 280                 285

Val Val Ala Glu Cys Thr Leu Lys Glu Ser Ala Ala Ile Asn Gln Ile

290                 295                 300

Leu Gly Arg Arg Trp His Ala Leu Ser Arg Glu Glu Gln Ala Lys Tyr
305                 310                 315                 320

Tyr Glu Leu Ala Arg Lys Glu Arg Gln Leu His Met Gln Leu Tyr Pro
                325                 330                 335

Gly Trp Ser Ala Arg Asp Asn Tyr Gly Lys Lys Lys Lys Arg Lys Arg
            340                 345                 350

Glu Lys Leu Gln Glu Ser Ala Ser Gly Thr Gly Pro Arg Met Thr Ala
        355                 360                 365

Ala Tyr Ile
    370

<210>  35
<211>  3495
<212>  DNA
<213>  Homo sapiens

<220>
<221>  CDS
<222>  (1190)..(2350)

<400>  35
agcgccggcg aggcgcggga ggaggagaag cagtggggag gcgcagccgc tcacctgcgg      60

ggcagggcgc ggaggaggga cccgggctgc gcgctctcgg gccgaggaac caggacgcgc     120

ccggagcctc gcacgcggcc aagctcgggg cgtcccctcc cctcggccgg gcgaactcaa     180

ggggcgcagc tctttgcttt gacagagctg gccggcggag gcgtgcagag cggcgagccg     240

gcgagccagg ctgagaaact cgagccggga acaaagaggg gtcggactga gtgtgtgtgt     300

cggctcgagc tccgggcaga ggcatttggg cccgaggccc ccgctgtgac tccccgagac     360

tccgcagtgc cctccactgc ggagtccccg cgcttgccgg caaaaacttt attcttggca     420

aacttctctt tctcttcccc tcctcctcgg cccccatctt ctgctcctcc tccttctcta     480

gcagattaaa tgagcctcga gaagaaaaac cgaagcgaaa gggaagaaaa taagaagatc     540

taaaacggac atctccagcg tgggtggctc ctttttcttt ttcttttttt cccacccttc     600

aggaagtgga cgtttcgtta tcttctgatc cttgcacctt cttttggggc aaacggggcc     660

cttctgccca gatcccctct cttttctcgg aaaacaaact actaagtcgg catccggggt     720

aactacagtg gagagggttt ccgcggagac gcgccgccgg accctcctct gcactttggg     780

gaggcgtgct ccctccagaa ccggcgttct ccgcgcgcaa atcccggcga cgcggggtcg     840

cggggtggcc gccggggcag cctcgtctag cgcgcgccgc gcagacgccc ccggagtcgc     900

cagctaccgc agccctcgcc gcccagtgcc cttcggcctc gggggcgggc gcctgcgtcg 960

gtctccgcga agcgggaaag cgcggcggcc gccgggattc gggcgccgcg gcagctgctc 1020

cggctgccgg ccggcggccc cgcgctcgcc cgccccgctt ccgcccgctg tcctgctgca 1080

cgaacccttc caactctcct ttcctccccc acccttgagt tacccctctg tctttcctgc 1140

tgttgcgcgg gtgctcccac agcggagcgg agattacaga gccgccggg atg ccc caa 1198
Met Pro Gln
1

ctc tcc gga gga ggt ggc ggc ggc ggg ggg gac ccg gaa ctc tgc gcc 1246
Leu Ser Gly Gly Gly Gly Gly Gly Gly Gly Asp Pro Glu Leu Cys Ala
    5                   10                  15

acg gac gag atg atc ccc ttc aag gac gag ggc gat cct cag aag gaa 1294
Thr Asp Glu Met Ile Pro Phe Lys Asp Glu Gly Asp Pro Gln Lys Glu
20                  25                  30                  35

aag atc ttc gcc gag atc agt cat ccc gaa gag gaa ggc gat tta gct 1342
Lys Ile Phe Ala Glu Ile Ser His Pro Glu Glu Glu Gly Asp Leu Ala
                40                  45                  50

gac atc aag tct tcc ttg gtg aac gag tct gaa atc atc ccg gcc agc 1390
Asp Ile Lys Ser Ser Leu Val Asn Glu Ser Glu Ile Ile Pro Ala Ser
            55                  60                  65

aac gga cac gag gtg gcc aga caa gca caa acc tct cag gag ccc tac 1438
Asn Gly His Glu Val Ala Arg Gln Ala Gln Thr Ser Gln Glu Pro Tyr
        70                  75                  80

cac gac aag gcc aga gaa cac ccc gat gac gga aag cat cca gat gga 1486
His Asp Lys Ala Arg Glu His Pro Asp Asp Gly Lys His Pro Asp Gly
    85                  90                  95

ggc ctc tac aac aag gga ccc tcc tac tcg agt tat tcc ggg tac ata 1534
Gly Leu Tyr Asn Lys Gly Pro Ser Tyr Ser Ser Tyr Ser Gly Tyr Ile
100                 105                 110                 115

atg atg cca aat atg aat aac gac cca tac atg tca aat gga tct ctt 1582
Met Met Pro Asn Met Asn Asn Asp Pro Tyr Met Ser Asn Gly Ser Leu
                120                 125                 130

tct cca ccc atc ccg aga aca tca aat aaa gtg ccc gtg gtg cag cca 1630
Ser Pro Pro Ile Pro Arg Thr Ser Asn Lys Val Pro Val Val Gln Pro
            135                 140                 145

tcc cat gcg gtc cat cct ctc acc ccc ctc atc act tac agt gac gag 1678
Ser His Ala Val His Pro Leu Thr Pro Leu Ile Thr Tyr Ser Asp Glu
        150                 155                 160

cac ttt tct cca gga tca cac ccg tca cac atc cca tca gat gtc aac 1726
His Phe Ser Pro Gly Ser His Pro Ser His Ile Pro Ser Asp Val Asn
    165                 170                 175

tcc aaa caa ggc atg tcc aga cat cct cca gct cct gat atc cct act 1774
Ser Lys Gln Gly Met Ser Arg His Pro Pro Ala Pro Asp Ile Pro Thr
180                 185                 190                 195

ttt tat ccc ttg tct ccg ggt ggt gtt gga cag atc acc cca cct ctt 1822
Phe Tyr Pro Leu Ser Pro Gly Gly Val Gly Gln Ile Thr Pro Pro Leu

200                 205                 210

ggc tgg ttt tcc cat cat atg att ccc ggt cct cct ggt ccc cac aca      1870
Gly Trp Phe Ser His His Met Ile Pro Gly Pro Pro Gly Pro His Thr
            215                 220                 225

act ggc atc cct cat cca gct att gta aca cct cag gtc aaa cag gaa      1918
Thr Gly Ile Pro His Pro Ala Ile Val Thr Pro Gln Val Lys Gln Glu
        230                 235                 240

cat ccc cac act gac agt gac cta atg cac gtg aag cct cag cat gaa      1966
His Pro His Thr Asp Ser Asp Leu Met His Val Lys Pro Gln His Glu
    245                 250                 255

cag aga aag gag cag gag cca aaa aga cct cac att aag aag cct ctg      2014
Gln Arg Lys Glu Gln Glu Pro Lys Arg Pro His Ile Lys Lys Pro Leu
260                 265                 270                 275

aat gct ttt atg tta tac atg aaa gaa atg aga gcg aat gtc gtt gct      2062
Asn Ala Phe Met Leu Tyr Met Lys Glu Met Arg Ala Asn Val Val Ala
                280                 285                 290

gag tgt act cta aaa gaa agt gca gct atc aac cag att ctt ggc aga      2110
Glu Cys Thr Leu Lys Glu Ser Ala Ala Ile Asn Gln Ile Leu Gly Arg
            295                 300                 305

agg tgg cat gcc ctc tcc cgt gaa gag cag gct aaa tat tat gaa tta      2158
Arg Trp His Ala Leu Ser Arg Glu Glu Gln Ala Lys Tyr Tyr Glu Leu
        310                 315                 320

gca cgg aaa gaa aga cag cta cat atg cag ctt tat cca ggc tgg tct      2206
Ala Arg Lys Glu Arg Gln Leu His Met Gln Leu Tyr Pro Gly Trp Ser
    325                 330                 335

gca aga gac aat tat ggt aag aaa aag aag agg aag aga gag aaa cta      2254
Ala Arg Asp Asn Tyr Gly Lys Lys Lys Lys Arg Lys Arg Glu Lys Leu
340                 345                 350                 355

cag gaa tct gca tca ggt gga aaa cga agc tca ttc cca acg tgc aaa      2302
Gln Glu Ser Ala Ser Gly Gly Lys Arg Ser Ser Phe Pro Thr Cys Lys
                360                 365                 370

gcc aag gca gcg acc cca gga cct ctt ctg gag atg gaa gct tgt tga      2350
Ala Lys Ala Ala Thr Pro Gly Pro Leu Leu Glu Met Glu Ala Cys
            375                 380                 385

aaacccagac tgtctccacg gcctgcccag tcgaccccaa aggaacactg acatcaattt      2410

taccctgagg tcactgctag agacgctgat ccataaagac aatcactgcc aacccctctt      2470

tcgtctactg caagagccaa gttccaaaat aaagcataaa aaggtttttt aaaaggaaat      2530

gtaaaagcac atgagaatgc tagcaggctg tggggcagct gagcagcttt tctcccctca      2590

tatctgcgtg cacttcccag agcatcttgc atccaaacct gtaacctttc ggcaaggacg      2650

gtaacttggc tgcatttgcc tgtcatgcgc aactggagcc agcaaccagc acatccatca      2710

gcaccccagt ggaggagttc atggaagagt tccctctttg tttctgcttc atttttcttt      2770

cttttctttt ctcctaaagc ttttatttaa cagtgcaaaa ggatcgtttt tttttgcttt      2830

tttaaacttg aattttttta atttacactt tttagtttta attttcttgt atattttgct      2890

agctatgagc ttttaaataa aattgaaagt tctggaaaag tttgaaataa tgacataaaa 2950

agaagccttc tttttctgag acagcttgtc tggtaagtgg cttctctgtg aattgcctgt 3010

aacacatagt ggcttctccg cccttgtaag gtgttcagta gagctaaata aatgtaatag 3070

ccaaacccac tctgttggta gcaattggca gccctatttc agtttatttt ttcttctgtt 3130

ttcttctttt ctttttttaa acagtaaacc ttaacagatg cgttcagcag actggtttgc 3190

agtgaatttt catttctttc cttatcaccc ccttgttgta aaaagcccag cacttgaatt 3250

gttattactt taaatgttct gtatttgtat ctgtttttat tagccaatta gtgggatttt 3310

atgccagttg ttaaaatgag cattgatgta cccatttttt aaaaaagcaa ggcacagcct 3370

ttgcccaaaa ctgtcatcct aacgtttgtc attccagttt gagttaatgt gctgagcatt 3430

tttttaaaag aagctttgta ataaaacatt ttttaaaatt gtcatttaaa aaaaaaaaaa 3490

aaaaa 3495

<210> 36
<211> 386
<212> PRT
<213> Homo sapiens

<400> 36

Met Pro Gln Leu Ser Gly Gly Gly Gly Gly Gly Gly Gly Asp Pro Glu
1               5                   10                  15

Leu Cys Ala Thr Asp Glu Met Ile Pro Phe Lys Asp Glu Gly Asp Pro
            20                  25                  30

Gln Lys Glu Lys Ile Phe Ala Glu Ile Ser His Pro Glu Glu Glu Gly
        35                  40                  45

Asp Leu Ala Asp Ile Lys Ser Ser Leu Val Asn Glu Ser Glu Ile Ile
    50                  55                  60

Pro Ala Ser Asn Gly His Glu Val Ala Arg Gln Ala Gln Thr Ser Gln
65                  70                  75                  80

Glu Pro Tyr His Asp Lys Ala Arg Glu His Pro Asp Asp Gly Lys His
                85                  90                  95

Pro Asp Gly Gly Leu Tyr Asn Lys Gly Pro Ser Tyr Ser Ser Tyr Ser
            100                 105                 110

Gly Tyr Ile Met Met Pro Asn Met Asn Asn Asp Pro Tyr Met Ser Asn
        115                 120                 125

Gly Ser Leu Ser Pro Pro Ile Pro Arg Thr Ser Asn Lys Val Pro Val
    130                 135                 140

Val Gln Pro Ser His Ala Val His Pro Leu Thr Pro Leu Ile Thr Tyr
145                 150                 155                 160

Ser Asp Glu His Phe Ser Pro Gly Ser His Pro Ser His Ile Pro Ser
                165                 170                 175

Asp Val Asn Ser Lys Gln Gly Met Ser Arg His Pro Pro Ala Pro Asp
            180                 185                 190

Ile Pro Thr Phe Tyr Pro Leu Ser Pro Gly Gly Val Gly Gln Ile Thr
        195                 200                 205

Pro Pro Leu Gly Trp Phe Ser His His Met Ile Pro Gly Pro Pro Gly
    210                 215                 220

Pro His Thr Thr Gly Ile Pro His Pro Ala Ile Val Thr Pro Gln Val
225                 230                 235                 240

Lys Gln Glu His Pro His Thr Asp Ser Asp Leu Met His Val Lys Pro
                245                 250                 255

Gln His Glu Gln Arg Lys Glu Gln Glu Pro Lys Arg Pro His Ile Lys
            260                 265                 270

Lys Pro Leu Asn Ala Phe Met Leu Tyr Met Lys Glu Met Arg Ala Asn
        275                 280                 285

Val Val Ala Glu Cys Thr Leu Lys Glu Ser Ala Ala Ile Asn Gln Ile
    290                 295                 300

Leu Gly Arg Arg Trp His Ala Leu Ser Arg Glu Glu Gln Ala Lys Tyr
305                 310                 315                 320

Tyr Glu Leu Ala Arg Lys Glu Arg Gln Leu His Met Gln Leu Tyr Pro
                325                 330                 335

Gly Trp Ser Ala Arg Asp Asn Tyr Gly Lys Lys Lys Lys Arg Lys Arg
            340                 345                 350

Glu Lys Leu Gln Glu Ser Ala Ser Gly Gly Lys Arg Ser Ser Phe Pro
        355                 360                 365

Thr Cys Lys Ala Lys Ala Ala Thr Pro Gly Pro Leu Leu Glu Met Glu
    370                 375                 380

Ala Cys
385

<210> 37
<211> 2660
<212> DNA
<213> Homo sapiens

<220>
<221> CDS
<222> (518)..(1429)

<400> 37
ttttatttgg ggtttgggac cggatcaggc cttaaatcta cgcagaagaa aggcggagaa 60

gcagcggaag ccgggcacta gtcctgcagg gtggaaggcg ctttatcggg tcttctggcc 120

cgctgcgggc gatagagctg gtaacctgag tagagtaaat agagacacgt gggtgctggc 180

tagagaaagc gcggagcagg gccgcgctgg agggctgttt gaagcctagt ccagagctgg 240

tctagggggc ggatggaccc tgatacccga cactccactg gtcacccttg ttccacaact 300

gggaaaccct ctggaggaaa tccggtagaa aagtcctgac acccgaatga tttaaccaac 360

gccttttccc tccaaagaaa cttggaggat ataccttttt ttccttaaat ggcatctttc 420

tacccccaaa aagaaagtgt gtggttttga gggcggggac attttgggtt ttaaacagcg 480

cgtttaagaa cacctcagat ctgccttctc tatccca atg gca gag gtg gcc aga 535
Met Ala Glu Val Ala Arg
1               5

caa gca caa acc tct cag gag ccc tac cac gac aag gcc aga gaa cac 583
Gln Ala Gln Thr Ser Gln Glu Pro Tyr His Asp Lys Ala Arg Glu His
            10                  15                  20

ccc gat gac gga aag cat cca gat gga ggc ctc tac aac aag gga ccc 631
Pro Asp Asp Gly Lys His Pro Asp Gly Gly Leu Tyr Asn Lys Gly Pro
        25                  30                  35

tcc tac tcg agt tat tcc ggg tac ata atg atg cca aat atg aat aac 679
Ser Tyr Ser Ser Tyr Ser Gly Tyr Ile Met Met Pro Asn Met Asn Asn
    40                  45                  50

gac cca tac atg tca aat gga tct ctt tct cca ccc atc ccg aga aca 727
Asp Pro Tyr Met Ser Asn Gly Ser Leu Ser Pro Pro Ile Pro Arg Thr
55                  60                  65                  70

tca aat aaa gtg ccc gtg gtg cag cca tcc cat gcg gtc cat cct ctc 775
Ser Asn Lys Val Pro Val Val Gln Pro Ser His Ala Val His Pro Leu
                75                  80                  85

acc ccc ctc atc act tac agt gac gag cac ttt tct cca gga tca cac 823
Thr Pro Leu Ile Thr Tyr Ser Asp Glu His Phe Ser Pro Gly Ser His
            90                  95                  100

ccg tca cac atc cca tca gat gtc aac tcc aaa caa ggc atg tcc aga 871
Pro Ser His Ile Pro Ser Asp Val Asn Ser Lys Gln Gly Met Ser Arg
        105                 110                 115

cat cct cca gct cct gat atc cct act ttt tat ccc ttg tct ccg ggt 919
His Pro Pro Ala Pro Asp Ile Pro Thr Phe Tyr Pro Leu Ser Pro Gly
    120                 125                 130

ggt gtt gga cag atc acc cca cct ctt ggc tgg ttt tcc cat cat atg 967
Gly Val Gly Gln Ile Thr Pro Pro Leu Gly Trp Phe Ser His His Met
135                 140                 145                 150

att ccc ggt cct cct ggt ccc cac aca act ggc atc cct cat cca gct 1015
Ile Pro Gly Pro Pro Gly Pro His Thr Thr Gly Ile Pro His Pro Ala
                155                 160                 165

att gta aca cct cag gtc aaa cag gaa cat ccc cac act gac agt gac 1063
Ile Val Thr Pro Gln Val Lys Gln Glu His Pro His Thr Asp Ser Asp
            170                 175                 180

cta atg cac gtg aag cct cag cat gaa cag aga aag gag cag gag cca 1111
Leu Met His Val Lys Pro Gln His Glu Gln Arg Lys Glu Gln Glu Pro
        185                 190                 195

aaa aga cct cac att aag aag cct ctg aat gct ttt atg tta tac atg 1159
Lys Arg Pro His Ile Lys Lys Pro Leu Asn Ala Phe Met Leu Tyr Met
    200                 205                 210

aaa gaa atg aga gcg aat gtc gtt gct gag tgt act cta aaa gaa agt 1207
Lys Glu Met Arg Ala Asn Val Val Ala Glu Cys Thr Leu Lys Glu Ser
215                 220                 225                 230

gca gct atc aac cag att ctt ggc aga agg tgg cat gcc ctc tcc cgt 1255
Ala Ala Ile Asn Gln Ile Leu Gly Arg Arg Trp His Ala Leu Ser Arg
                235                 240                 245

gaa gag cag gct aaa tat tat gaa tta gca cgg aaa gaa aga cag cta 1303
Glu Glu Gln Ala Lys Tyr Tyr Glu Leu Ala Arg Lys Glu Arg Gln Leu
            250                 255                 260

cat atg cag ctt tat cca ggc tgg tct gca aga gac aat tat ggt aag 1351
His Met Gln Leu Tyr Pro Gly Trp Ser Ala Arg Asp Asn Tyr Gly Lys
        265                 270                 275

aaa aag aag agg aag aga gag aaa cta cag gaa tct gca tca ggt aca 1399
Lys Lys Lys Arg Lys Arg Glu Lys Leu Gln Glu Ser Ala Ser Gly Thr
    280                 285                 290

ggt cca aga atg aca gct gcc tac atc tga aacatggtgg aaaacgaagc 1449
Gly Pro Arg Met Thr Ala Ala Tyr Ile
295                 300

tcattcccaa cgtgcaaagc caaggcagcg accccaggac ctcttctgga gatggaagct 1509

tgttgaaaac ccagactgtc tccacggcct gcccagtcga ccccaaagga acactgacat 1569

caattttacc ctgaggtcac tgctagagac gctgatccat aaagacaatc actgccaacc 1629

cctctttcgt ctactgcaag agccaagttc caaaataaag cataaaaagg ttttttaaaa 1689

ggaaatgtaa aagcacatga gaatgctagc aggctgtggg gcagctgagc agcttttctc 1749

ccctcatatc tgcgtgcact tcccagagca tcttgcatcc aaacctgtaa cctttcggca 1809

aggacggtaa cttggctgca tttgcctgtc atgcgcaact ggagccagca accagcacat 1869

ccatcagcac cccagtggag gagttcatgg aagagttccc tctttgtttc tgcttcattt 1929

ttctttcttt tcttttctcc taaagctttt atttaacagt gcaaaaggat cgtttttttt 1989

tgctttttta aacttgaatt tttttaattt acacttttta gttttaattt tcttgtatat 2049

tttgctagct atgagctttt aaataaaatt gaaagttctg gaaaagtttg aaataatgac 2109

ataaaaagaa gccttctttt tctgagacag cttgtctggt aagtggcttc tctgtgaatt 2169

gcctgtaaca catagtggct tctccgccct tgtaaggtgt tcagtagagc taaataaatg 2229

taatagccaa acccactctg ttggtagcaa ttggcagccc tatttcagtt tattttttct 2289

tctgttttct tcttttcttt ttttaaacag taaaccttaa cagatgcgtt cagcagactg 2349

gtttgcagtg aattttcatt tctttcctta tcaccccctt gttgtaaaaa gcccagcact 2409

tgaattgtta ttactttaaa tgttctgtat ttgtatctgt ttttattagc caattagtgg 2469

gattttatgc cagttgttaa aatgagcatt gatgtaccca ttttttaaaa aagcaaggca 2529

cagcctttgc ccaaaactgt catcctaacg tttgtcattc cagtttgagt taatgtgctg 2589

agcatttttt taaaagaagc tttgtaataa aacattttta aaaattgtca tttaaaaaaa 2649

aaaaaaaaaa a 2660

<210> 38
<211> 303
<212> PRT
<213> Homo sapiens

<400> 38

Met Ala Glu Val Ala Arg Gln Ala Gln Thr Ser Gln Glu Pro Tyr His
1               5                   10                  15

Asp Lys Ala Arg Glu His Pro Asp Asp Gly Lys His Pro Asp Gly Gly
            20                  25                  30

Leu Tyr Asn Lys Gly Pro Ser Tyr Ser Ser Tyr Ser Gly Tyr Ile Met
        35                  40                  45

Met Pro Asn Met Asn Asn Asp Pro Tyr Met Ser Asn Gly Ser Leu Ser
    50                  55                  60

Pro Pro Ile Pro Arg Thr Ser Asn Lys Val Pro Val Val Gln Pro Ser
65                  70                  75                  80

His Ala Val His Pro Leu Thr Pro Leu Ile Thr Tyr Ser Asp Glu His
                85                  90                  95

Phe Ser Pro Gly Ser His Pro Ser His Ile Pro Ser Asp Val Asn Ser
            100                 105                 110

Lys Gln Gly Met Ser Arg His Pro Pro Ala Pro Asp Ile Pro Thr Phe
        115                 120                 125

Tyr Pro Leu Ser Pro Gly Gly Val Gly Gln Ile Thr Pro Pro Leu Gly
    130                 135                 140

Trp Phe Ser His His Met Ile Pro Gly Pro Pro Gly Pro His Thr Thr
145                 150                 155                 160

Gly Ile Pro His Pro Ala Ile Val Thr Pro Gln Val Lys Gln Glu His
                165                 170                 175

Pro His Thr Asp Ser Asp Leu Met His Val Lys Pro Gln His Glu Gln
            180                 185                 190

Arg Lys Glu Gln Glu Pro Lys Arg Pro His Ile Lys Lys Pro Leu Asn
        195                 200                 205

Ala Phe Met Leu Tyr Met Lys Glu Met Arg Ala Asn Val Val Ala Glu
    210                 215                 220

Cys Thr Leu Lys Glu Ser Ala Ala Ile Asn Gln Ile Leu Gly Arg Arg
225                 230                 235                 240

Trp His Ala Leu Ser Arg Glu Glu Gln Ala Lys Tyr Tyr Glu Leu Ala
                245                 250                 255

Arg Lys Glu Arg Gln Leu His Met Gln Leu Tyr Pro Gly Trp Ser Ala
            260                 265                 270

Arg Asp Asn Tyr Gly Lys Lys Lys Lys Arg Lys Arg Glu Lys Leu Gln
        275                 280                 285

Glu Ser Ala Ser Gly Thr Gly Pro Arg Met Thr Ala Ala Tyr Ile
    290                 295                 300